# EUROPEAN PATENT SPECIFICATION

(11) **EP 1 064 300 B1**
(45) Date of publication and mention of the grant of the patent: **04.03.2009**
(21) Application number: 99911522.3
(22) Date of filing: 24.03.1999
(51) Int. Cl.: C07K 5/12, C07K 5/06, C07D 243/08, C07D 243/10, C07D 255/02

(54) **PEPTIDE TURN MIMETICS**
PEPTIDSCHLEIFENMIMETIKA
MIMETIQUES D'ENROULEMENT PEPTIDIQUE

(30) Priority: 24.03.1998 AU PP254898
(43) Date of publication of application: 03.01.2001
(73) Proprietor: Mimetica Pty Limited, Milton, QLD 4064 (AU)
(72) Inventor: CASSIDY, Peter, Joseph, Ashgrove, Queensland 4065 (AU); HUNT, Peter, Alan, Merck Research Laboratories, Harlow, Essex CM20 2QR (GB); ALEWOOD, Paul, Francis, Moggill, QLD 4070 (AU); RAMSDALE, Tracie, Elizabeth, Tarragindi, QLD 4065 (AU)
(74) Representative: Brierley, Anthony Paul
(86) International application number: PCT/AU1999/000207
(87) International publication number: WO 1999/048913

(56) References cited:
- WO-A-92/13878
- WO-A-95/25120
- WO-A-96/22304
- WO-A-97/15577
- WO-A-98/49168
- WO-A-98/49168
- MA X ET AL: "SYNTHESIS OF A HETEROCYCLIC GAMMA-TURN PEPTIDOMIMETIC SEQUENCE" PROTEIN AND PEPTIDE LETTERS, BENTHAM SCIENCE PUBLISHERS, SCHIPHOL, NL, 1995, pages 347-350, XP008059252 ISSN: 0929-8665
- NOUVET A ET AL: "Synthesis of perhydrodiazepinones as new putative peptidomimetics" TETRAHEDRON, ELSEVIER SCIENCE PUBLISHERS, AMSTERDAM, NL, vol. 55, no. 15, 9 April 1999 (1999-04-09), pages 4685-4698, XP004223084 ISSN: 0040-4020
- CURRENT MEDICINAL CHEMISTRY, Volume 5, No. 1, issued February 1998, DAVID P. FAIRLIE et al., "Towards Protein Surface Mimetics", pages 29-62. XP000987330
- SYNLETT, issued November 1993, MICHAEL KAHN, "Peptide Secondary Structure Mimetics: Recent Advances and Future Challenges", pages 821-826. XP000979574
- TETRAHEDRON, Volume 49, No. 17, 1993, W.C. RIPKA et al., "Protein beta-Turn Mimetics II: Design, Synthesis and Evaluation in the Cyclic Peptide Gramicidin S", pages 3609-3628. XP000979571
- TETRAHEDRON, Volume 49, No. 17, 1993, W.C. RIPKA et al., "Protein beta-Turn Mimetics I: Design, Synthesis and Evaluation in Model Cyclic Peptides", pages 3593-3608. XP000979570
- TETRAHEDRON, Volume 49, No. 17, 1993, JAMES F. CALLAHAN et al., "The Use of gamma-Turn Mimetics to Define Peptide Secondary Structure", pages 3479-3488. XP000979569
- TETRAHEDRON, Volume 49, No. 17, 1993, BENJAMIN GARDNER et al., "Conformationally Constrained Nonpeptide beta-Turn Mimetics of Enkephatin", pages 3433-3448. XP000979568
- BIOORGANIC AND MEDICINAL CHEMISTRY LETTERS, SATO M. et al.,Volume 3, No. 6, 1993, "Design and Synthesis of Hypertrehalosemic Hormone Mimetics", pages 1277-1282. XP000982937
- BIOORGANIC AND MEDICINAL CHEMISTRY LETTERS, SU T. et al., Volume 3, No. 5, 1993, "Nonpeptide beta-Turn Mimetics of Enkephalin", pages 835-840. XP000982938
- PESTICIDE SCIENCE, Volume 51, 1997, URSULA EGNER et al., "Turn Mimetics for Peptide Design", pages 95-99. XP000979573
- TETRAHEDRON, Volume 53, No. 38, 1997, STEPHEN HANESSIAN et al., "Design and Synthesis of Conformationally Constrained Amino Acids as Versatile Scaffolds and Peptide Mimetics", pages 12789-12854. XP004106190

## Description

### FIELD OF THE INVENTION

THIS INVENTION relates to new compounds designed to be peptide turn mimetics, and to new compounds useful for the synthesis of peptide mimetics, especially turn mimetics. Peptide mimetics are used to reproduce, the important structural and functional elements contained in a bio-active peptide sequence principally in order to develop novel pharmaceuticals with increased binding affinity, selectivity, stability and/or oral bioavailability compared to the bio-active peptide.

### BACKGROUND OF THE INVENTION

Reverse turns (beta and gamma turns and beta buldges) are localised on the protein surface (Kuntz, 1972) and are of importance in protein interactions (Rose *et al.,* 1985; Chalmers and Marshall, 1995) (and references contained therein). In addition reverse turns are important structures of peptide hormones and other biologically active peptides and cyclic peptides.(Giannis and Kolter, 1993; Olson *et al.,* 1993; Kessler *et al.,* 1995)

Peptide mimetics and peptide turn mimetics have as their object the replacement of a peptide sequence (a peptide turn) with a new compound which retains the elements essential for biological activity, thereby enabling or facilitating the development of novel pharmaceuticals devoid of the inherent problems of peptides - namely flexibility and poor pharmacodynamics. The essential elements for biological activity are thought to be the peptide sidechain groups (Farmer and Arièns, 1982: Ball and Alewood, 1990), therefore a peptide mimetic should include the side chain groups to have the best chance of retaining biological activity. A peptide mimetic may then take the form of a framework for displaying sidechain groups in an appropriate arrangement.

The majority of reverse turns are beta turns. The generally

accepted definition of the beta turn is a sequence of four residues where the distance between the alpha carbons of residue (i) and residue (i+3) (defined as d) is less than 7Å, and the central residues (i+1, i+2) are non-helical.(Lewis *et al.,* 1973) The general structure is shown below and includes the phi (φ) and psi (ψ) backbone dihedral angles that are used to describe the conformation of the peptide backbone. A schematic conversion of the beta turn to a beta turn mimetic is also shown - the peptide backbone is here replaced by an undefined framework.

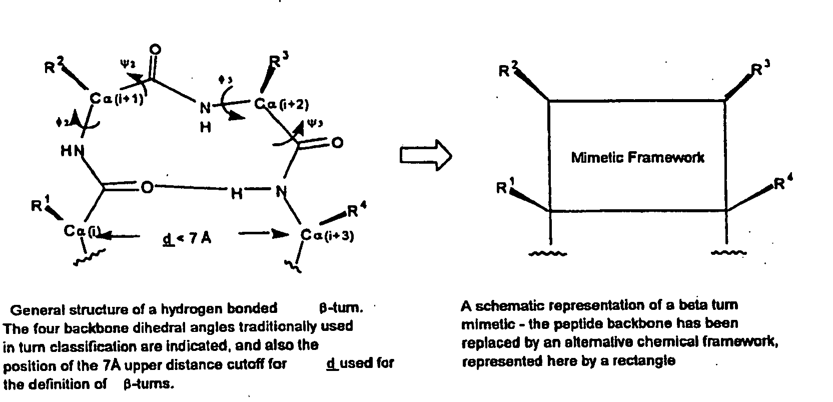

The gamma turn is generally defined by the presence of a hydrogen bond between C=O (i) and N-H (i+2) to form a pseudo seven membered ring as illustrated below (Milner-White, 1988). Where the equivalent hydrogen bond is present in a beta turn a pseudo ten membered ring is formed.

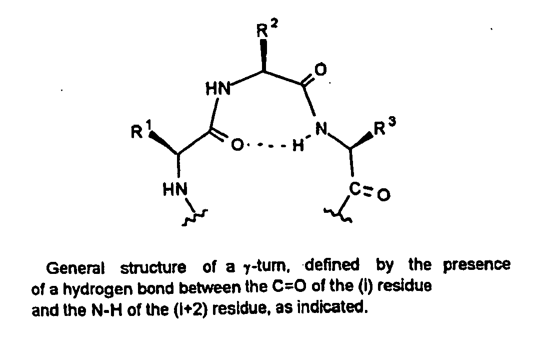

The chemical synthesis of a framework having four independent chiral groups each with a wide range of possible functionality (for example, a beta turn mimetic) is a very significant synthetic challenge (Nakanishi and Kahn, 1996) as illustrated by the the fact that most proposed beta turn mimetics either do not provide for the incorporation of any sidechain functionality, or provide for a limited range of functionality, and at a limited number of positions. Reference may be made to reviews by Ball and by Hölzemann for illustration of these points (Ball and Alewood, 1990; Hölzemann, 1991; Hölzemann, 1991). In the case of mimetics that do provide for the incorporation of sidechain functionality, the syntheses are often complex and lengthy, and most seriously may require a different synthetic method for different sidechain sequences (i.e. the synthetic method is not generic). For example, in the work of Callahan, Huffman and Newlander on gamma turn mimetics the synthetic method varied depending on the sidechain sequence required - a 10 step sequence for a Gly-Phe-Leu mimetic, 13 steps for Phe-Gly-Val and 21 steps for Ala-Phe-Ala (Huffman *et al.,* 1988; Callahan *et al.,* 1992; Newlander *et al*., 1993). Given that the possible combinations of three residue sequences of the 20 natural amino acids is 8000 (20x20x20), and 160,000 for the four residue beta turn sequence, such non-generic methods are of limited use. The methods of Callahan and Huffman were further hampered by a lack of chiral control, as are most methods in the art.

In the development of peptide turn mimetics a further important issue is the reproduction of the variety of different turn conformations, particularly of the beta turn. Several different methods of describing turn conformation have been proposed, the traditional method having several turn types based on the backbone dihedral angles of the (i+1) and (i+2) residues i.e. I, I', II, II', III, III', IV, V, VIa, VIb, VII and VIII, with even this diversity of types being insufficient to adequately describe turn conformations.(Richardson, 1981; Wilmont and Thornton, 1990; Ball *et al*., 1993) No *single* mimetic framework can accurately mimic this diversity of turns; a selection of mimetic frameworks is required.

The problems encountered in the development of peptide turn mimetic syntheses are discussed in a review by Kahn (Kahn, 1993) and reference may also be made to a review article entitled "Design of Peptidomimetics" (Nakanishi and Kahn, 1996) which discusses aspects of mimetic design and developments regarding peptide mimetics.

The uses of reverse turn mimetics (and peptides or other compounds containing reverse turn mimetics) in drug development have been described in the art, notably in publications by Kahn and co-workers (Kahn, 1996; Nakanishi and Kahn, 1996; Qabar *et al*., 1996) and references contained therein. An important example of the application of reverse turn mimetics is the production of mimetics of known biologically active cyclic peptides (typically penta- or hexapeptides), as illustrated by Hirschmann and co-workers with D-glucose based mimetics.(Hirschmann *et al*., 1992; Hirschmann *et al.*, 1993)

Other beta turn mimetics having biological activity are known in the art. For example, U.S. Patent 4535169 discloses a method for the synthesis of beta turn mimetics which can incorporate a functional substitution for the (i+3) sidechain (only), and Krystenansky *et al.* disclose a leucine enkephalin mimetic based on this method which had analgesic activity one third the potency of morphine (Krstenansky *et al.,* 1982).

Reference may also be made to U.S. Patents 5475085 and 5618914 and International Publication WO96/22304 (all Kahn, M) which describe methods for the synthesis of a range of reverse turn mimetics. These mimetics are all produced by a modular synthesis technique (that may be applied to solid phase synthesis) which involves amino acid derivatives and various dipeptide azetidinones synthesised by a variety of techniques. An important common step in all of the syntheses of these mimetics is the cyclisation reaction which involves the azetidinone as activated ester component. Conformational variation is introduced to these mimetics by the inclusion of a variable component ("X") in the ring of the cyclic turn mimetics. It should be noted that with two exceptions (the parent mimetics which have X=NH and have a ten or eleven membered ring) the beta turn mimetics produced by these methods have ring sizes of twelve members and above. Such large rings allow many conformations with d>7Å, the mimetic conformations are therefore biased away from the accepted definition of a beta turn (d less than 7A), or more importantly the conformations are biased away from the most common reverse turn conformations which have d in the range of 4.5Å to 6Å (Rose *et al.,* 1985; Gardner *et al.*,1993). Enkephalin mimetics have been made (Gardner *et al.,* 1993) and also mimetics of a loop of CD4 that inhibit binding of HIV gp120 and infection of human lymphocytes (Chen *et al.*, 1992). The synthetic methods described for the majority of these mimetics appear to be limited with respect to the possible functionality at the (i) and (i+1) positions, and indeed no mimetic with any functionality at the (i+1) position (other than -H = glycine = no sidechain) appears to have been described at this time.

Reference may also be made to International Publication WO97/15577 (Kahn, M) which describes the synthesis of bicyclic reverse turn mimetics and chemical libraries containing such reverse turn mimetics. While concise, the synthetic methods do not provide for control of chirality at all positions, and the degree of sidechain function generality is questionable at two of the four positions. Furthermore the structure of the mimetics means they are not able to be easily incorporated in a peptide sequence, nor do they reproduce the relative positioning of the sidechain groups in the ideal manner (each sidechain attachment position should ideally be separated by three covalent bonds, as in a peptide).

Reference may also be made to the turn mimetics of Virgilio *et al.* (Valle *et al.*, 1989; Virgilio and Ellman, 1994; Virgilio *et al.,* 1996) that incorporate functionality at the (i+1), (i+2) and (i+3) positions (but not the (i) position), and that do not allow for incorporation of the mimetic in a peptide sequence (i.e. no amino and carboxy terminal groups in addition to the sidechains are present).

Reference may be made to U.S. Patents 5438188 and 5470849 (Callahan and Huffman) that describe biologically active compounds containing gamma turn mimetics, providing further illustration of the general utility of reverse turn mimetics.

Reference may also be made to International Publication WO95/25120 that describes the use of turn mimetics in the synthesis of peptide vaccines for generating a protective immune response in warm blooded animals.

In the methods and mimetics of the aforementioned references several common problems are evident: limited numbers of sidechains are able to be reproduced, there is limited control of chirality in the syntheses and a limited range of sidechain functions could be included. In addition, many of the syntheses of turn mimetics described are relatively long and complex, even when not all the sidechain functions are included, for example the syntheses of certain enkephalin mimetics were in the range of approximately 15 to 21 steps (Gardner *et al.,* 1993). There is therefore still a need in the art for peptide mimetics that can incorporate a wide range of sidechain functions in all positions, that can be readily synthesised with control of chirality, and that have a wide range of conformations corresponding to those found in native peptides.

### OBJECT OF THE INVENTION

It is the object of the invention to provide novel compounds useful as, and useful for the synthesis of, conformationally constrained mimetics of biologically active peptides and proteins (peptide mimetics). In particular, the invention provides new compounds and methods for the synthesis of new peptide reverse turn mimetics that can display a wide range of sidechain functions at all sidechain positions, can be incorporated in a peptide sequence, can be readily synthesised, and have a variety of conformations.

### SUMMARY OF THE INVENTION

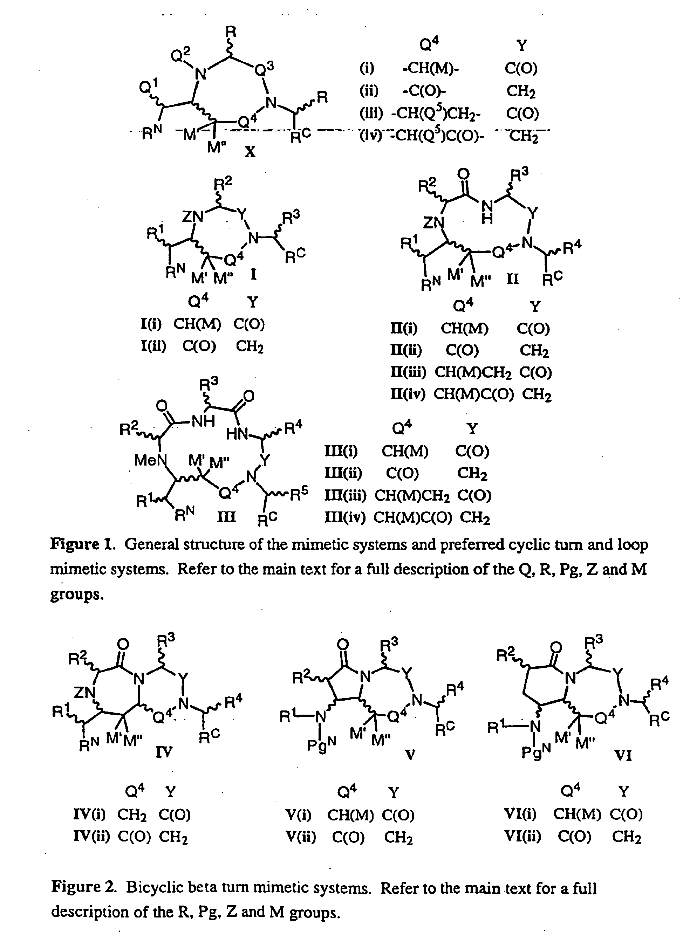

This invention describes novel compounds useful for the synthesis of peptide mimetics, and describes the use of these compounds for the synthesis of novel reverse turn mimetics. The reverse turn mimetics of the invention have the general structure **X,** or in a preferred embodiment the general structures **I-VI** (which are subsets of the general structure **X;** see below and Figures 1 and 2 on the attached sheets; the structures are fully described in the detailed description following this summary).

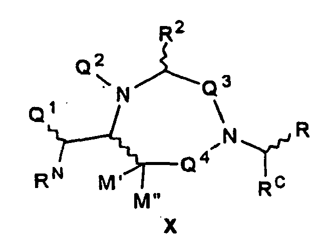

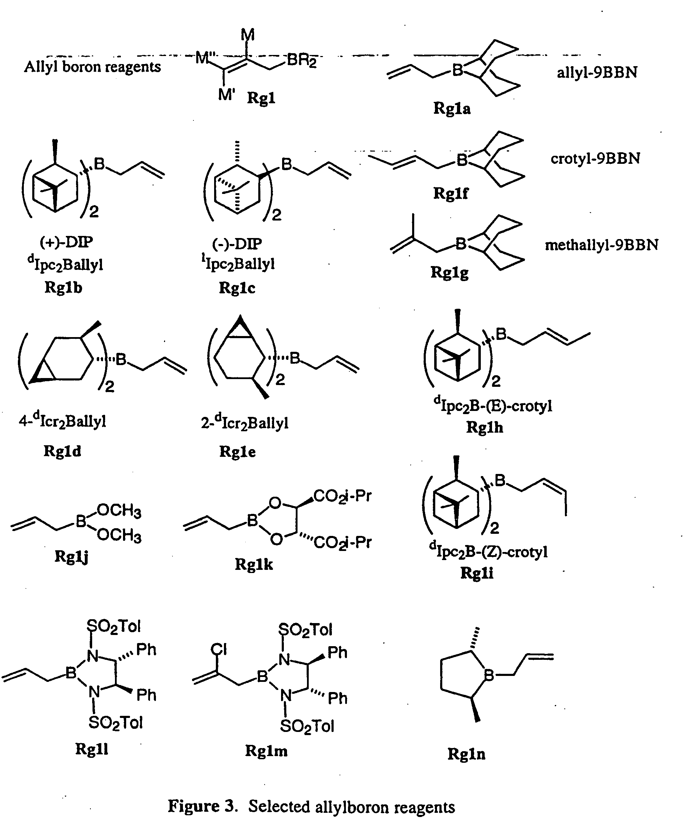

It has now been discovered that B-allyldialkylboranes (e.g. **Rg1a-i**, Figure 3) react with imines **3** (Scheme 1) to give the novel allyl amines **4a-d** in good yield and with a very high degree of chemo- and stereoselectivity. This is surprising because in contrast to these good results, allylation with the related B-allyldialkoxyboranes (e.g. **Rg1j,** Figure 3) or allylcopper or allylzinc reagents gave inferior results with racemisation and reaction at other functional groups. The reaction of imines **3** to form compounds **4a-d** and formation of the related compounds **5-8a-d** (all of which are made from compounds **4a-d)** forms the basis of the synthesis of all the compounds of the invention, and hence the invention. Thus the allyl amines **4a-d** are suprisingly valuable intermediates for the synthesis of new peptide mimetics, particularly reverse turn mimetics, enabling the synthesis of the significant variety of new reverse turn mimetics of the invention (having the general structure **X**), by the variety of different pathways described herein. All the mimetic systems of the invention can be incorporated into peptide sequences (i.e. they include amino and carboxy termini in addition to the sidechain functions), or if desired the amino and/or carboxy termini can be omitted from the mimetic.

As described above, there is a need for a wide range of different mimetics to better reproduce the wide range of conformations found in native reverse turns. The turn mimetics of the invention have a large variety of novel functionalised ring structures, each of these therefore having novel conformational characteristics. Furthermore, the structure and ring sizes of many of the turn mimetics make them well suited to the reproduction of the geometry of the more common native reverse turn conformations (those having d of 4.5Å to 6Å).

The synthetic methods described in this invention are generally superior to the prior art in terms of the capacity to include a wide range of side chain functions, in all the sidechain positions, without significant changes in the synthetic method; that is, the methods are more truly *generic.* In addition, the control of chirality in the synthesis of the mimetics of the invention is superior to the prior art - an important consideration in the elucidation of structure-activity relationships and the development of novel pharmaceuticals, and other commercially useful peptide mimetics, as diastereomeric mixtures are normally unsuitable and may be impractical or impossible to separate on a commercial basis. Furthermore, selective access to a range of different diastereomers for a particular mimetic with a given sequence provides a selection of different conformations. Thus in a mimetic with four chiral centres there are a total of 16 (2⁴) possible diastereomers - each having a different conformation. The methods of the invention allow for a high level of chiral control by using available chiral starting materials, non-racemising conditions and -diastereoselective reactions.

### DETAILED DESCRIPTION OF THE INVENTION

The peptide mimetics of this invention have the general structure X, shown below and defined as follows:- 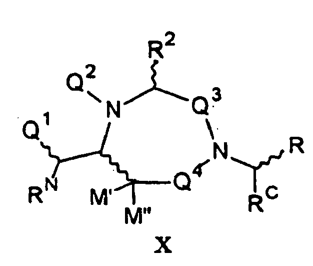 wherein R and R² and other R groups referred to hereinafter inclusive of R¹, R³, R⁴, Rⁿ⁺³ and Rⁿ⁺⁴ etc. unless otherwise indicated, are amino acid side chain groups, each independently chosen and therefore the same or different (two separate R groups in the same mimetic do not require a different suffix to indicate that they are independently chosen and can be the same or different). The definition of "amino acid side chain group" as used in this document is the same as the definition of "amino acid side chain moiety or derivative" as described in international Publication WO97/15577, pages 7-9 (Kahn, M) as follows.

As used herein, the term "amino acid side chain moiety" represents any amino acid side chain moiety present in naturally occurring proteins including the naturally occurring amino acid side chain moieties identified in Table 1. Other naturally occurring amino acid side chain moieties of this invention include the side chain moieties of 3,5-dibromotyrosine, 3,5-diiodotyrosine, hydroxylysine, γ-carboxyglutamate, phosphotyrosine and phosphoserine. In addition, glycosylated amino acid side chains may also be used in the practice of this invention, including glycosylated threonine, serine and asparagine.

**Table 1**

| Amino Acid Side Chain Moieties | |
|---|---|
| Amino Acid Side Chain Moiety | Amino Acid |
| -H | Glycine |
| -CH3 | Alanine |
| -CH(CH₃)₂ | Valine |
| -CH₂CH(CH₃)₂ | Leucine |
| -CH(CH₃)CH₂CH₃ | Isoleucine |
| -(CH₂)₄NH3⁺ | Lysine |
| -(CH₂)₃NHC(NH₂)NH₂⁺ | Arginine |
| 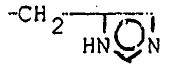 | Histidine |
| -CH₂COO⁻ | Aspartic acid |
| -CH₂CH₂COO⁻ | Glutamic acid |
| -CH₂CONH2 | Asparagine |
| -CH₂CH₂CONH₂ | Glutamine |
| 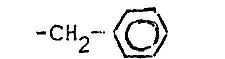 | Phenylalanine |
| 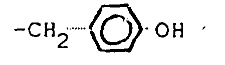 | Tyrosine |
| 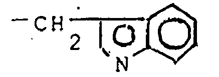 | Tryptophan |
| H | |
| -CH₂SH | Cysteine |
| -CH₂CH₂SCH₃ | Methionine |
| -CH₂OH | Serine |
| -CH(OH)CH₃ | Threonine |
| 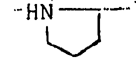 | Proline |
| 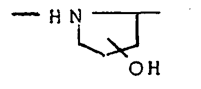 | Hydroxyproline |

In addition to naturally occurring amino acid side chain moieties, the amino acid side chain moieties of the present invention also include various derivatives thereof, including modifications and/or variations to naturally occurring amino acid side chain moieties. For example, the amino acid side chain moieties of alanine, valine, leucine, isoleucine and phenylalanine may generally be classified as lower chain alkyl, aryl, or aralkyl moieties. Derivatives of amino acid side chain moieties include other straight chain or branched, cyclic or noncyclic, substituted or unsubstituted, saturated or unsaturated lower chain alkyl, aryl or aralkyl moieties.

In the context of defining amino acid derivatives, "lower chain alkyl moieties" contain from 1-12 carbon atoms, "lower chain aryl moieties" contain from 6-12 carbon atoms and "lower chain aralkyl moieties" contain from 7-12 carbon atoms. Thus, the amino acid side chain derivative is selected from a C₁₋₁₂ alkyl, a C₆₋₁₂ aryl and a C₇₋₁₂ aralkyl, and more preferred from a C₁₋₇ alkyl, a C₆₋₁₀ aryl and a C₇₋₁₁ aralkyl.

Amino side chain derivatives in this invention further include substituted derivatives of lower chain alkyl, aryl, and aralkyl moieties, wherein the substituent is selected from one or more of the following chemical moieties: -OH, -OR, -COOH, -COOR, -CONH₂, -NH₂, -NHR, -NRR, -SH, -SR, -SO₂R, -SO₂H, -SOR and halogen (including F, Cl, Br and I), wherein each occurrence of R is independently selected from a lower chain alkyl, aryl and aralkyl moieties. Moreover, cyclic lower chain alkyl, aryl and aralkyl moieties of this invention include naphthalene, as well as heterocyclic compounds such as thiophene, pyrrole, furan, imidazole, oxazole, thiazole, pyrazole, 3-pyrroline, pyrrolidine, pyridine, pyrimidine, purine, quinoline, isoquinoline and carbazole. Amino acid side chain derivatives further include heteroalkyl derivatives of the alkyl portion of the lower chain alkyl and aralkyl moieties, including alkyl and aralkyl phosphonates and silanes. Amino acid side chain groups typically correspond to, those found in natural amino acids and derivatives and in common unnatural amino acids. Thus for glycine R = hydrogen; for alanine R = methyl; for phenylalanine R = -CH₂Ph; for homophenylalanine R = -CH₂CH₂Ph; for valine R = -CH(CH₃)₂; for leucine R = -CH₂CH(CH₃)₂; p-nitrophenylalanine R -CH₂((4-NO₂)Ph); naphthylalanine R = -CH₂-naphthyl etc. Also included are cyclic amino acid sidechains such as for proline, hydroxyproline and homoproline which involve a cyclization to the adjcent backbone nitrogen atom or the equivalent position, but only where this is possible (i.e. the amine or equivalent atom is not already substituted as part of the heterocyclic mimetic framework).

Z is normally hydrogen, methyl, ethyl, formyl or acetyl, and may alternatively be R or -CH₂R or -C(O)R where R is an amino acid side chain group, or alternatively Z is part of a cyclic amino acid side chain group joined to R² (for example to mimic a proline residue at position (i+1)). For II(i) referred to hereinafter, Z cannot be hydrogen due to compound instability.

R^{C} is the carboxy terminal part of the mimetic, typically -C(O)Pg^{C} or alternatively hydrogen or an amino acid side chain group R or -CH₂R.

Pg^{C} (and Pg^{C'} etc.) is a protecting group for carboxylic acid, typically including, alkoxy, benzyloxy, allyloxy, fluorenyl methyloxy, amines forming easily removable amides, or alternatively an appropriate cleavable linker to a solid phase support, or such a support itself, or alternatively hydroxy -OR, -NHR or remaining C-terminal portion of the mimetic system as described below.

R^{N} is the amino terminal part of the mimetic, i.e. -N(Z')Pg^{N},

Z' is normally hydrogen, alternatively methyl (to mimic an N-methyl amino acid residue at position (i)), or alternatively part of a cyclic amino acid side chain group joined to R¹ (for example, to mimic a proline residue at position (i)).

Pg^{N} (and Pg^{N'}) is a protecting group for amine, typically including Boc, Cbz, Fmoc, Alloc, trityl; or alternatively an appropriate cleavable linker to a solid phase support, or such a support itself, or alternatively hydrogen or R or -C(O)R where R is an amino acid side chain group, or alternatively part or all of the remaining N-terminal portion of the mimetic system, as described below.

M', M" are normally hydrogen, alternatively one or more may be C₁-C₄ alkyl (preferred methyl), chloro, C₁-C₄ alkoxy (preferred methoxy).

Q¹ = R¹ and Q² = Z; alternatively there is a cyclisation from Q¹ to Q² and then in preferred embodiments of the invention Q¹Q² = CH(R)C(O) or -CH₂CH(R)C(O)- or -CH₂CH₂CH(R)C(O)-. Q¹Q² can also be: -CH(R)CH₂- or -CH₂CH(R)CH₂- or -CH₂CH₂CH(R)CH₂- or -CH₂CH(R)- or -CH₂CH₂CH(R)- or -CH(R)CH₂CH₂- or -CH₂CH(R)CH₂CH₂- or -CH(R)CH₂C(O)- or -CH₂CH(R)CH₂C(O)-.

Q⁵ = hydrogen, C₁-C₄ alkyl, chloro or C₁-C₄ alkoxy and Q³ = Y or -C(O)NHCH(R)Y- or -C(O)ENHCH(R)Y-; or alternatively when Q³ = -C(O)N(Q⁵)CH(R)Y- Q⁵ is a covalent bond from the Q⁴ group to the nitrogen atom in Q³ (a cyclisation forming a bicyclic ring system).

Y is selected from the group consisting of C(O) and CH₂ and Q⁴ is selected from the group consisting of CHM¹, C(O), CH(Q⁵)CH₂ and CH(Q⁵)C(O) with the provisos that:
(i) Q⁴ = CH(M¹), Y is C(O);
(ii) Q⁴ = C(O), Y is CH₂;
(iii) Q⁴ = CH(Q⁵)CH₂, Y is C(O); and
(iv) Q⁴ = CH(Q⁵)C(O), Y is CH₂.

E=-(AA)ₙ- where n = 1, 2, 3, 4... (n = 1 to about 300, but more typically n is between 1 and 30) and AA is an amino acid residue (e.g. AA = -NHCH(CH₃)C(O)- for alanine); E is therefore a loop of n amino acids which are linked in a cycle by the rest of the mimetic system. The loop may also incorporate non-alpha amino acids, alpha dialkyl amino acids or any other amino acid which confers favourable properties on the mimetic system, for example increased binding affinity, or ease of detection, identification or purification. The invention, when used with such larger loops, is functioning as a covalent hydrogen bond mimic as generally described by Arrhenius *et al*. (Arrhenius *et al.*, 1987) and also in U.S. Patent 5807979 (Arrhenius *et al.*)*.*

Preferred embodiments of the invention are the structures **I-VI**, as ilustrated in Figures 1 and 2 and defined in Table 1:-

**Table 1**

| Mimetic | Q¹ | Q² | Q³ | Q⁵ |
|---|---|---|---|---|
| I | R¹ | Z | Y | - |
| II | R¹ | Z | -C(O)NHCH(R)Y- | M¹ |
| III | R¹ | Z | -C(O)NHCH(R)C(O)-NHCH(R)Y- | M¹ |
| IV | R¹ | Z | -C(O)N(Q⁵)CH(R)Y- | Q³ |
| V | -CH(R)C(O)Q² | Q¹ | Y | M¹ |
| VI | -CH₂CH(R)C(O)Q² | Q¹ | Y | M¹ |

Recursive entries of Q groups in Table 2 indicate a cyclisation - thus mimetics **V** and **VI** have a cyclisation between Q¹ and Q², and mimetic **IV** has a cyclisation between Q³ and Q⁵. In the Tables, the groups Q¹-Q⁵ and Y are as defined above, and the other groups are asdefined herein.

The compounds of this invention have been designed to allow for incorporation in a peptide or protein chain, or for covalent attachment to any molecule or group that may be useful for the enhancement of the biological activity, or other property, of the peptide mimetic. Thus the mimetics typically contain amino and carboxy termini independent of the sidechain functions. The term "remaining C- (or N-) terminal portion of the mimetic" is any group, molecule, linker, support, peptide, protein, nucleoside, glycoside or combination of these, covalently linked to the mimetic. Typically such remaining portions would be peptides or combinations of peptides and other mimetics, or compounds to facilitate detection or identification, or to improve the pharmacodynamics or other useful feature of the mimetic system.

In addition, any R group (an amino acid side chain group) may serve as an attachment point to a solid support, or to a linker to a solid support, or as a covalent attachment point for another molecule that may be useful for the enhancement of the biological activity, or other property, of the mimetic, as described above for the remaining C- or N-terminal portions of the mimetic.

The term "cleavable linker" and "solid phase support" are as defined in International Publication WO97/155

The use of a wavy line for one of the bonds at a chiral centre in the general structures **X** and **I-VI** and in the other structures in the Figures and Schemes indicates that the centre may be in either the (R) or (S) configuration, or be a mixture in any proportion of the (R) and (S) configurations. In most circumstances it is preferable to avoid mixtures of configurations unless the intention is to provide a mixture of diastereomers for example for the purpose of more efficient screening (by the use of a mixture) or for synthetic expediency. Chirality at the amino acid side chain positions in the compounds of the invention (e.g. at R¹ to R⁴) is controlled by the use of chiral starting materials (L or D amino acids) and the avoidance of synthetic conditions which cause racemisation. The configuration at chiral centres formed in the mimetic synthesis is dependent on several factors and can be controlled in several cases, but in other cases mixtures of diastereomers will result, which can potentially be separated by physical means. A significant advantage of the invention is the superior level of chiral control possible at the chiral centres in the mimetics.

### EXAMPLES OF PREFERRED EMBODIMENTS OF THE MIMETICS

γ-Turn mimetics **I(i)a, I(ii)a** (M, M'. M", Z and Z' = hydrogen):

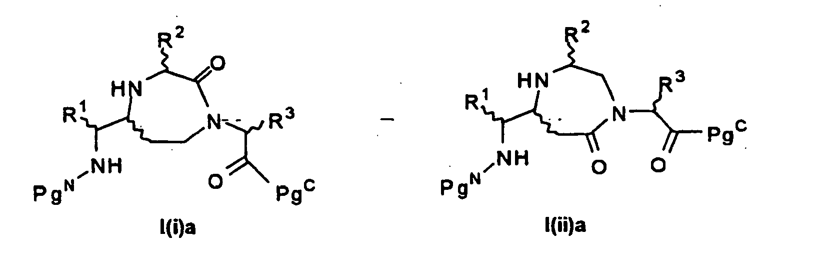

β-Turn mimetics **II(i)a, II(iii)a** (M, M', M" and Z' = hydrogen, Z = Me):

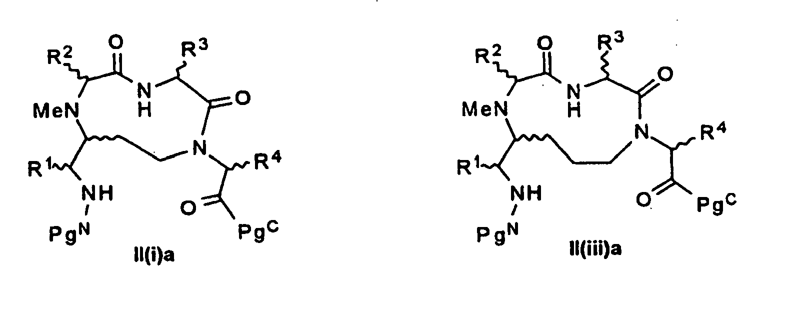

β-Bulge mimetics **III(i)a, III(iii)a** (M, M', M" and Z' = hydrogen, Z = Me):

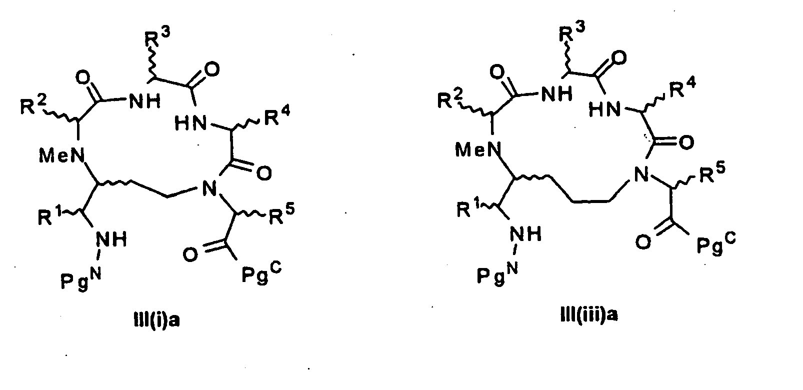

Bicyclic β-turn mimetics **IV(i)a, IV(ii)a** (M, M', M", Z and Z' = hydrogen):

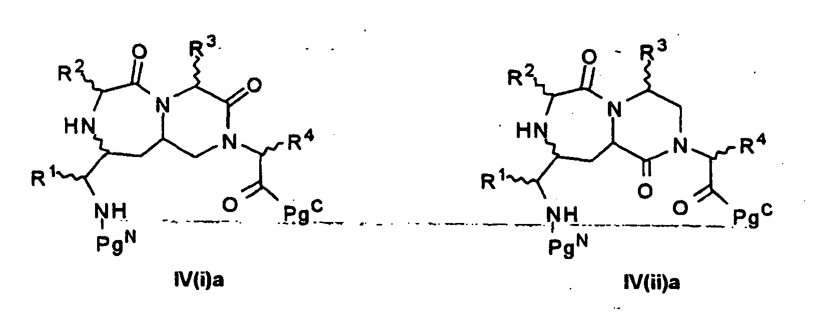

Bicyclic β-turn mimetics **V(i)a, VI(i)a, V(ii)a, VI(ii)a** (M, M'and M" = hydrogen)

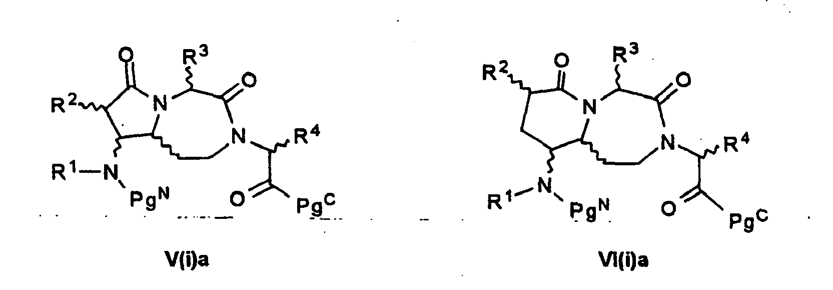

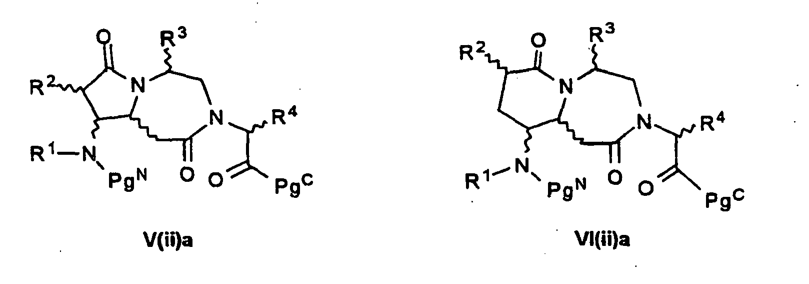

The synthesis of all the mimetics described in this specification may proceed initially by the same general synthetic procedure for formation of the common intermediates - reaction of imines **3** with allyl metal reagents **Rg1** (allyl boranes preferred) to give the allyl diamines **4,** which are new, as described in Scheme 1. The other compounds of Scheme 1 (i.e. **5-8**) may all be derived from the allyl diamines **4**, as described in Scheme 1 and in the comments below. The allylation reaction of imines **3**, which falls within the scope of the invention, is remarkable for its mildness and selectivity - allowing a wide range of functional groups to be present in the rest of the molecule, a very important consideration in the synthesis of peptide mimetics. Another important feature of the reaction of allylboranes with the imines **3** is that it proceeds in good yield (e.g. >50% isolated yield) in the sterically hindered general case where R¹ and R² are both not hydrogen - i.e. for all mimetics of dipeptides not containing glycine. Scheme 1 and all subsequent Schemes describe the preferred case of R^{N}=NHPg^{N} and R^{C}=C(O)Pg^{C} (Figures 1 and 2), analogous methods apply in the general case.

In relation to Scheme 1, preparation of the imines **3** is completed by condensation of an amino acid aldehyde (compound **1)** with an amine **(2a-d).** The aldehydes **1** may be prepared by either oxidative procedures from the corresponding N-protected amino alcohol, or reduction of an N-protected amino acid derivative (Fehrentz and Castro, 1983), the different approaches have been reviewed, (Jurczak and Golebiowski, 1989) (see also Goel et al., 1988, Org. Syn. 67 69). The amines **2a** are amino acid esters (or other acid protected amino acid derivatives), which are commercially available or may be synthesised by standard procedures from amino acids. Amines **2b-2d** are prepared by reductive amination of an amine **2a** and an amino acid aldehyde **1:**

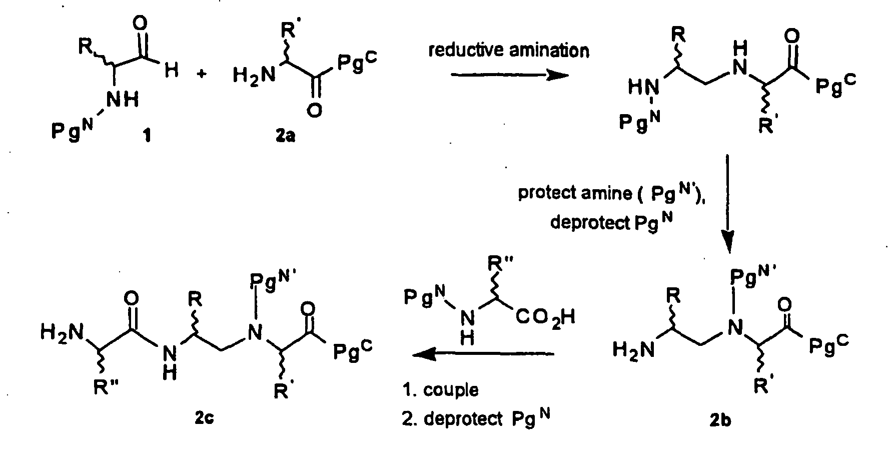

Amines **2d** are prepared by repeated coupling/deprotection steps (as in conversion of **2b** to **2c**), standard techniques of peptide synthesis.

The reductive amination procedure for the alkylation of amines by aldehydes is well established in the art. (See for example, Sasaki and Coy, 1987, Peptides **8** 119), the preferred reagents are sodium cyanoborohydride (Borch *et al.,* 1971; Hutchins and Natale, 1979; Gribble and Nutatits, 1985), or more preferred sodium triacetoxyborohydride in dichloroethane. (Abdel-Magid *et al.,* 1996).

Methods for the formation of amide bonds (coupling) are well established in the art. For coupling at more hindered amines the use of certain reagents, for example those based on 1-hydroxy-7-azabenzotriazole (Ehrlich *et al.*, 1993; Carpino *et al.,* 1994), or the use of amino acid fluorides (Carpino *et al.,* 1990; Wenschuh *et a*/., 1994) is advantageous.

Protecting strategies for the synthesis of peptides and peptide mimetics are well established in the art, for example a five dimensional orthogonal strategy was used by Hirschmann and co-workers in the synthesis of a somatostatin mimetic.(Hirschmann *et al*., 1996) A more general reference work on protection/deprotection is the monograph by Greene and Wuts.(Greene and Wuts, 1991).

The example syntheses described in this document use solution phase chemistry. The mimetics may also be synthesised by analogous solid phase techniques, or by a combination of solid phase and solution phase techniques, or the mimetics may be incorporated in normal solid phase peptide synthesis in the same way as a standard protected amino acid derivative. A review by Fruchtel and Jung (Früchtel and Jung, 1996) details the state of the art in solid phase organic synthesis (in 1996).

It will be clear to those skilled in the art that the mimetics of the invention, due to their generic methods of synthesis, are suited to the application combinatorial chemistry techniques (more specifically combinatorial organic synthesis) and certain associated identification and screening techniques. The application of combinatorial and associated technologies to drug discovery are well known in the art and have been reviewed, see for example papers by Gallop et al. and by Gordon *et al.,* and references therein, (Gallop *et al.*, 1994; Gordon *et al.*, 1994). Additionally, reference may be made to a review by Thompson and Ellman on the synthesis and application of small molecule libraries, and references therein (Thompson and Ellman, 1996).

The imines **3** form rapidly at room temperature on mixing of the amine and aldehyde in an appropriate solvent, e.g. CH₂Cl₂ or diethyl ether, with liberation of water. The water is removed with a drying agent, e.g. dried MgSO₄, which is subsequently removed by filtration. The imines are then reacted with an allyl metal reagent (**Rg1**) to give, after work-up, compounds **4** (Scheme 1).

In relation to reagents **Rg1**: standard allyl organometals, such as allyl magnesium bromide, are unsuitable for reaction with imines **3** due to a lack of selectivity for the imine function over the carboxylic acid derived groups (esters, amides) also present in **3**. Allyl copper and zinc reagents have been used in selective reactions with imines (Bocoum et *al*., 1991; Basile *et al.,* 1994) but in the case of imines **3** these reagents result in extensive racemisation at the α-imine chiral centre, and attack esters present in the imine to a significant extent. While some of the desired target **4** may be produced by many allyl metal reagents on reaction with **3,** the reaction product typically contains a mixture of four diastereomers and also by-products from reaction at the carboxylic acid derived groups (especially esters). In contrast to these results, reaction of the imines **3** with allyl boranes, such as B-allyl-9-borabicyclo[3.3.1]nonane (allyl-9-BBN), **Rg1a**, gives excellent results and reasonable diastereoselectivity (>50% isolated yield based on crude aldehyde, and ~80:20 diastereoselectivity where R¹ is not H).

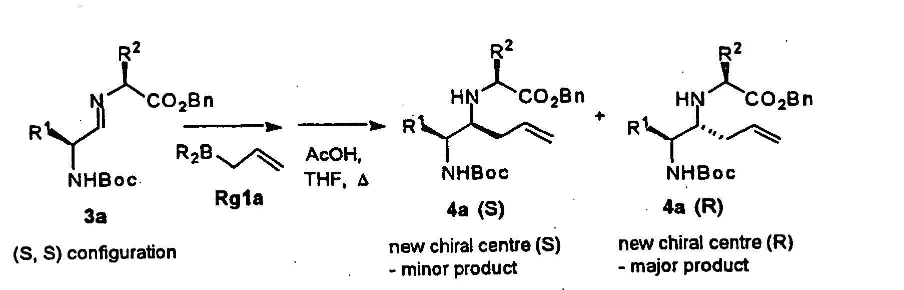

By the use of allyl trialkylboranes with appropriate chiral alkyl groups such as B-allyl-diisopinocampheylborane (allyl-DIP, **Rg1b** and **Rg1c**)**,** or the diisocaranylboranes **Rg1d-e** it is possible to produce give only the major product (one diastereomer, >99:1) in good yield and purity. The configuration at the new stereocentre was determined to be (R) when using aldehyde derived from natural (S) configuration amino acids, and the stereocontrol exerted by the aldehyde chiral centre was dominant over the effect of chiral boron ligands and over the effect of the other amino acid chirality in all cases examined. The (+)DIP reagent **Rg1b** gave higher diastereoselectivity on imines derived from natural (S) configuration aldehydes than **Rg1c** (from (-)DIP). The purity of the allylation products **4a** may also be improved by the removal of the ester protecting group Pg^{C} to give a crystalline amino acid which can be recrystallised (e.g. from ethanol/water) to the desired level of purity and then reprotected. 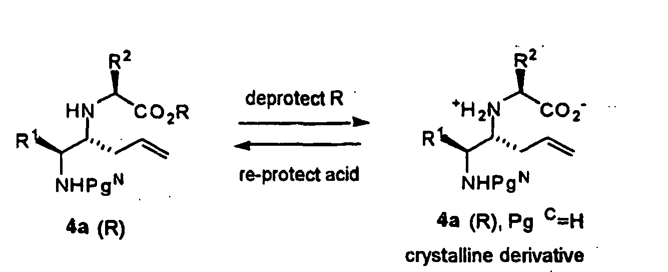 The use of crotyl **(Rg1f, Rg1h-i),** methallyl **(Rg1g)** or other substituted allyl derivatives leads to bridge substituted mimetics (mimetics where at least one of M, M' and M" is not hydrogen) with further opportunities for stereocontrol. The less reactive allyl boronate allyldimethoxyboron **(Rg1j)** was found to give inferior results (significant epimerisation at Cα(i)) compared to the allyltrialkylboranes. Many N allylboronate and related reagents (e.g. **Rg1k-m**) are described in the literature, and some of these may be more effective than allyldimethoxyboron for the conversion of **3** to **4.** Selective reactions using allylic metals have been reviewed by Yamamoto and Asao, Tables **IV** and **V** in the review (pp 2224-2230) list a wide variety of allyl boron reagents.(Yamamoto and Asao, 1993) The preparation of allyl-9-BBN and other allyltrialkylboranes has been described by Brown and coworkers (Kramer and Brown, 1977; Brown and Jadhav, 1983; Brown and Jadhav, 1984; Brown and Bhat, 1986; Brown, Randad *et al*., 1990) Allyltrialkylboranes may also be prepared by the reaction of the corresponding B-chloro or B-methoxy derivative with an allylmagnesium bromide (-78 °C, diethyl ether), and reacted in situ with the imine (Yamamoto and Asao, 1993). The imines **3** formed from two non-glycine derivatives (i.e. R¹ and R² not H) are significantly hindered about the imine nitrogen, and the use of bulky boron ligands (such as diisopinocampheyl) can reduce the reaction yield. For high yield and selectivity smaller chiral B-allyl compounds, e.g. those based on 2,5-dimethylboracyclopentane are preferred (e.g. **Rg1n,** Figure 3).

In relation to protection and deprotection of compounds **4** and **5**: addition of formaldehyde solution to **4** results in the rapid formation of imidazolidines **5;** the relative configuration in the major allylation products **4** results in a 4,5-cis-substituted imidazolidine **5.** This protection strategy is important for further reaction of these compounds. The protecting group is removed by treatment with aqueous acid (e.g. aqueous methanolic acetic acid).

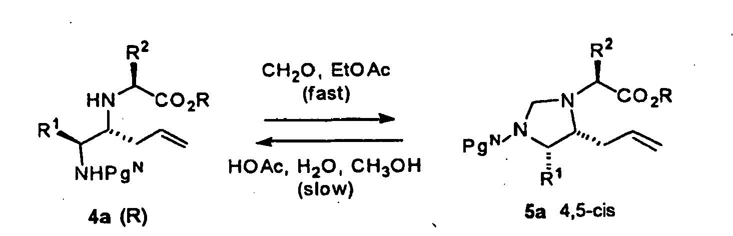

A similar protection system is the dibenzyltriazone group of Knapp and co-workers, (Knapp *et a*/*.,* 1992) the paper describes other deprotection conditions. An alternative deprotection method involves the hydrogenation of the imidazolidine system to an amine N-methyl group (40psi H2, Pd-C, MeOH, 48hrs), a conversion that can be used to give mimetics where Z = Me.

In relation to oxidation of alkenes **5:** acids **6** can be synthesised directly by oxidative cleavage of the alkenes **5,** e.g. by RuCl₃/NalO₄; aldehydes/ketones **8** may be synthesised directly from **5** by ozonolysis (for oxidation methods see for example the monograph by Hudlicky (Hudlicky) and references therein), but this process is not sufficiently selective and results in over-oxidation and the formation of other by-products. Preferred is the two step process of dihydroxylation (OsO₄, N-methylmorpholine-N-oxide (NMO),tBuOH/water) (VanRheenen *et al.,* 1976; Ray and Matteson, 1980) to **7** followed by oxidative cleavage (Pb(OAc)₄ in benzene or H₅IO₆ in THF).(Hudlicky, 1990) Examination of the products of the oxidation reactions led to the surprising discovery that cleavage with Pb(OAc)₄ resulted in isomerised product with the 4,5-substituents now trans, not cis as in the starting material. It was further discovered that oxidation of the diol with H₅IO₆ in dry THF resulted in retention of the 4,5-cis configuration in the aldehyde product **8**. The cis aldehydes can also be isomerised to the trans by treatment with catalytic acid, e.g. HCl in CHCl₃.

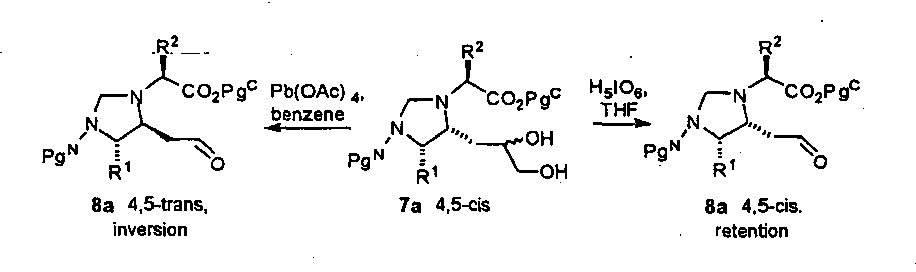

These important discoveries now allow selective access to all of the eight possible diastereomers of the aldehydes **8** and the acids **6,** and therefore control of the majority of the chirality in all the mimetic systems described in the invention.

In relation to the oxidation of aldehydes **8** to acids **6:** many oxidation reagents may effect this conversion, e.g. pyridinium dichromate.(Hudlicky, 1990) Glycols **7** may also be oxidised directly to acids, e.g. by RuCl₃/NaIO₄. In relation to reduction of acids **6** to aldehydes 8: carboxylic acids **6** can be converted to aldehydes by the same general methods used for the formation of protected amino aldehydes described above.(Jurczak and Golebiowski, 1989). The carboxylic acid can be selectively reduced to the alcohol in the presence of carboxylic esters by the use of borane (Brown and Krishnamurthy, 1979), then oxidised to the aldehyde as previously described.(Jurczak and Golebiowski, 1989)

In relation to **Scheme 2:** Aldehydes/ketones **8** undergo reductive amination with amino esters **9** by the methods previously described. The preferred method is NaBH(OAc)₃ in dichloroethane (room temperature). Surprisingly, it was discovered that the reductive amination of 4,5-cis imidazolidine aldehydes **8** resulted in the formation of the 4,5-trans amines **10** (~9:1 trans:cis). This isomerisation reaction is rapid (much faster than that of aldehydes **8**) as the reductive amination reaction is complete in only a few minutes: It was further discovered that the isomerisation reaction could be prevented by the pre-formation of the imine between the aldehyde **8** and amine **9** (in MeOH, 2-4 h at room temperature) with rigorous exclusion of acid, followed by reduction with sodium borohydride to give the cis amine **10** from the cis aldehyde. This discovery allows the selective synthesis of either the 4,5-cis diastereomer or 4,5-trans (9:1 with cis) diastereomer of the amines **10** starting from the 4,5-cis aldehyde **8.**

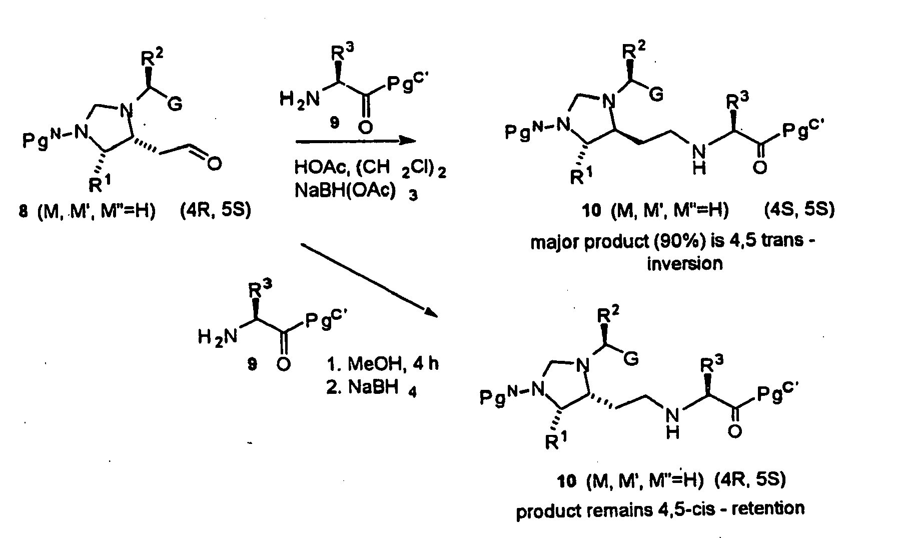

It is important to appreciate that the methods described above allow the selective synthesis of all sixteen relative and absolute diastereomers of compounds **8** and **6,** and all thirty two diastereomers of compounds **10.** The ability to selectively synthesise these diastereomers is a significant advantage of the invention.

In relation to **Scheme 3:** Deprotection of **10** is by standard methods consistent with the overall protecting strategy, as previously discussed. Many coupling agents are suitable for effecting the cyclisation of **11** to **12,** typical conditions: THF, BOP or HBTU or HBTU. EtN(*i*-Pr)₂ (DIEA). The imidazolidine group is then deprotected (as previously described) by hydrogenation (MeOH, H₂-Pd/C) when Z = Me, and by hydrolysis (H⁺, H₂O) for Z = H (other Z groups may be introduced by acylation or alkylation of the deprotected secondary amine).

In relation to **Scheme 4:** Deprotection and cyclisation of **6b** to **13, 14** and I(ii): - standard deprotection and coupling (cyclisation) methods are used. Other conversions are as previously described.

In relation to **Scheme 5:** As previously discussed, coupling reactions to relatively hindered (usually secondary) amines often require the use of specialised coupling conditions such as acid fluorides **15,** as described by Carpino *et al*. (Carpino *et al.,* 1990; Wenschuh *et al.,* 1994) Protecting groups Pg^{N'} and Pg^{C'} (in **16**) are typically benzyloxycarbonyl (Cbz) and benzyl ester, simultaneously deprotected by hydrogenation (0.1 M HCl in EtOH, H₂-Pd/C), cyclised using the BOP coupling reagent in THF or DMF, followed by conversion (deprotection) of the imidazolidine group to N-Me by hydrogenation as previously described.

In relation to **Scheme 6:** Standard deprotection/ coupling conditions as previously described.

In relation to **Scheme 7:** Where R⁴ is a branched amino acid side chain (such as in Valine) then the coupling of **6a** and **20** may require the use of HATU or other system suitable for a hindered coupling when bulky sidechain groups are present, as previously discussed. Conditions and protecting groups for the conversion of **21** to **19** are the same as for the conversion of **16** to **II(i),** Scheme 5.

In relation to **Scheme 8:** Hydroboration of alkenes is well known in the art, see for example monographs by Brown (Brown, 1975; Pelter *et al.,* 1988) The resulting alkyl boranes can be oxidised to alcohols (using alkaline hydrogen peroxide, or in a preferred embodiment using trimethylamine oxide, or other amine oxide, to form the borate with subsequent liberation of the alcohol by transesterification) (Soderquist and Najafi, 1986). Alternatively, treatment of the borane with acid dichromate or, in a preferred embodiment, with pyridinium chlorochromate (PCC) gives the aldehyde (Brown *et al.,* 1980; Brown *et al*., 1986). The aldehydes so formed may be reductively aminated on to amines **9** by the methods previously described.

In relation to **Schemes 9-11:** Standard synthetic techniques, previously described.

Methods for the synthesis of beta bulge (n=1, **lll(i-iv))** and higher loop mimetics (n>1), follow the corresponding methods for the synthesis of beta turn mimetics **II(i-iv)**. Appropriate protecting groups are chosen so that extra residues can be added to the system prior to cyclisation, as illustrated in Scheme 11 for the synthesis of a **III(i)** mimetic.

In relation to **Scheme 12:** Conversion of 1,2-diols **7** to epoxides **29** (dehydration) may be achieved with a number of reagents, for example triphenylphosphine and a dialkylazodicarboxylate (the Mitsunobu reagents) (Carlock and Mack, 1978; Robinson, Barry *et al.,* 1983) or TsCl/NaOH/PhCH₂NEt₃⁺ Cl⁻.(Szeja 1985). The epoxides **29** alkylate amines **9** on warming in ethanol or DMSO solution to give the amino alcohols **30.** The alcohol may then be oxidised to the ketone **32** by the use of TPAP (tetrapropylammonium perruthenate) with N-methylmorpholine-N-oxide in CH₂Cl₂/acetonitrile by the method of Griffith and Ley (Griffith and Ley ,1990). For **32** typically Pg^{N'}=Cbz and Pg^{C'}=O-benzyl, then by the use of catalytic hydrogenation conditions (EtOH, H₂-Pd/C) the protecting groups are both removed and intramolecular reductive amination of the free amine to the ketone occurs to give **33.** Coupling using the BOP reagent (or other suitable conditions) followed by deprotection of the imidazolidine group as previously described gives the bicyclic mimetic **IV(i)**. Alternative syntheses are possible with the use of mild oxidising reagents to convert the glycols to carbonyl compounds, followed by reductive amination (Frigerio and Sangostino, 1994).

In relation to **Scheme 13**: 1,2 diols can be oxidised without carbon-carbon bond cleavage by the use of certain mild reagents e.g. IBX (Frigerio and Sangostino, 1994). Conversion of **35c** to **36** proceeds by intramolecular reductive amination, or alternatively **35a** can be reductively aminated onto **2b,** as indicated. Reductive amination, coupling and deprotection details are as previously described.

The syntheses for the bicyclic turn mimetic systems **V** and **VI** are accomplished from the corresponding turn mimetic systems **I**, where the R¹ side chain group is derived from an aspartic acid (**V**) or glutamic acid (**VI**) derivative.

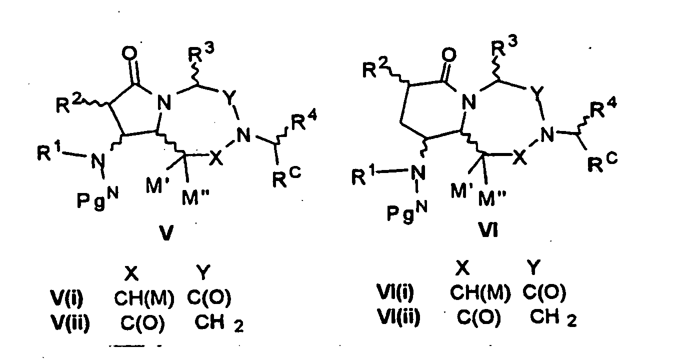

The synthesis of mimetics **V** and **VI** thus proceeds as in Scheme 1, with the aldehyde component 1 (Scheme 1) being of the form **1d** or **1e** (Scheme 14), with the R and Pg groups as previously defined. The synthesis follows the synthesis of turn mimetic systems **I**, and is completed by the method illustrated in Scheme 15.

In relation to the preparation of alkylated aspartic and glutamic acid derivatives **1d** and **1e** the alkylated derivatives **39-42** can be prepared by a number of methods known in the art. Selected methods are summarised in Schemes 16 and 17. Rapoport and co-workers have developed methods for the selective alkylation of N-phenylfluorenyl protected aspartic and glutamic acid derivatives (Koskinen and Rapoport; 1989; Wolf and Rapoport, 1989). A review by Sardina and Rapoport, and references contained therein, describe several methods for the synthesis of alkylated aspartic and glutamic acid derivatives (Sardina and Rapoport. 1996). Derivatives **39-42** are converted to aldehydes **1d** and **1e** by the methods previously described for for the preparation of aldehydes **1**.

The use of standard chemical techniques, in particular the Arndt-Eistert homologation reaction (Meier and Zeller, 1975) and reductions of carboxylic acids to aldehydes (Jurczak and Golebiowski, 1989), and also the synthesis of ketones C(O)R from amides -C(O)N(OMe)Me (Nahm and Weinreb, 1981), to modify the aspartic and glutamic acid or their alkylated derivatives, or the use of similar derivatives of non-natural amino acids, such as homo-glutamic acid, enables the synthesis of the other compounds of the invention in which -Q¹Q²- (in the general structure X) forms part of a cyclic system, defined as: -Q¹Q²- = -CH₂CH₂CH(R)C(O)- (from sidechain alkylated homoglutamic acid); -CH(R)CH₂- (from aspartic acid by reduction of the carboxylate and reductive amination); -CH₂CH(R)CH₂- (from glutamic acid by reduction of the carboxylate and reductive amination); -CH₂CH₂CH(R)CH₂- (similarly from homoglutamic acid); -CH₂CH(R)-(from an aspartic acid sidechain ketone -CH₂C(O)R by reductive amination); -CH₂CH2CH(R)- (from a glutamic acid sidechain ketone -CH₂CH₂C(O)R by reductive amination); -CH(R)CH₂C(O)- (post-alkylation sidechain homologated aspartic acid); -CH₂CH(R)CH₂C(O)-(post-alkylation sidechain homologated glutamic acid); -CH(R)CH₂CH₂- or -CH₂CH(R)CH₂CH₂- (from reductive amination of reduced post-alkylation sidechain homologated aspartic acid or glutamic acid derivatives).

In relation to **Scheme 18:** An alternative procedure for the synthesis of intermediate compounds **10** (or equivalent) can be used in the case where R¹ is hydrogen and M, M' and M" are also hydrogen, as described in **Scheme 18**. Compound **49** is available commercially with certain N-protecting groups or can be made by coupling N-protected glycine with N,O-dimethylhydroxylamine. Reaction with vinylmagnesium bromide in analogy to the general procedure of Rapoport and co-workers (Cupps *et al.*, 1985; Boutin and Rapoport, 1986) results in formation of the α, β-unsaturated ketone **50.** Conjugate addition of an amino acid ester **9** (0 °C, THF) results in the formation of aminoketones **51** which can be N-protected by standard procedures to form ketones **52** before reductive amination of an amino acid ester **9** under the conditions described by Abdel-Magid *et al*. (Abdel-Magid *et al.*, 1996) (NaBH(OAc)₃, dichloroethane) to form **54**. Deprotection to **55** and coupling gives the turn mimetics **I(i)a** (where R¹=H) as indicated. Alternatively the aminoketones **51** can be acylated with an amino acid fluoride **15** to give compounds **53** which can be deprotected and cyclised (by reductive amination) by hydrogenation in mild acid conditions (H₂/Pd-C, 0.1 M HCl in EtOH). The reductive amination-cyclisation is diastereoselective, only one diastereomer of the mimetics **I(i)a** were formed from **53,** with the configuration at the new stereocentre controlled by the R² stereocentre. The (S) configuration at R² gives (S) at the new centre. In contrast, the reductive amination to form amines **54** proceeds with lower stereoselectivity (∼3:1) with the major diastereomer having the (R) configuration when R² is (S). These discoveries provide further opportunity for stereocontrol in the synthesis of the turm mimetics. Deprotection of compounds **54** and reaction with formalin in THF is an alternative method for synthesis of compounds **10** (R¹=H), as described in Scheme **18.**

### EXAMPLE SYNTHESES

### Example (A). Synthesis of a turn mimetic I(i) by the general procedure

A mimetic for the sequence HTyr-Gly-Gly-Phe, which is found in the enkephalins, was synthesised with a turn mimetic based on the Tyr-Gly-Gly tripeptide: Similar mimetics have shown activity at opiate receptors (Huffman, Callahan *et al.,*1988*;* Huffman *et al.,* 1989).

The synthesis is summarised in the following scheme:-

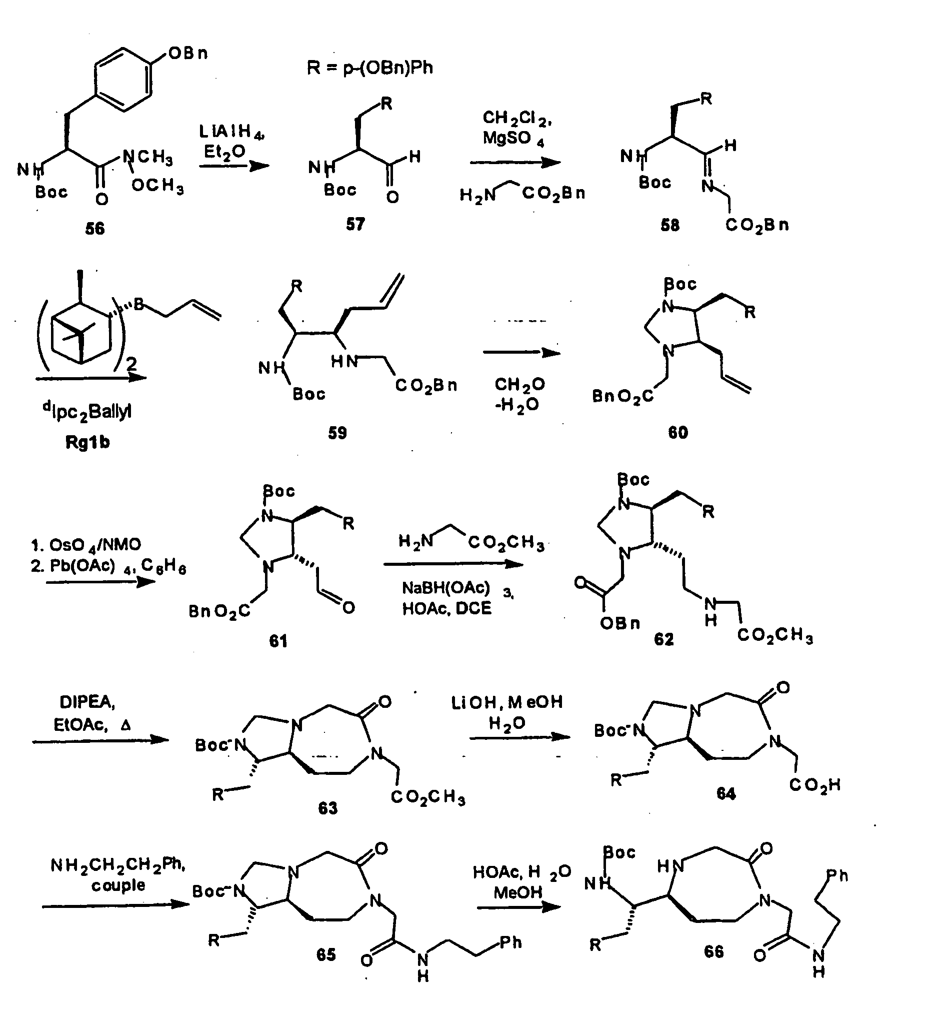

### Preparation of 56:

The amide **56** was synthesised from commercially avaliable Boc-Tyrosine(OBn)OH by coupling- with -N,O-dimethylhydroxylamine hydrochloride, 1 equivalent, in DMF/CH₂Cl₂ (1:5) using HBTU reagent (1 eq.) and DIEA (2 eq.) at room temperature. The CH₂Cl₂ was evaporated *in vacuo* and the residue partitioned between diethyl ether and aq. NaHCO₃. The aqueous layer was separated and the ether layer washed in turn with 1 M HCI (x2), aq. NaHCO₃, brine, and then dried over MgSO₄. Filtration and removal of the solvent *in vacuo* left the product amide **56** as a white crystalline solid in >90% yield. Further purification was carried out by silica gel chromatography eluting with ethyl acetate in petroleum ether, or by recrystallisation from ether **¹H NMR** (300 MHz, CDCl₃): δ7.46-7.28, 5H, m, OBn; 7.08, 2H, d, J=8.5 Hz, Tyr Ar; 6.90, 2H, d, J=8.5 Hz, Tyr Ar; 5.15, bd, J=8 Hz, NH; 5.04, 2H, s, )OCH₂Ph; 4.91, 1 H, bm, Phe□; 3.65, 3H, s, OCH₃; 3.16, 3H, bs, NCH₃; 3.00, 1H, dd, J=6, 13.5 Hz, Phe□; 2.83, 1 H, dd, J=7, 13.5 Hz, Phe; 1.40, 9H, s, Boc. **¹³C NMR** (75 MHz, CDCl₃): 172.3; 157.6, Tyr Ar-O; 155.1, carbamate; 137.0 ipso; 130.4; 128.8; 128.5; 127.8; 127.4; 114.7; 79.5, tBoc; 69.89, OCH₂Ph; 61.43, Tyr; 51.55, OCH₃; 37.89, NCH₃; 32.00, Tyr; 28.26, Boc.

### Preparation of 57:

The aldehyde **57** was prepared by the method of Fehrentz and Castro (Fehrentz and Castro, 1983) as follows: to a stirred solution of 4.2 g of amide **56** in 100 mls of anhydrous diethylether cooled to 0°C was added 0.51 g lithium aluminium hydride. After 10 minutes a solution of 1.5g NaHSO₄ in 30 mls of water was added. The reaction mixture was diluted with more ether and washed with 1 M HCl, saturated aqueous sodium bicarbonate and brine and dried over magnesium sulphate. The volatiles were removed under reduced pressure to give a waxy solid which was recrystallised from cold ether/hexane to give 2.6 g (72%) of 57 as a white solid. **¹H NMR** (300 MHz, CDCl₃): 9.62, 1H, s, aldehyde; 7.50-7.25, 5H, m, Ar(OBn); 7.10, d, J=8 Hz, Ar(Tyr); 6.93, 2H, d, J=8 Hz, Ar(Tyr); 5.10, 1H, b, NH; 5.05, 2H, s, OCH₂Ph; 4.39, 1H, q, J=7 Hz; Tyr; 3.06, 2H, d(ABX), J=7 Hz, Tyr; 1.44, 9H, s, Boc. **¹³C NMR** (75 MHz, CDCl₃): 199.6; 157.8, TyrOAr; 155.3, carbamate; 136.9, ipso; 130.3; 128.5, 127.9, 127.4: ArCH; 115.0, ArCHTyr; 80.08, tBoc; 69.69, OCH₂Ph; 60.82, Tyr; 34.51, Tyr; 28.22, Boc.

### Preparation of 58:

The imine **58** was formed by the reaction of the aldehyde **57** (1.4 g) with one equivalent of glycine benzyl ester in 10ml CH₂Cl₂ (stir at room temperature 1 h) the water formed was removed with magnesium sulphate which was then removed by filtration.

**¹H NMR**. (300 MHz, CDCl₃): 7.68, 1H, s, imine; 7.49-7.30, 10H, Ar; 7.15, 2H, d, J=8 Hz, TyrAr; 6.92, 2H, d, J=8 Hz, TyrAr; 5.67, 1H, bd, J=6 Hz, NH; 5.20, 2H, s, OCH₂Ph; 5.05, 2H, s, OCH₂Ph; 4.51, 1H, bm, Tyr□; (4.26, 4.22), 2H, AB, J=15.5 Hz, Gly; 3.15, 1H, bdd, J=5.0, 13.5 Hz, Tyrb; 2.93, 1H, dd, J=8.0, 13.5 Hz, Tyrb; 1.48, 9H, s, Boc. **¹³C NMR** (75 MHz, CDCl₃): 169.3; 167.4, CH imine; 157.5; 155.1; 136.9, 135.3: 2x ipso; 130.4, CHAr; 128.8, Tyr ipso; 128.44, 128.39, 128.26, 128.19, 127.76, 127.29, 114.65: ArCH; 79.22, tBoc; 69.81, TyrOCH₂Ph; 66.60, GlyOCH₂Ph; 60.48, Tyr; 54.73, Gly; 37.97, Tyr; 28.23, Boc.

### Preparation of 59:

A 0.5 molar solution of allyl borane reagent ^{d}Ipc₂Ballyl (**Rg1b**) was prepared by the addition of allylmagnesium bromide to one equivalent of (+)DIP-Cl in anhydrous diethyl ether under dry nitrogen. Brown and Jadhav, 1983). The solution of imine **58** in CH₂Cl₂ was stirred and cooled to -78°C under dry nitrogen and one equivalent of the previously prepared ^{d}Ipc₂Ballyl solution added. The mixture was allowed to warm gradually to room temperature (overnight). The volatiles were removed under reduced pressure and the residue dissolved in THF and 1 ml of glacial acetic acid added. The mixture was refluxed overnight and then the volatiles removed under reduced pressure. The crude product was dissolved in CH₂Cl₂ / petroleum ether and the precipitate filtered off. The residual oil was chromatographed on flash silica eluting with ethyl acetate / petroleum ether to give 1.3 g (60% yield based on **57**) of **59**. TLC 1:2 EtOAc:light pet. Rf=0.40. **¹H NMR** (300 MHz, CDCl₃): 7.48-7.30; 10H, Ar; 7.13, 2H, d, J=8.5 Hz, TyrAr; 6.91, 2H, d, J=8.5 Hz, TyrAr; 5.84, 1H, m, vinyl CH; 5.17, 2H, s, TyrOCH₂Ph; 5.16, 2H, m, vinyl CH₂; 5.05, 2H, s, GlyOCH₂Ph; 4.90, 1H, bd, J=8.5 Hz, NHBoc; 3.95, 1 H, bm, Tyr; 3.54, 2H, s, Gly; 3.82, 1H, dd, J=4.5, 14.4 Hz, Tyr; 2.73, 3H, be; NH(amine), Tyr; CH(homoallyl); 2.28, 2H, m, allyl; 1.35, 9H, Boc. **¹³C NMR** (75 MHz, CDCl₃): 172.1; 157.3; 155.6; 137.1, 135.4: ipso; 134.9, CHvinyl; 130.6, ipsoTyr; 130.0, 128.5, 128.4, 128.3, 127.8, 127.3: ArCH; 117.8, CH₂vinyl; 114.7, TyrArCH; 79.05, tBoc; 69.90, TyrOCH₂Ph; 66.51, GlyOCH₂Ph; 59.38, Tyr; 53.46, CH; 49.28, Gly; 35.44: coincident allyl carbon and Tyr; 28.20, Boc. Mass Spectrum (ISMS) m/z 545.1 (MH⁺), calculated for C₃₂H₄₅N₃O₅: 544.

### Preparation of 60:

The amine **59** (930 mg, 1.7 mmol) was dissolved in ethyl acetate (15 mL) and 37% aq. formaldehyde solution added (1 mL). The solution was stirred vigorously at room temperature for 1 h (or until the reaction was complete) and then diluted with ether (100 mL) and washed in turn with aq. NaHCO₃, water (x3), brine and then dried (MgSO₄). Removal of solvent *in vacuo* left an approximately quantitative yield (950 mg) of the crude product 60 which was used in the next reaction or further purified by flash chromatography eluting with 10-15% ethyl acetate in light petroleum. TLC 33%EtOAc:light pet. Rf=0.56. The NMR spectra were quite broad in CDCl₃, amide rotamers were present in the approximate ratio 2:1. **¹H NMR** (300 MHz, CDCl₃): 7.50-7.27, 10H, m's, Ar; 7.09, 2H, m, Ar; 6.90, 2H, d, J=8.5 Hz, Ar; 5.64, 1H, bm; vinyl CH; 5.19, 2H, s, OCH₂Bn; ∼5.1, 2H, m, vinyl CH₂; 5.05, 2H, s, OCH₂Bn; 4.59, 1H, bm, ring NCH₂N(a); 4.17, 1H, bm, ring NCH₂N(b); 4.06, 1H, bm, Tyr; 3.70, 1H, d, J=17 Hz, Gly(a); 3.42, 1H, bd, J=17 Hz, Gly(b); 3.16, 1H, bm, TyrC'H(ring); 2.84, 2H, bm, Tyr; 2.31, 2H, m, allylCH₂; 1.38, ∼3H, bs, Boc minor rotamer; 1.19, ∼6H, s, Boc major rotamer. **¹³C NMR** (75 MHz. CDCl₃): (peaks due to the carbamate rotamers are placed in parentheses, major rotamer first) 169.8 (ester); 157.2 (tyrosine O-ipso); (153.1, 152.8) carbamate; 137.2 (ipso); 135.4 (ipso); 134.2 (CH vinyl); 131.3 (ipso); 130.5, 128.5, 128.4, 128.3, 127.8, 127.4, 127.3, 126.9: ArCH; 117.5 (vinyl CH₂); 114.7 (2xTyrArCH); 79.52 (Boc tertiary); 69.93 (CH₂); 66.95 (CH₂); 66.46 (CH₂); 64.27 (CH); (59.65, 58.76) (CH); 51.60 (CH₂); 34.34 (CH₂), (32.20, 31.93) (CH₂); (27.93, 28.25) (Boc 3xCH₃). Mass Spectrum (ISMS) m/z 557.1 (MH⁺), calculated for C₃₄H₄₀N₂O₅: 556 fragments (OR 60): 501.1, (-tBu).

### Preparation of 61:

To 220 mg of **60** was added 60 mg of N-methylmorpholine-N-oxide (NMO), 40 mg of a 2.5% (by weight) solution of osmium tetroxide in t-butanol, 4 mls of *t*-butanol and 0.5 mls water. The mixture was stirred at room temperature until the reaction was complete (about 24 hours). 3 mls of 10% NaHSO₃ was added, the solution stirred for 10 minutes, then neutralised with sodium bicarbonate, diluted with brine and extracted three times with ethyl acetate. The combined extracts were washed with brine and dried over magnesium sulfate. Removal of volatiles under reduced pressure gave the crude diol in good yield as an oil which could be used in the next reaction or purified if required by chromatography on silica gel eluting with ethyl acetate. Mass Spectrum (ISMS) m/z 591.3 (MH⁺), calculated for C₃₄H₄₂N₂O₇: 590.

Oxidation of diol using Pb(OAc)₄ : The diol (100 mg, 0.17 mmol) was dissolved in dry benzene (4 mL) and Pb(OAc)₄ (85 mg, moistened with acetic acid) was added. After 10 min stirring at room temperature the reaction was filtered, the solvent removed *in vacuo* and the residue purified by flash chromatography eluting with 25%EtOAc in light petroleum. Yield of the aldehyde **61** was 32% (30 mg). (No efforts to optimise the yield were made. Yield might be improved, for example, by partitioning the crude reaction mixture between aq.base and EtOAc to ensure none of the amine product was lost on filtration of the insoluble salts.) TLC 50%EtOAc in light pet. Rf=0.51. NMR analysis (NOESY experiment) indicated the 4,5-trans ring conformation (i.e. the 4(S) isomer). **¹H NMR** (300 MHz, CDCl₃): 9.52, 1H, t, J=1.5 Hz, aldehyde; 7.50-7.25, 10H, m, ArH; 6.92, 2H, d, J=9 Hz, TyrAr; 5.15, 2H, s, OCH₂Ph; 5.05, 2H, s, OCH₂Ph; 4.65, 1H, bm, ringCH₂(i); 3.88, 1H, bm, Tyr□; 3.80, 1H, bm, ringCH₂(ii); 3.45, 1H, d, J=16 Hz, Gly; 3.44, 1H, m, ringCH(aldehyde); 3.28, bd, J=16 Hz, Gly□; 3.17, 1H, bm, Tyr; 2.80, 1H, dd, J=9.0, 13.5 Hz, Tyr□; 2:51; 1H; J=6, 17 Hz, aldehyde; 2.28, 1H, dd, J=17, 4.5 Hz, □aldehyde; 1.50, 9H, Boc. **¹³C NMR** (75 MHz, CDCl₃), (rotamers): 200.5; 169.9; 157.5; 153.1; 136.9; 135.3; 130.5, 129.6, 128.6, 128.5, 128.4, 127.6; 127.4, 115.0: Ar; 80.21, tBoc; 69.92, OCH₂Ph; (67.08, 66.86) br, CH₂; 66.58, OCH₂Ph; (62.93, 62.56) br, CH; (61.35, 60.72) br, CH; 52.14, CH₂; 46.36, CH₂; (38.5, 37.27) br, CH₂; 28.38, Boc. Mass Spectrum (ISMS) m/z 559.1 (MH⁺), calculated for C₃₃H₃₈N₂O₆: 558.

### Preparation of 62 and 63:

The aldehyde **61** (30 mg, 50 µmol) was dissolved in 1,2-dichloroethane (5 mL) and glycine methyl ester hydrochloride (50 mg) and NaBH(OAc)₃ (50 mg) added. The reaction was stirred at room temperature and was complete in a few minutes (<15 min). The reaction was diluted with ethyl acetate, and washed in turn with aq.NaHCO₃, water, brine and then dried (MgSO₄). Evaporation of the solvent left the crude product **62** as a clear oil: TLC 1:1 EtOAc:light pet. Rf=0.17. Mass Spectrum (ISMS) m/z 632.3 (M+H⁺), calculated for C₃₂H₄₅N₃O₅: 631 Analysis of the product or the reaction mixture after overnight standing revealed the formation of a new product with a mass spectrum corresponding to the target cyclised material **63** (MH⁺=524Da). Thus the amine product **62** was not generally isolated but converted directly to 63. The spontaneous cyclisation was accelerated by the addition of base (i-Pr₂NEt). After removal of solvent by evaporation under reduced pressure and the product was purified by flash chromatography eluting with 10-20% EtOAc in light pet. TLC: 1:1 EtOAc:light pet. Rf=0.51. **¹H NMR** (300 MHz, CD₃CN): 7.47-7.29, 5H, m, ArH; 7.12, 2H, m, Tyr; 6.92, 2H, m, Tyr; 5.07, 2H, s, OCH₂Ph; 4.35, 1H, d, J=5.4 Hz; AB_{q,a}=4.05, _{b}=4.02, J_{AB}=17.4 Hz; 3.70-3.52, 6H, overlapped signals (includes: 3.65, 3H, s; 3.58, 1H, dd, J=11.2, 15.2 Hz); 3.49-3.32, 2H, br m's; 3.15, 1 H, br dd, J=5.5, 15.5 Hz; 2.99, 1H, br dd, J=13.4, 14.9 Hz; 2.80, 1H, vbr m; 2.68, 1 H, vbr m; 1.64, 1 H, m; 1.46, 10H, s + m, Boc resonance obscures multiplet. **¹³C NMR** (75 MHz, CD₃CN), rotamers, in approximate ratio 3:2, split some peaks and are recorded in parentheses: 173:3; 171.5; 158.8; 155.0, br; 138.9; 132.0; 129.9; 129.2; 129.0; 116.1; 80.84; 71.01; (70.87, 69.99); (68.12, 67.45); (65.47, 64.89); 55.76; 52.93; 51.45; 49.95; (39.00, 37.53); 31.87; 28.97 (Boc). Mass Spectrum (ISMS) m/z 524.3 (M+H⁺), calculated for C₂₉H₃₇N₃O₆: 523.

### Preparation of compounds 64 to 66:

The product **63** was hydrolysed with LiOH/H₂O/MeOH to the acid **64** (mass spectrum MH⁺=510) and then coupled (DMF/CH₂Cl₂/HBTU/DIEA) with phenethylamine using standard procedures and work-up to give **65**. The imidazolidine ring of **65** was deprotected with a solution of acetic acid-methanol-water (∼1:1:1, stirred as a very dilute solution for several days then lyophilised) to give crude **66** as a white amorphous solid. Mass Spectrum (ISMS) m/z 601 (M+H⁺), calculated for C₃₅H₄₄N₄O₅: 600.

### Example (B). Synthesis of a (4,5)-cis imidazolidine aldehyde by oxidation of a diol.

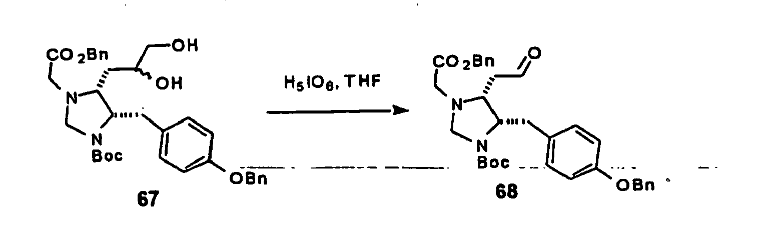

For the preparation of the 4,5-cis aldehyde **68** (in this case the 4(R) isomer) the diol **67** prepared from alkene **60** (as described above) (1mmol) was dissolved in THF (10 mL) and H₅IO₆ (1 mmol) dissolved in THF (∼20 mL) was added and the reaction stirred at room temperature. A precipitate of iodic acid rapidly formed and the reaction was complete in <5 min. The THF solution was diluted with ether and washed in turn with 10% aq.Na₂CO₃, water, brine and then dried (MgSO₄). The product aldehyde **68** was formed in good yield and purity. Contact with acid should be minimised to prevent isomerisation to the trans aldehyde and/or decomposition, for example avoid chloroform as an NMR solvent unless recently made acid free. Yield was 60-80%. TLC: 50%EtOAc in light pet. Rf=∼0.5. **¹H NMR** (300 MHz, CD₃CN): (peaks moderately broad; the Boc rotamers were not resolved although the Boc peak was asymmetric and very broad) 9.48, 1 H, bm, aldehyde; 7.5-7.3, 10H, m, 2xBn; 7.09, 2H, bd, J=7.5 Hz, Tyr Ar; 6.88, d, 8.2 Hz, Tyr Ar; 5.13, s, 2H, OCH₂Ph; 5.05, s, 2H, OCH₂Ph; 4.38, 1H, d, 6.0 Hz, NCH₂N(a); 4.22, 1H, m, Tyr; 4.02, 1H, br, NCH₂N(b); 3.56, 1H, bd, J=17.2 Hz, Gly□(a); 3.48, 1H, m, TyrC'H; 3.29, 1H, bd. J=17.2 Hz, Gly(b); 2.57-2.88, 4H, e, Tyr CH₂ and □-aldehyde CH₂; 2.22, s, H₂O; 1.4481.08 (1.20 peak), 9H, vbr, Boc 3xCH₃. **¹³C NMR** (75 MHz, CD₃CN): 201.9; 171.4; 158.7; 154.3; 139.0; 137.6; 132.6; 131.9, 129.92, 129.85, 129.6, 129.2, 128.9, 116.0: ArCH; 80.41 (Boc tert.); 70.99 (CH₂); 67.62 (br, CH₂); 67.44 (br, CH₂); 60.29 (2xCH, co-incident peaks determined by comparative intensity); 52.99 (br, CH₂); 43.58 (br, CH₂); 35.94 (br, CH₂); 28.78 (br, Boc 3xCH₃).

### Example (C). Synthesis of turn mimetics I(i) for the Gly-Phe-Leu sequence by the short method (which can be used when R¹ = hydrogen)

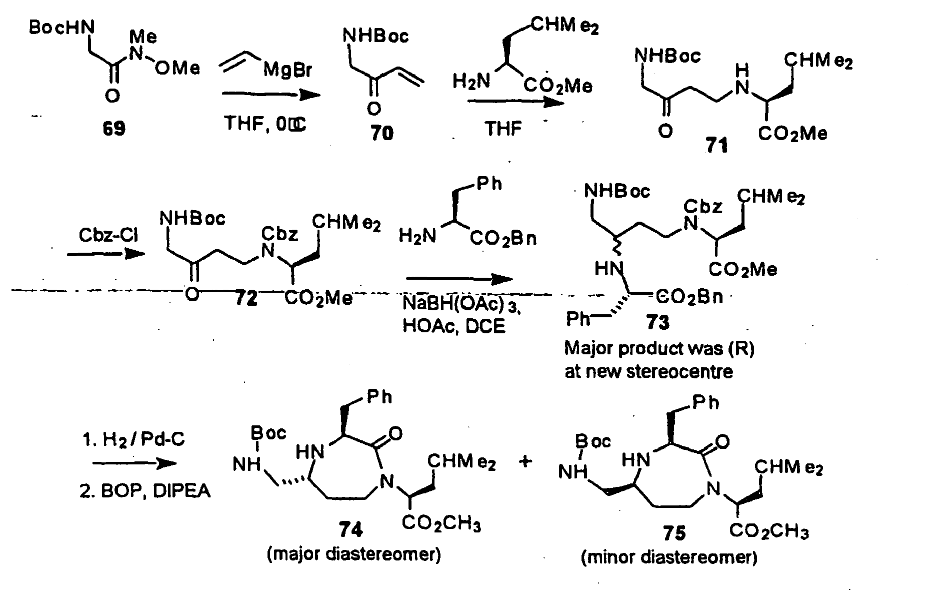

### Preparation of 69:

Boc-glycine was coupled with N,O-dimethyl hydroxylamine hydrochloride, 1 equivalent, in DMF/CH₂Cl₂ (1:5) using HBTU reagent (1 eq.) and DIEA (2 eq.) at room temperature. The CH₂Cl₂ was evaporated *in vacuo* and the residue partitioned between diethyl ether and aq. NaHCO₃. The aqueous layer was separated and the ether layer washed in turn with 1M HCl (x2), aq. NaHCO₃, brine, and then dried over MgSO₄. Filtration and removal of the solvent *in vacuo* left the product amide 69 as a viscous oil that slowly crystallised to a waxy solid and was further purified by chromatography on silica gel. Yield was >90%. ¹H NMR (300 MHz, CDCl₃): 5.3, 1H, bs, NH; 4.09, 2H, bd, H₂; 3.72, 3H, s, OCH₃; 3.20, 3H, s, NCH₃; 1.46, 9H, s, Boc. **¹³C NMR** (75 MHz, CDCl₃): 79.6; 61.4; 41.7; 32.4; 28.3.

### Preparation of 70:

A solution of 11.6 g (53 mmol) of Boc-glycine N,O-dimethylhydroxylamide in dry THF (70 mL) under nitrogen in a 250 mL round bottom flask was stirred and cooled in an ice bath. To this was added vinyl magnesium bromide in THF (∼120 mmol of a 1 M solution) by syringe over 10 minutes. The solution was stirred for 2 h and then quenched by pouring into a mixture of crushed ice and 1 M HCl which was then extracted with CH₂Cl₂ (x2). The organic extracts were washed with water/brine (x2), aq. NaHCO₃ and water/brine followed by drying over MgSO₄. Evaporation of the solvent left 9.6 g of a mobile oil (98% crude) which by NMR was ∼95% the ketone product 70. This material was used without further purification in the conjugate addition step. **¹H NMR** (300 MHz, CDCl₃): 6.37, 2H, m (ABX, J_{ab}=2.5 Hz, J_{ax/bx}=9.0, 17.5 Hz), vinyl CH₂; 5.95, 1H, dd, J=2.5, 9.0 Hz, vinyl CH; 5.37, 1H, bs, NH; 4.26, 2H, d, J=4.6 Hz, glycyl H₂; 1.46, 9H, s, Boc. **¹³C NMR** (75 MHz, CDCl₃): 194.9 ketone; 155.8 carbamate; 133.6 vinyl; 129.6 vinyl; 79.8 tBoc; 48.32 Gly; 28.28 Boc.

### Preparation of 71:

To a solution of 3.0 g (∼15 mmol) of crude **70** in THF (40 mL) was added 3.4 g of leucine methyl ester hydrochloride (∼1.2 eq) and 2.4 g (1.2 eq) of diisopropylethylamine. After 2 h the reaction was diluted with ether (200 mL) and extracted with cold 1M-HCI (3x50 mL) (discard this ether layer). The aq. extracts were immediately neutralised with solid NaHCO₃ and this solution was then back extracted with ether, and the ether washed with water (x3) and finally brine and dried over MgSO₄. Evaporation of the solvent left ∼5.3 g of product **71** as an oil with very good purity, contaminated with a small amount of leucine methyl ester. Flash chromatography to separate the product was not very successful as the amine and amino ketone tended to co-elute. TLC EA/LP Rf=0.35. **¹H NMR** (300 MHz, CDCl₃): 5.36, 1H, bm, NHBoc; 4.03, 2H, d, J=5 Hz, Gly□; 3.72, 3H, s, OCH₃; 3.26, 1H, t, J=7.5 Hz, Leu; 2.93, 1H, dt, J=12, 6 Hz; 2.72, 1H, dt, J=12, 6 Hz; 2.50, 2H, m; 2.0, 1H, bs, NH; 1.69, 1 H, m, Leu; 1.45, 11H, m, Boc(9H) and Leu (2H); 0.90, 6H, m, Leu **¹³C NMR** (75 MHz, CDCl₃): 205.1; 176.1; 155.5; 79.8 tBoc; tt 60.04; 51.64; 50.53; 42.63; 42.57; 40.55; 28.26 Boc; 24.81; 22.63; 22.17. Mass Spectrum (ISMS) m/z 331.4 (M+H⁺), calculated for C₁₆H₃₀N₂O₅: 330; fragments (OR 60): 275.2 (-tBu).

### Preparation of 72:

The amine **71** was protected as the benzyl carbamate by standard procedures as follows: the crude amine product **71** (1.68 g, ∼5 mmol) was dissolved in ethyl acetate (30 mL) to which was added a solution of KHCO₃ (1.2 g) in water (15 mL). This mixture was vigorously stirred and cooled in an ice bath and to it was added benzyl chloroformate (780 uL of a 95% solution, 5.2 mmol) dropwise over 5 min. The reaction was stirred for a further 15 min then allowed to warm to room temperature with stirring for an additional 2 h. After this time the mixture was diluted with ether (100 mL), the aqueous layer seperated, and the organic layer washed with 1M HCI, aq. NaHCO₃, brine and then dried over MgSO₄. Evaporation of the solvent left ∼2.6 g crude oil which was purified by flash chromatography eluting with 25%EtOAc in light pet; combination of the main fractions gave a yield of 86% (2.02 g) of **72**. TLC EA:2LP Rf=0.56. NMR signals split due to amide rotamers (∼1:1) are placed in parentheses where possible. **¹H NMR** (300 MHz, CDCl₃): 7.40-7.23, 5H, Ar; 5.28-5.02, 3H, m's, CH₂Ph + NH; (4.64, m, 4.43, m) 1 H; (3.98, bs, 3.88, bs) 2H; 3.72-3.51, 4H, includes (3.67, s, 3.55, s) OCH₃ + 1 H; 3.45, 1 H, m; 2.78, 2H, m; 1.75, 2H, m; 1.53, 1 H, m; 1.43, 9H, s, Boc; 0.91, 6H, m, Leu□ CH₃x2. **¹³C NMR** (75 MHz, CDCl₃): (204.9, 204.5) ketone; (172.5, 172.3) ester; (156.1, 155.8) carbamate; 155.6, carbamate; (136.2, 136.0) ipso; 128.5, 128.2, 128.1, 128.0: ArCH; 79.80, tBoc; 67.48; (58.50, 58.32); 52.12; 50.30; (41.37, 39.87, 39.78, 38.87, 38.60, 37.98) 3C; 28.23, Boc; (24.83, 24.67); 23.09; (21.46, 21.39). Mass Spectrum (ISMS) m/z 465.3 (MH⁺), calculated for C₂₄H₃₆N₂O₇: 464; fragments (OR 70): 409.2, (-tBu); 365.2, (-Boc).

### Preparation of amines 73:

To a solution of **72** (700 mg, 1.5 mmol) in 15 mL of 1,2-dichloroethane was added phenylalanine benzyl ester p-toluene sulfonate (900 mg, 2.1 mmol) and sodium triacetoxy borohydride (850 mg, 4.0 mmol). The mixture was stirred at room temperature for 24 h and then the solvent removed under vacuum and the residue partitioned between ethyl acetate and aq. NaHCO₃, the aqueous layer separated, and the organic layer washed with water then brine and then dried over MgSO₄. Evaporation of the solvent left 1.2 g crude oil which was purified by flash chromatography eluting with 25-40% EtOAc in light petroleum ether to give a yield of 76% (800 mg) of the product (a clear oil). The product diastereomers **73** were not seperable under these chromatography conditions. TLC 40%EA in LP Rf=0.48. **¹H NMR** (300 MHz, CD₃CN): (not very informative due to the presence of rotamers/diastereomers) 7.45-7.05 aromatic protons; (5.46 m, 5.31 m)∼1/2H; 5.15-5.00, ∼4H, m, OCH₂Ph; 4.95, ∼1/4H, m; (4.51, m, 4.37, m): 1H; 3.85-3.10, ∼5H, e (including 3.63, s, 3.58, s: 3H, OCH₃); 3.10-2.70, 5H, e; 2.45 broad water peak; 1.80-1.45, 5H, m's; 1.40, 9H, s, Boc; 0.90, 6H, bs, Leu. **¹³C NMR** (75 MHz, CD₃CN): (signals are grouped in parentheses where they can be reasonably assigned to equivalent carbons in the different diastereomers/rotamers) (175.6, 175.4(br)); 173.6; 157.4, 157.2 (br); (139.0, 139.2, 138.5, 138.3, 137.3) 3x ipso; 130.8, 130.7 129.9 129.71, 129.66, 129.3, 129.0, 128.0: Ar CH; (79.87, 79.62) Boc tertiary; 68.22 (CH₂, OBn); 67.75 (CH₂, OBn); (61.67, 61.55) (CH); 59.39 (CH); (56.51, 55.82, 55.61) (CH); 53.11 (OCH₃); (45.56, 45.16, 44.73, 44.61, 44.43, 44.24, 43.42, 43.04) (2xCH₂); (40.77, 40.15, 40.03, 39.42, 39.27) (2xCH₂); (39.66, 32.60, 32.45, 31.44) (CH₂); 29.04 (CH₃ Boc); 29.93 (CH); 23.88 (CH₂); 22.36 (CH₂). Mass Spectrum (ISMS) m/z 704.4 (M+H⁺), calculated for C₄₀H₅₃N₃O₈: 703.

### Preparation of 74 and 75:

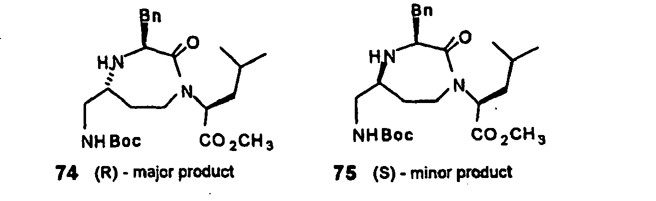

The mixture of epimeric amines **73** (260 mg, 0.4 mmol) was dissolved in methanol (20 mL) and 10% palladium on carbon added (100 mg). The solution was hydrogenated (40 psi. H₂) at room temperature for 3 h to give the deprotected amino acid (MH⁺=480Da). After filtration, the solvent was removed and the residue (170 mg) was dissolved in DMF (5 mL) and diluted with CH₂Cl₂ (50 mL). To this solution was added HBTU (180 mg, 0.48 mmol) and DIEA (150 mg, 1.2 mmol). After stirring for 10 min at room temperature the solution was diluted with aq.NaHCO₃, the aqueous layer separated, and the organic layer washed with water (x3) then brine and then dried over MgSO₄. Evaporation of the solvent left an oil which was purified by flash chromatography eluting with 20-40% EtOAc in light petroleum ether. The product diastereomers were just separable under these conditions, with the minor diastereomer **75** eluting first to give a yield of 18% (30 mg) followed by the major diastereomer **74** in 50% (85 mg) yield. TLC EA:LP 1:1 Rf=0.43, 0.29. **¹H NMR** (300 MHz, CD₃CN): **Isomer 75**: 7.29, 4H, m, ArH; 7.22, 1H, m, ArH; 5.17, 1H, dd, J=6.5, 8.4 Hz; 5.08, 1H, m; 3.65, 3H, s, OCH₃; 3.61, 1 H, dd, J=11.4, 15.6 Hz; 3.27, 1 H, ddd, J=1.5, 5.7, 15.9 Hz; 3.12, 1H, dd, J=4.5, 14.3 Hz; 2.98, 1H, bm; 2.72, 1H, m; 2.64, 1H, dd, J=9.9, 14.3 Hz; 2.57, 1 H, bm; (2.17, H₂O); 1.68, 3H, m; 1.60, 1H, m, Leu; 1.36, 9H, s, Boc; 1.16, 1 H, m; 0.95, 3H, d, J=6.4 Hz, Leu; 0.93, 3H, d, J=6.6 Hz. **Isomer 74**: 7.29, 4H, m, ArH; 7.22, 1H, m, ArH; 5.11, 1H, dd, J=5.6, 9.4 Hz; 4.29, 1H, br, NHBoc; 3.81, 1 H, dd, J=4.6, 9.8 Hz; 3.65, 3H, s, OCH₃; 3.59, 1H, dd, J=10.8, 15.2 Hz; 3.19, 1H, dd, J=5.5, 15.2 Hz; 3.13, 1H, dd, J=4.5, 13.8 Hz; 2.94, 2H, m's; 2.71, 1H, m; 2.64, 1H, dd, J=10.3, 13.3 Hz; (2.17, H₂O); 1.76, 1H, m; 1.69, 2H, m; 1.57, 2H, m; 1.36, 9H, s, Boc; 0.93, 6H, d, J=6.5 Hz. **¹³C NMR** (75 MHz, CDCl₃): Isomer **75** (5S): 175.2; 172.5; 155.9; 138.9; 129.3; 128.5; 126.4; 79.2; 60.91; 60.62; 55.65; 52.19; 45.70; 43.98; 38.12; 37.99; 33.46; 28.30, Boc; 25.01; 23.10; 21.93. Isomer **74** (5R): 175.1; 172.5; 155.7; 139.3; 129.3; 128.7; 126.8; 78.9; 56.01; 55.80; 53.05; 52.14; 42.07; 40.70; 38.01; 37.98; 31.51; 28.26, Boc; 25.03; 23.11 21.74. Mass Spectrum (ISMS) m/z 462.3 (MH⁺), calculated for C₃₂H₄₅N₃O₅: 461 fragments (OR 70): 406.2 (-tBu).

### Example (D). Selective synthesis of the 3(S), 5(S) diastereomer 75 by the short methods

The 3(S)5(S) diastereomer, the minor product formed as described above, can be selectively synthesised by the use of an intramolecular reductive amination-cyclisation as described below:

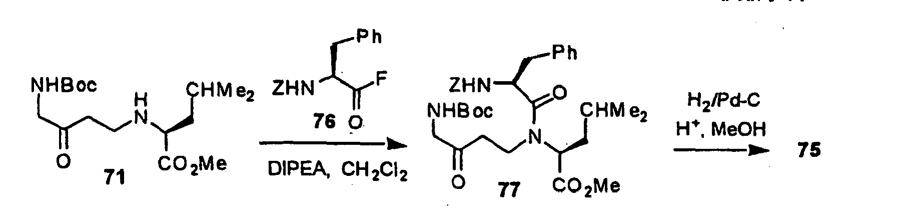

### Preparation of acyl fluoride 76:

Z-phenylalanine acid fluoride was prepared by general literature methods (Carpino *et al.,* 1990; Wenschuh *et al.,* 1994) as follows: 1.1 equivalents of diethylaminosulfurtrifluoride (DAST) were added to ZPheOH in dry dichloromethane solution under nitrogen at 0°C. After stirring for 15 min the reaction was worked up by pouring onto iced water and separating the organic layer, washing once with cold water and then drying over MgSO₄. The product was purified by precipitation from ether/petroleum ether and dried *in vacuo.* **¹H NMR** (300 MHz, CDCl₃): 7.36, 8H, m's; 7.28, 2H, m; 5.30, 1H, bd; J=7.5 Hz, NH; 5.13, 2H, s, OCH₂Ph; 4.85, 1 H, m, H; 3.20, 2H, m, H₂. **¹³C NMR** (75 MHz, CDCl₃): 161.8, d, ¹J_{CF}=370 Hz; 155.5; 135.7; 134.2; 129.1; 129.0; 128.5; 128.3; 128.1; 127.7; 67.36; 53.50, d, ²J_{CF}=59 Hz; 36.70.

### Preparation of 77:

To the amine **71** (2.7 g, 8.2 mmol) dissolved in CH₂Cl₂ (40 mL) was added Z-phenylalanine acid fluoride **76** (prepared as described above) (3.0 g, 10 mmol) and DIEA (1.3 g, 10 mmol) and the solution stirred at room temperature under nitrogen for 30 h. The solvent was then evaporated *in vacuo* and the residue dissolved in ether and extracted in turn with 1 M HCI (x2), 10% aq. Na₂CO₃ (x2), then brine and then dried over MgSO₄. The solution was filtered and the solvent removed *in vacuo.* The resulting oil was purified by flash chromatography eluting with 20-40% ethyl acetate in light petroleum ether for a yield of about 80% of the target **77** as a clear oil. TLC 40%EA:LP Rf=0.40. **¹H NMR** (300 MHz, CDCl₃): 7.41-7.13, 10H, Ar; 5.48, 1H, bd, J=9.2 Hz, NHCbz; 5.19, 1 H, bm, NHBoc; 5.09, 2H, s, OCH₂Ph; 4.76, 1H, dt, J=6.4, 8.9 Hz, Phe; 4.38, 1 H, dd, J=5.2, 9.3 Hz, Leu; 3.92, 2H, d, J=4.5 Hz, Gly; 3.60, 3H, s, OCH₃; 3.54, 1H, m; 3.38, 1 H, m; 3.08, 1 H, dd, J=8.4, 13.3 Hz; 2.93, 1H, dd, J=6.1, 13.1 Hz; 2.65, 2H, m; 2.80, 1 H, m; 2.64, 1H, m; 1.46, 9H, s, Boc; ∼1.38, 1H, m; 0.90, 6H, 2xd, J=6.6, 6.5, Leu. **¹³C NMR** (75 MHz, CDCl₃) amide rotamers (∼5:1): only the major peak of rotamer peak pairs is reported: 204.1; 172.1; 171.4; 156.7; 155.6; 136.2; 135.8; 129.4-127.1: ArCH; 79.8; 66.82; 58.15; 52.25; 52.05; 50.28; 41.32; 39.58 (2 coincident signals as determined by relative intensity, shift and the presence of both minor rotamer peaks); 37.82; 28.23, Boc; 24.67; 23.08; 21.67. Mass Spectrum (ISMS) m/z 612.3 (M+H⁺), calculated for C₃₃H₄₅N₃O₈: 611; fragments: (OR 60): 556.3 (-tBu); 512.3 (-Boc).

### Selective preparation of 75 from 77:

The ketone **77** (1mmol) was dissolved in 0.1M methanolic HCl (30ml) and 10% palladium on activated carbon (200mg) was added. The solution was hydrogenated at 30 psi H₂ (room temperature) for 8 h and then diluted with aq. NaHCO₃ and extracted with ethyl acetate. The organic layer was washed with water (x2) and then brine then dried over MgSO₄. Filtration and removal of solvent in vacuo left the crude product **75** in good yield and purity. Analysis of the crude product by NMR and by TLC did not reveal any of diastereomer **74**. The reaction was estimated to be >95% stereoselective.

### Example (E). Synthesis of a biologically active turn mimetic for the Arg-Gly-Asp sequence

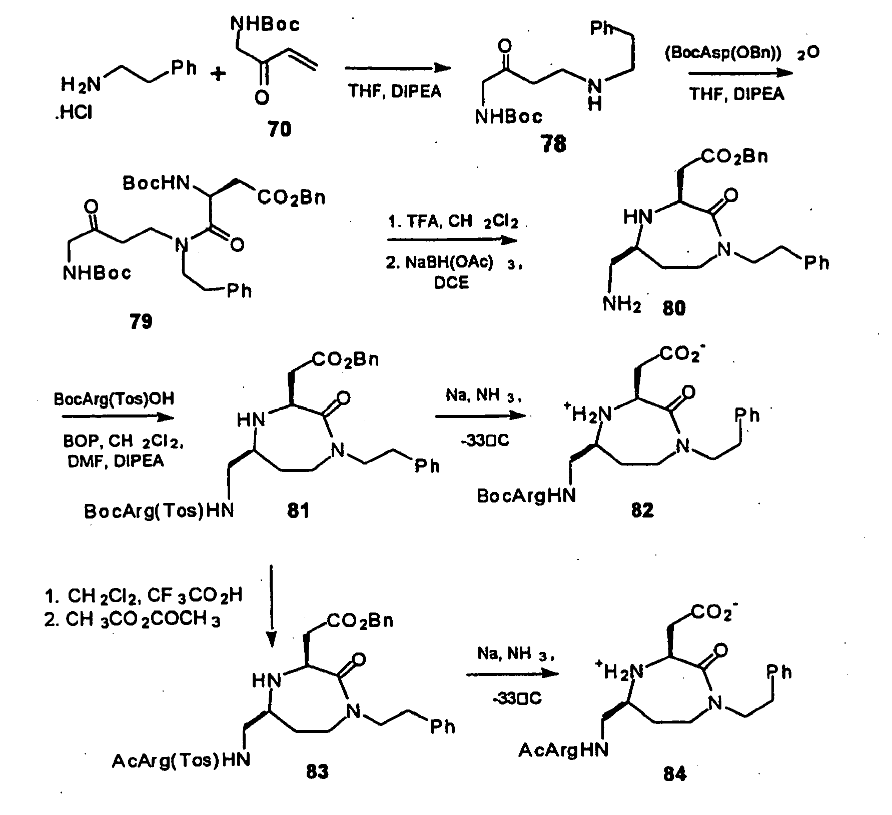

### Preparation of 78:

The α,β-unsaturated ketone **70** (1.0 g, 5.4 mmol, prepared as previously described) was reacted with phenethylamine hydrochloride (1.07 g, 6.8 mmol) and DIEA in THF by the method previously described for the preparation of **71.** The crude product **78** was used without further purification for the next reaction. Mass Spectrum (ISMS) m/z 307.2 (MH⁺), calculated for C₁₇H₂₆N₂O₃: 306; fragments (OR 60): 250.9 (-tBu).

### Preparation of 79:

To a stirred solution of Boc-aspartic acid benzyl ester (3.23 g, 10 mmol) in CH₂Cl₂ (10 mL) was added dicyclohexylcarbodiimide (10 mL of 0.5M solution in CH₂Cl₂) at room temperature. A copious precipitate of dicyclohexylurea soom formed; after 10 min the solution was filtered, and the solvent removed *in vacuo*. The residual oil was added to a solution of crude **78** (1.3 g) in THF, followed by DIEA (645 mg, 5 mmol), and the solution stirred for 4 h. The reaction mixture was diluted with ether/ethyl acetate and washed with 1 M HCl, aq. NaHCO₃, water, brine and dried over MgSO₄. The crude product was purified by flash chromatography eluting with 30-50% ethyl ether in petroleum ether to give a reasonable yield of **79** (estimated as 80% based on **78**) as a clear oil. **¹H NMR** (300 MHz, CDCl₃, amide rotamers present): 7.38-7.16, 10H, m, Ar; 5.37, 1H, bd, J=9 Hz, AspNHBoc (minor rotamer 5.33, J=10 Hz. 5.25, m, 1 H (Gly NH); 5.10, 2H, m, OCH₂Ph; 4.89, 1 H, m; 3.93, 2H, d, J=4.4 Hz, Gly; 3.67-3.53, 3H, m's; 3.47, 1H. m; 2.95-2.52, 6H, m's (including 2.88, 2H, m; 2.63, 2H, ABX, J=15.8, 7.3, 5.8 Hz, H₂Asp); 1.44, 18H, multiple singlets, 2xBoc. **¹³C NMR** (75 MHz, CDCl₃): (major rotamer only) 204.7; 171.0; 170.3; 155.6; 154.8; 137.7; 135.5: 128.9, 128.6, 128.5, 128.2, 126.6: ArCH; 80.06; 79.73 (2x tBoc); 66.57; 50.55; 50.33; 46.99; 42.24; 37.69 (2 signals); 35.50; 28.22 (2x Boc). Mass Spectrum (ISMS) m/z 612.3 (MH⁺), calculated for C₃₃H₄₅N₃O₈: 611 fragments (OR 60): 556.1 (-tBu); 512.1 (-Boc).

### Preparation of 80:

The ketone **79** (390 mg, 0.64 mmol) in CH₂Cl₂ (2 mL) was treated with trifluoroacetic acid (2 mL) and the solution stirred for 30 min at room temperature. The volatiles were then removed *in vacuo* and CH₂Cl₂ (3 mL) added and removed *in vacuo* (x2). The residual oil was dissolved in 1,2-dichloroethane (5 mL) and NaBH(OAC)₃ (270 mg, 1.3 mmol) added. The mixture was stirred for 20 min then the solvent removed and the residue dissolved in ethyl acetate and washed with aq. Na₂CO₃ and then brine and then dried over MgSO₄. The crude product **80** (after solvent removal 210 mg, 84%) was of good purity by MS and NMR, with only one diastereomer observed (>95% diastereoselectivity). **¹H NMR** (300 MHz, CDCl₃): 7.39-7.10, 10H, m, Ar; {5.20, 5.16, 5.14, 30 5.10}, 2H, ABq, J=12.5 Hz) OCH₂Ph; 3.86, 1 H, t, J=6.3; 3.76-3.43, 3H, m's; 3.14, 1H, bdd, J=15, 5 Hz; 2.98-2.76, 5H, e; 2.70, 1H. dd, J=7.4, 16 Hz; 2.46, 1H. m; 1.64, 1H, bm; 1.06, 1H bm. **¹³C NMR** (75 MHz, CDCl₃): 173.9; 172.0; 138.9; 135.9; 128.7, 128.4, 128.0, 126.3: Ar; 66.16; 60.49; 56.55; 51.24; 48.39; 45.14; 38.05; 34.15; 33.01. Mass Spectrum (ISMS) m/z 396.2 (MH⁺), calculated for C₂₃H₂₉N₃O₃: 395.

### Preparation of 81:

The crude amine product **80** (140 mg, ∼0.35 mmol) was coupled with BocArg(Tos)OH (182 mg, 1.2 eq) using the BOP reagent (188 mg) and DIEA (55 mg) in DMF/CH₂Cl₂ (5ml). The CH₂Cl₂ was evaporated *in vacuo* and the residue partitioned between diethyl ether/ethyl acetate and aq. NaHCO₃. The aqueous layer was separated and the organic layer washed in turn with 1 M HCl (x2), water (x2), aq. NaHCO₃, brine, and then dried over MgSO₄. Filtration and removal of the solvent *in vacuo* left the crude product amide **81** which was purified by flash chromatography eluting with 5-10% ethanol in ethyl acetate (yield 260 mg, 90%). TLC 10% EtOH in EtOAc Rf=0.38. **¹H NMR (300** MHz, CD30D): 7.74, 2H, d, J=7 Hz; 7.4-7.15, 12H, m's; 5.15, 2H abq, J=11 Hz, OBn; 4.26, 1H, m; 4.03, 1H, m; 3.73, 2H, m; 3.48-3.07, 7H, e; 3.07, 1H, m; 2.92-2.73, 3H, m's; 1.92, 1H, m; 1.73, 1H, m; 1.66-1.45, 4H, e; 1.42, 9H, s, Boc. **¹³C NMR** (75 MHz, CD₃OD): 176.1; 172.5; 172.0 (br); 158.8; 158.1; 143.7; 142.2; 140.3; 137.5; 130.4; 130.1; 129.72; 129.68; 129.4; 128.4; 127.6; 127.3; 127.2; 80.92 (t); 67.75 (CH₂); 62.55 (CH); 57.27 (CH); 56.00 (CH); 52.55 (CH₂); 48.74 (CH₂); 44.42 (CH₂); 41.22 (br, CH₂); 37.00 (CH₂); 35.10 (CH₂); 32.41 (CH₂); 30.15 (CH₂); 28.87 (Boc CH₃); 27.24 (br, CH₂); 21.57 (CH₃). Mass Spectrum (ISMS) m/z 806.4 (MH⁺), calculated for C₄₁H₅₅N₇O₈S: 805.

### Preparation of 82:

The amine 81 (50 mg, 0.06 mmol) in THF (0.6 mL) was cooled in a dry ice acetone bath and ammonia gas added until ~30 mL of ammonia had condensed. Small pieces of sodium metal (3-6 mg) were added until the blue colour persisted. The reaction was quenched by the addition of ammonium carbonate (25 mg), the dry ice bath removed and the solvent allowed to evaporate at room temperature. The residue (which gave a crude mass spectrum with the product mass as the only significant peak) was purified by reversed phase HPLC (Vydac C18) eluting with 85% solvent A (=0.1 % CF₃COOH in H₂O):15% solvent B (=0.1 % CF₃COOH and ~10% H₂O in CH₃CN) for 2 minutes followed by a 2%/min gradient. Only one product diastereomer was observed in the HPLC traces. Mass Spectrum (ISMS) m/z 562.3 (M+H⁺), calculated for C₂₇H₄₃N₇O₆.

### Preparation of 83:

The amine **81** was dissolved in CH₂Cl₂/CF₃CO₂H (2ml, 1:1) and stirred at room temperature for 30 minutes after which the Boc group had been removed. 10ml of CH₂Cl₂ was then added and the volatiles removed in vacuo (repeat once). The residue was again dissolved in CH₂Cl₂ and acetic anhydride (2 eq.) added along with diisopropylethylamine (DIEA, 5 eq.), and the reaction stirred at room temperature for 2 h. The volatiles were removed *in vacuo* and the residue dissolved in ethyl acetate and washed with aq. NaHCO₃ then brine and then dried over MgSO₄. Filtration and removal of the solvent *in vacuo* left the crude product **83** as an oil in reasonable purity. The ¹H NMR was badly broadened in common solvents at room temperature. **¹³C NMR** (75MHz, CDCl₃): 173.7; 172.4; 171.9; 171.0; 157.0; 142.1; 140.4; 138.8; 135.8; 129.2, 128.7, 128.4, 128.1, 128.0, 126.3, 125.8: ArCH; 66.22, OCH₂Ph; 60.08, CH; 56.09, CH; 52.94, br, CH; 51.06, CH₂; 48.21, CH₂; 44.31, CH₂; 40.13, br, CH₂; 37.79, CH₂; 34.16, CH₂; 32.97, CH₂; (29.59, 29.50) 1C, br, CH₂; 25.64, br, CH₂; 22.91, CH₃; 21.32, CH₃. Mass Spectrum (ISMS) m/z 748.2 (MH⁺), calculated for C₃₇H₄₉N₇O₇S: 747.

### Preparation of 84:

Compound **84-was** prepared from **83** by dissolving metal reduction as described for the preparation of **82** above. Purification was carried out by HPLC under the same conditions as for **82.**

### Testing of Arg-Gly-Asp mimetics 82 and 84 for inhibition of platelet aggregation in human platelet rich plasma (PRP)

The peptide sequence arginine-glycine-aspartic acid (RGD) is important to the binding of proteins to certain integrin receptors, such as the GP_{IIb-IIIa} receptor found on the surface of platelets. Several cyclic peptides having the RGD sequence have been found to antagonise the binding of plasma proteins to the GP_{IIb-IIIa} receptor, thereby inhibiting blood clotting. GP_{IIb-IIIa} antagonists have therapeutic potential as anti-thrombotics, there are several in early clinical trials(Humphries, Doyle *et al.,* 1994). Mimetics based on turn structures centred on the Asp residue have been successful, this structure was chosen to test the compounds of the invention.

Solutions of the compounds to be tested were made up in water. Platelet aggregation induced by adenosinediphosphate (ADP, 10 µM) in human PRP was measured by the decrease in light scattering on aggregation, measured with a platelet aggregometer. The tetrapeptide Ac-Arg-Gly-Asp-Ser-NH₂ was used as a positive control.(Callahan *et al.,* 1992) Compounds **82** and **84** were both found to inhibit platelet aggregation in a dose dependent manner, and both exhibited stronger inhibition than the control peptide. Compound **84** was the strongest, having inhibitory activity approximately five times more potent than Ac-Arg-Gly-Asp-Ser-NH₂ under the conditions of the test.

### Example (F). Synthesis of fully substituted turn mimetics for the Phe-Leu-Ala sequence in both the 4(R) and 4(S) configurations

The synthesis up to the final common intermediate for the 4(R) and 4(S) diastereomers, the aldehyde 93, is summarised below:-

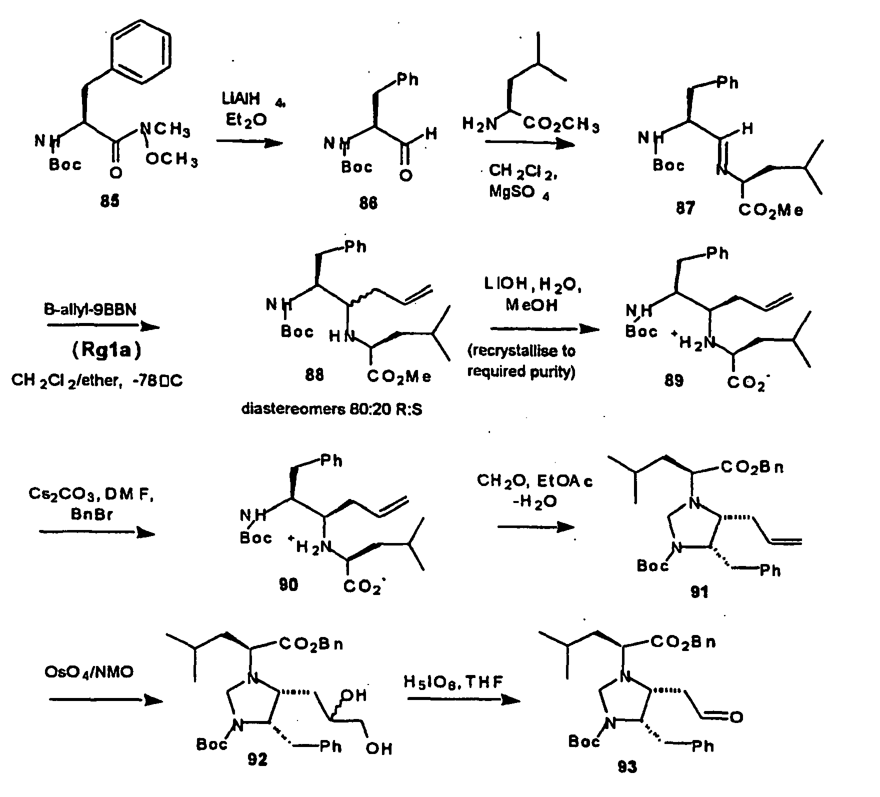

Bocphenylalanine N,O-dimethylhydroxylamide **85** was synthesised by the general solution phase coupling procedure as previously described from Boc-phenylalanine and N,O-dimethyl hydroxylamine hydrochloride. Yield: ~quantitative. Purification: on a short silica column eluting with ether. **¹H NMR** (300 MHz, CDCl₃): 7.33-7.12, 5H, m, Ar; 5.20, 1 H, bd, J~7 Hz, NH; 4.95, 1H, bm, Phe, 3.66, 3H, s, OCH₃; 3.17, 3H, s, NCH₃; 3.06, 1 H, dd, J=6, 13.5 Hz, Phe 2.88, 1H, dd, J=7.5, 13.5 Hz; 1.40, 9H, s, Boc. **¹³C NMR** (75 MHz, CDCl₃): 172.2; 155.1; 136.5; 129.4; 128.2; 126.7; 79.5; 61.4; 51.4; 38.8, Phe; 32.0; 28.2, Boc.

The amide **85** was reduced to Bocphenylalanine aldehyde **86** by the method of Fehrentz and Castro (Fehrentz and Castro, 1983) Briefly: amide (2 mmol) dissolved in dry ether (20 mL) and cooled and in an ice bath under nitrogen, then LiAlH₄ (95 mg, 2.5 mmol) added and stirring continued 15 min. Then KHSO₄ (477 mg, 3.5 mmol) in 10 mL water added and then 150 mL ether and wash with 1 M HCl (cold) (x3), aq. NaHCO₃, brine, and dried over MgSO*₄. Removal of the solvent left the solid aldehyde in ~90% crude yield containing some of the overreduced alcohol as the only significant impurity. TLC EtOAc:light pet. Rf=0.5. **¹H NMR** (300 MHz, CDCl₃): 9.63, 1H, s, aldehyde; 7.37-7.13, 5H, m, Ar; 5.07, 1H, bs, NH; 4.43, 1H, m, Phe□; 3.11, 2H, d(AB) Phe 1.43, 9H, s, Boc. **¹³C NMR** (75 MHz, CDCl₃): 199.4, aldehyde; 155.3, carbamate; 135.7, ipso; 129.3, 128.7, 127.1: ArCH; 80.2, tBoc; 60.8, Phe□; 35.5, Phe□; 28.2, Boc

Methyl leucinate hydrochloride (0.80 g, 4.4 mmol) was neutralised with 10% aq. Na₂CO₃ solution (25 mL), and the solution was mixed with brine (25 mL) and extracted with CH₂Cl₂ (3x20 mL). The organic extracts were dried over MgSO₄ and most of the solvent removed under vacuum (~-2 mL residue). This solution of methyl leucinate was added to Boc phenylalanine aldehyde **86** (1.1 g, 4.4 mmol) in CH₂Cl₂ (5 mL), the stirred solution soon became turbid due to the separation of water, dried MgSO₄ (500 mg) was added and the solution cleared. After 30 min the solution was filtered into a dried flask under nitrogen. NMR analysis showed that all the aldehyde had been converted to the imine **87** and that significant racemisation had not taken place. The imine was used without further purification for the allylation reaction. **¹H NMR** (300 MHz, CDCl₃): 7.61, 1H, d, J=1.3 Hz, imine; 7.32-7.14, 5H, m, Ar; 5.69, 1 H, bd, J=4.5 Hz, NH; 4.49, 1H, m, Phe; 3.85, 1 H, dd, J=5.5, 8.5 Hz, Leu 3.69, 3H, s, OCH₃; 3.20, 1H, dd, J=5.0, 14.5 Hz, Phe; 2.96, 1H, dd, J=8.0, 13.5 Hz; Phe; 1.63, 1H, m; 1.46, 9H, s, Boc; 1.42, 1 H, m; 1.30, 1H, m; 0.88, 3H, d, J=6.5 Hz, leu; 0.80, 3H, d, J=6.5 Hz, Leu. **¹³C NMR** (75 MHz, CDCl₃): 171.7, ester; 164.3, CH, imine; 154.6, carbamate; 136.1, ipso; 128.9, 127.7, 126.0: ArCH; 78.56, tBoc; 69.51; 54.08; 51.32; 41.02, CH₂; 38.04, CH₂; 27.73, Boc; 22.35; 22.48; 20.63.

B-allyl-9-borabicyclononane **Rg1 a** can be synthesised from B-methoxy-9-borabicyclononane (synthesised in turn from the methanolysis of 9-BBN (Kramer and Brown, 1974)) by the method of Kramer (Kramer and Brown, 1977). Alternatively the following one-pot synthesis from 9-BBN was used: a suspension of 9-BBN (crystalline dimer, 8.97 g, 73.5 mmol) in anhydrous ether (75 mL) was stirred under nitrogen and cooled to 0°C. Methanol (3.3 mL, 81 mmol) was slowly added by syringe (gas evolved), and vigorous stirring continued for ~3 h (9-BBN gradually dissolves, gas evolution ceases). Allylmagnesium bromide in ether (81 mL of a 1.0M solution) was slowly added to the solution (still cooled to 0°C); (a thick grey ppt. forms, stirring may be difficult). Stirring was continued for 1 h then the solution was allowed to warm to room temperature and the ether was pumped off under moderate vacuum (-300->20bar). The residue was re-suspended in anhydrous hexane (100 mL) and then stirring stopped to allow the magnesium salts to settle out. The solution was estimated by reaction with a known amount of methylphenylketone in ether (found to be ~0.57M, equal to 78% yield). The clear solution of B-allyl-9-BBN was used directly for allylation of the imines. (This procedure was adapted from one described by Rachlera and Brown (Racherla *et al*., 1992)) The imine **87** (~23 mmol) was dissolved in dry diethylether (100 mL) under nitrogen and the stirred solution cooled to -78°C. B-allyl-9-BBN (47.5 mL of ~0.57M solution in hexane, ~27 mmol) was added and the solution stirred for 1 h and then allowed to warm to room temperature with stirring for an additional 1 h. Glacial acetic acid (1.5 mL) was added and the ether was removed *in vacuo.* The residue was dissolved in acetonitrile (100 mL) and more glacial acetic acid (5 mL) added. The solution was then refluxed until all of the borane adduct had been converted to the amine (~24 h, monitored by TLC: Rf adduct>Rf amine = 0.32 in 1:5 EtOAc:light pet.). The acetonitrile was removed *in vacuo* and the residue partitioned between ether/light petroleum and 10% aq. Na₂CO₃. The organic layer was washed again with 10% aq. Na₂CO₃ and then extracted with a solution of 25% methanol in 0.5M HCl (three times), the organic layer containing the neutral reaction products (~6 g) was discarded. The aq. acid extracts were immediately neutralised with solid NaHCO₃ and then extracted with ether. The ether solution was washed with water then brine and then dried over MgSO₄. Evaporation of the solvent left the amine products (5.9 g) which were further purified by flash chromatography eluting with 7.5-15% ethyl acetate in light petroleum for a yield of 50+% of the amines 88 based on the crude aldehyde **86** used in the imine formation. Some separation of the diastereomers was observed in the chromatography, but they were not well resolved. Alternatively the crude amines were hydrolysed to the amino acid as described below and purified by recrystallisation. **¹H NMR** (300 MHz, CDCl₃), major diastereomer: 7.32-7.13, 5H, m, Ar; 5.84, 1H, m, vinylCH; 5.11, 2H, m, vinylCH₂; 5.00, 1H, d, J=8 Hz, NHBoc; 3.88, 1H, m, Phe; 3.66, 3H, s, OCH₃; 3.40, 1H, t, J=7 Hz, Leu; 2.87, 1 H, dd, J=5, 13 Hz, Phe 2.69, 2H, m's: Phe +CH(homoallyl) 2.23, 2H, m, allyl; 1.7, 1H, b, NH(amine); (1.65, 1H, m; 1.47, 2H_{,} m) Leu; 1.33, 9H, s, Boc; 0.90, 6H, t(2 doublets) J=7, 7 Hz, Leu **¹³C NMR** (75 MHz, CDCl₃), major isomer: 176.1; 155.4; 138.6, ipso; 135.2, CH vinyl; 129.2, 128.2, 126.1: CHAr, 117.4, CH₂ vinyl; 78.8, tBoc; 58.94; 58.56; 54.10; 51.71; 42.87; 36.52; 35.61; 28.24, Boc; 24.78; 22.68; 22.23. Mass Spectrum (ISMS) m/z 419.2 (MH⁺), calculated for C₃₂H₄₅N₃O₅: 418 fragments (OR 65): 363.2, (-tBu).

The crude amine product 88 (1.7 g, ~4 mmol) was dissolved in methanol/water and LiOH.H₂O (800 mg, 19 mmol) added. The solution was stirred at room temperature until the hydrolysis was complete (12 h) and then neutralised with 1 M HCl (19 mL). On standing a copious white precipitate formed which was filtered off and washed with water. The solid was recrystallised from ethanol-water (~95:5) to give fine needles of (mainly) the major diastereomer **89** (first crop 1 g), m.p.:175-177°C. The product was further recrystallised as required. **¹H NMR** (300 MHz, CO₃OD): (ref. 3.31 ppm) 7.33-7.18, 5H, m; 5.90, 1H, m; 5.35. 1 H. d, J=17.1 Hz; 5.26, 1H, d, J=10.2 Hz; 4.31, 1H, m; 3.65, 1H, dd, J=5.7, 7.9 Hz; 3.27, 1H, m; 2.92, 1H, dd, J=5.2, 14.0 Hz; 2.76, 1H, dd, J=10.1, 14.0 Hz; 2.59, 1H, m; 1.82, 1 H, m; 1.37, 9H, s, (Boc); 0.97, 3H, d, J=7 Hz; 0.94, 3H, d, J=7 Hz. **¹³C NMR** (75 MHz, CD₃OD): (ref. 49.15 ppm) 173.7; 159.4; 138.8; 134.5; 130.33; 129.8; 128.0; 120.5; 81.34; 63.65; 55.84; 41.19; 37.90; 32.70; 28.78; 26.11; 23.56. Mass Spectrum (ISMS) m/z 405 (MH⁺), calculated for C₂₃H₃₆N₂O₄: 404.

The amino acid **89** was esterified to **90** by the method of Bodansky and Bodansky (Bodansky and Bodansky, 1984) as follows: the amino acid **89** (400 mg, 1 mmol) was dissolved in methanol/water and neutralised with Cs₂CO₃ (300 mg), then the solvents were removed *in vacuo,* then DMF added and removed *in vacuo.* The residue was dissolved in DMF (10 mL) and benzyl bromide (190 mg, 1.1 mmol, purified by passage through a short column of basic alumina) added to the stirred solution. After 2 h the reaction was diluted with aq. NaHCO₃ and extracted with 1:1 EtOAc:light pet. The organic layer was washed in turn with aq.NaHCO₃, water (x2), brine and then dried over MgSO₄. Evaporation of the solvent left the product 90 as a clear oil which solidified to a low melting solid (m.p. ~55°C) on standing (500 mg, ~100%). TLC 25%EtOAc in light pet. Rf=0.57. **¹H NMR** (300 MHz, CDCl₃): 7.38-7.32, 4H, m; 7.28-7.14, 6H, m; 5.82, 1H, m; 5.19-5.05, 4H, m's, (OBn ABq, J=12.5 Hz, (ₐ =5.16, _{b}=5.12 ppm); 4.9, 1 H, br; 3.88, 1H, br; 3.44, 1H, bt, J=7 Hz; 2.88, 1H, dd, J=5, 14 Hz; 2.77-2.60, 2H, bm; 1.63, 1 H, m; 1.56-1.35, m, 2H; 1.33, 9H, bs (Boc); 0.88, 3H, d, J=6.5 Hz; 0.85, 3H, d, J=6.5 Hz. **¹³C NMR** (75 MHz, CDCl₃): 175.5; 155.5; 138.6; 135.8; 135.2; 129.2; 128.5; 128.2; 126.1; 117.4; 78.90; 66.40; 58.96; 58.49; 54.25; 42.83; 36.33; 35.71; 28.27 (Boc); 24.77; 22.63; 22.32. Mass Spectrum (ISMS) m/z 495 (M+H⁺), calculated for C₃₀H₄₂N₂O₄: 494.

The amine **90** (500 mg, 1 mmol) was dissolved in ethyl acetate (20 mL) and 37% aqueous formaldehyde solution (0.5 mL) was added. The solution was stirred for 12 h and then diluted with light petroleum (40 mL) and washed in turn with aq. NaHCO₃, water (x2) and brine and then dried (MgSO₄). Removal of the solvent *in vacuo* gave the product. **91** as a clear oil in approximately quantitative yield. Further purification was carried out by flash chromatography eluting with 10% ethyl acetate in light pet. **¹H NMR** (500 MHz, CD₃CN): (rotamers were present in a ratio of 7:3) 7.36, 4H,m, Ar; 7.27-7.11, 6H, Ar; 5.70, 1H, m, vinyl CH; 5.17-4.97, 4H, m's, vinyl CH₂ and OCH₂Ph; 4.44, 0.7H, d, J=5.0 Hz, ring CH₂(a), major rotamer; 4.33, 0.3H, d, J=4.4 Hz, ring CH₂(a), minor rotamer; 4.19, 0.7H, d, J=5.0 Hz, ring CH₂(b), major rotamer; 4.09, 0.3H, d, J=4.6 Hz, ring CH₂(b), minor rotamer; 4.06, 0.3H, m, Phe□, minor; 4.02, 0.7H, m, Phe, major; 3.74, 0.7H, dd, J=9.8, 6.0 Hz, and 3.69, 0.3H, m, Leu; 3.10, 1H, m, ring methine (homoallyl); 2.88, 0.3H, m, Phe (a); 2.84, 0.7H, dd, J=4.1, 13.4, Phe (a); 2.72, 0.3H, dd, J=6.5, 13.5, Phe (b); 2.65, 0.7H, dd, J=9.5, 13.2. Phe (b); 2.49, 1 H, m, allyl(a); 2.15, 1H, m, allyl(b); 1.76-1.42, 3H, m's, Leu 1.33, 2.5H, s, Boc, minor rotamer; 1.09, 6.5H, s, Boc, major rotamer; 0.97-0.84, 6H, d's, Leu major rotamer: 0.94, J=6.3 Hz; 0.90, J=6.2 Hz). **¹³C NMR** (75 MHz, CD₃CN), only major rotamer reported except where indicated: (ref. 118.69 ppm) 173.3; 154.2; 140.9; 137.8; 136.3 (CH); 131.3; 129.9; 129.7; 129.6; 129.5; 127.2; 118.2 (CH₂); 79.98 (Boc tertiary); 67.17 (CH₂); 63.49 (CH); 62.47 (CH₂); 60.91 (CH); 57.68 (CH); 40.34 (CH₂); 36.04 (CH₂); 33.18 (CH₂); (29.08 Boc minor rotamer); 28.61 (Boc major rotamer); 25.98 (CH); 23.79 (CH₃); 22.36 (CH₃). Mass Spectrum (ISMS) m/z 507 (MH⁺), calculated for C₃₁ H₄₂N₂O₄: 506.

The alkene **91** was dihydroxylated with OsO₄/N-methylmorpholine-N-oxide in tBuOH/water as previously described for the dihydroxylation of **60.** The crude product **92** was used directly in the next reaction. TLC 1:1 EtOAc:light pet. Rf=0.36. Mass Spectrum (ISMS) m/z 541 (M+H⁺), calculated for C₃₁H₄₄N₂O₆: 540.

The glycol **92** (87 mg, 0.16 mmol) was dissolved in THF (4 mL) and H₅lO₆ (37 mg, 0.16 mmol) dissolved in THF (3 mL) was added and the reaction stirred at room temperature. A precipitate of iodic acid rapidly formed and the reaction was complete in <5 min. The THF solution was diluted with ether and washed in turn with 10% aq.Na₂CO₃, water, brine and then dried (MgSO₄). The product aldehyde 93 was of good purity but was not particularly stable to storage. Any traces of acid must be rigorously excluded to prevent isomerisation to the trans isomer. A portion was purified by flash chromatography, eluting with 15%EtOAc in light petroleum. TLC 15%EtOAc in light pet. Rf=0.27. The yield was good (>80%). Amide rotamers were evident in the NMR spectra, ratio ~3:1, only the peak due to the main rotamer is reported unless otherwise noted. **¹H NMR** (300 MHz, CD₃CN, ref 1.94 ppm): 9.53, 1H, s; 7.42-7.10, 10H, m's; 5.11, 2H, s, (OCH₂Ph); 4.41, 1H, br; 4.25, 1H, q, J=6.3 Hz; 4.15, 1H, br; 3.56, 1H, dt, J=8.5, 5.7 Hz; 3.54, 1H, bm; 2.90-2.58, 4H, m; 1.75-1.45, 3H, bm; 1.37, bs, Boc minor rotamer; 1.20, bs, Boc major rotamer; 0.92, 3H, d, J=6 Hz; 0.88, 3H, d, J=5.7 Hz. **¹³C NMR** (75 MHz, CD₃CN, ref 118.69 ppm): 202.0; 173.1; 154.2; 140.4_{;} 137.6; 131.1; 129.9; 129.62; 129.55; 127.26; 80.28 (Boc tertiary); 67.31 (CH₂); 61.90 (CH₂); 60.43 (CH); 58.56 (CH); 57.95 (CH); 43.75 (CH₂); 40.36 (CH₂); 36.48 (CH₂); 28.66 (Boc); 25.83 (CH); 23.67 (CH₃); 22.25 (CH₃). Mass Spectrum (ISMS) m/z 509 (MH⁺), calculated for C₃₀H₄₀N₂O₅: 508.

Conversion of 4,5-cis aldehyde **93** to the 4,5-cis 4(S) amine product was completed by a two step reductive amination procedure as illustrated below:

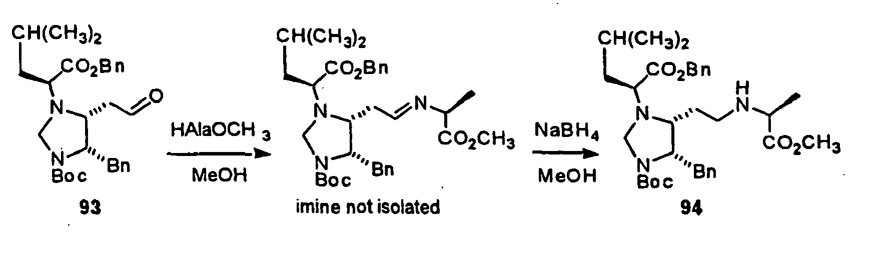

Alanine methyl ester hydrochloride (120 mg, 0.86 mmol) was dissolved in 1:1 brine: 10%aq.Na_{2C}O₃ and extraction into CH₂Cl₂ (x2). The organic extracts were dried (MgSO₄), filtered and the majority of the solvent removed *in vacuo* to leave the volatile amine which was added to a solution of the freshly prepared aldehyde **93** (100 mg, 0.2 mmol) dissolved in methanol (~7 mL, strictly acid free). The solution was stirred at room temperature for 2 h whereupon analysis of a test portion reduced with NaBH₄ showed imine formation to be complete (none of the alcohol formed on reduction of aldehyde was detected). Solid NaBH₄ (50 mg, 1.3 mmol) was added to the solution and stirring continued for 10 min and then the reaction partitioned between ethyl acetate and a water/brine/10%aq.Na₂CO₃ mixture. The aqueous phase was separated and the organic layer washed with water (x2) then brine and then dried (MgSO₄). NMR analysis of the crude product failed to detect the corresponding trans (S) diastereomer (<5%). Evaporation of the solvent left an oil which was purified by flash chromatography eluting with 20-40% EtOAc in light petroleum for a 60-70% yield of **94.** TLC 40%EtOAc:light pet. Rf=0.43. Rotamers observed in the NMR spectra, ratio ~3:1, separate signals due to the minor rotamer recorded only where indicated. **¹H NMR** (300 MHz, CD₃CN, ref. 1.94 ppm): 7.37, 4H, m,; 7.3-7.1, 6H, m; 5.12, 5.09: 2H, ABq, J=12 Hz; 4.39 (major rotamer), 4.29 (minor): 1H. d, J=5 Hz; 4.15, 1 H, J=5 Hz; 4.06, 1 H, m, PheH; 3.75-3.57, 4H, m, LeuH +OCH₃; 3.25-3.10, 1H, m; 3.03, 1H, m; 2.87-2.60, 2H, m, Phe; 2.52-2.25, 2H, m; 1.81, 1H, m; 1.67, 1H, m; 1.6-1.38, 2H, m; 1.34, bs, Boc minor rotamer; 1.19, m, Ala; 1.15, bs, Boc major rotamer; 0.93, 3H, d, J=6.6 Hz; 0.89, 3H, d, J=6.3 Hz. **¹³C NMR** (75 MHz, CD₃CN, ref. 118.69 ppm): 177.3; 173.4; 154.2; 141.0; 137.7; 131.2; 130.9; 129.9; 129.7; 129.6; 129.5; 127.1; 80.02 (Boc tertiary); 67.18 (CH₂); 62.55 (CH); 62.25 (CH₂); 60.75 (CH); 57.67 (2xCH, coincident signals); 52.55 (OCH₃); 45.96 (CH₂); 40.96 (CH₂); 36.15 (CH₂); 29.00 (Boc, minor rotamer); 28.73 (CH₂); 28.62 (Boc, major rotamer); 25.96 (CH); 23.66 (CH₃); 22.35 (CH₃); 19.7 (CH₃). Mass Spectrum (ISMS) m/z 596 (M+H⁺), calculated for C₃₄H₅₀N₃O₆: 595.

Reductive amination of aldehyde **93** (or the 4,5-trans isomer) with NaBH(OAc)₃ in dichloroethane gave rise to a mixture of products **94** and **95** in the ratio 1:9.

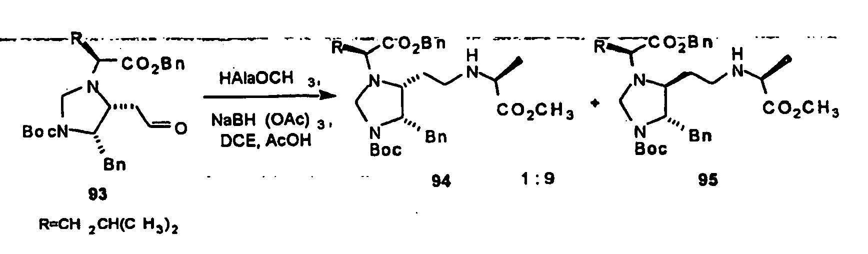

The aldehyde **93** (50 mg, 0.1 mmol) was dissolved in 1,2-dichloroethane (5 mL) and alanine methyl ester (~2 equivalents) and acetic acid (1drop, ~14 mg) were added. The mixture was stirred at room temperature for 5 min and then NaBH(OAc)₃. (40 mg, 2 eq.) was added and stirring continued for 30 min. The solvent was then removed *in vacuo* and the residue partitioned between EtOAc and 10% aq. Na₂CO₃, the organic layer was washed with water and brine and then dried (MgSO₄). The product contained both diastereomers in the ratio ~9:1, trans:cis. The products were purified by flash chromatography eluting with 20-45% EtOAc in light petroleum. TLC 40% EtOAc:light pet. Rf=0.43 (minor diastereomer, **94,** cis), 0.23 (major diastereomer, **95,** trans). Combined yield ~60%. Rotamers were not observed although significant peak broadening was present, as observed for the corresponding trans aldehyde. The configuration of the major product was determined by NMR (NOESY experiment). **¹H NMR** (300 MHz, CD₃CN, ref 1.94 ppm): 7.24-7.14, 10H, m's; 5.13, 2H, s, OCH₂Ph; 4.38, 1H, br, ring methylene(i); 3.97, 1H, bd, ring methylene(ii); 3.61, 3H, s, OCH₃; 3.75, 1H, ddd, J=2.7, 4.3, 8.7 Hz, PheH□; 3.50, 1H, m, LeuH. 3.13, 1H, m, PheC'H(ring); 2.97-2.88, 2H, m, AlaH +PheH(i); 2.72, 1H, dd, J=2.9, 8.7 Hz, PheH(ii); 2.33, 1H, ddd, J=11.5, 7.3, 5.5 Hz, CH₂NH(bridge)(i); 1.98, 1 H, m (dt, overlaps with solvent peak), CH₂NH(bridge)(ii); 1.53, 2H, m, Leu; 1.43, 9H(s)+l H(m), Boc+Leu; 1.35, 1 H, m, bridge CH₂(i); 1.29, 1H, m, bridge CH₂(ii); 1.06, 3H, d, J=7.0 Hz, Ala; 0.88, 6H, m, Leu, **¹³C NMR** (75 MHz, CD₃CN, ref 118.69 ppm): 177.2; 174.6; 154.5; 140.1; 137.6; 131.0; 129.9; 129.7; 129.6; 127.6; 80.61 (Boc tertiary); 67.50 (CH₂); 63.62 (CH₂); 63.5 (CH, br); 62.4 (CH, v.br); 60.67 (CH); 57.70 (CH); 52.47 (CH₂); 45.15 (CH₂); 40.65 (CH₂, v.br); 39.76 (CH₂); 32.81 (CH₂); 29.00 (CH₃, Boc); 26.21 (CH); 23.47 (CH₃); 22.88 (CH₃); 19.62 (CH₃). Mass Spectrum (ISMS) m/z 596 (MH⁺), calculated for C₃₄H₄₉N₃O₆: 595.

The diastereomeric amines were converted to the protected turn mimetic compounds **96** and **97** as described below:

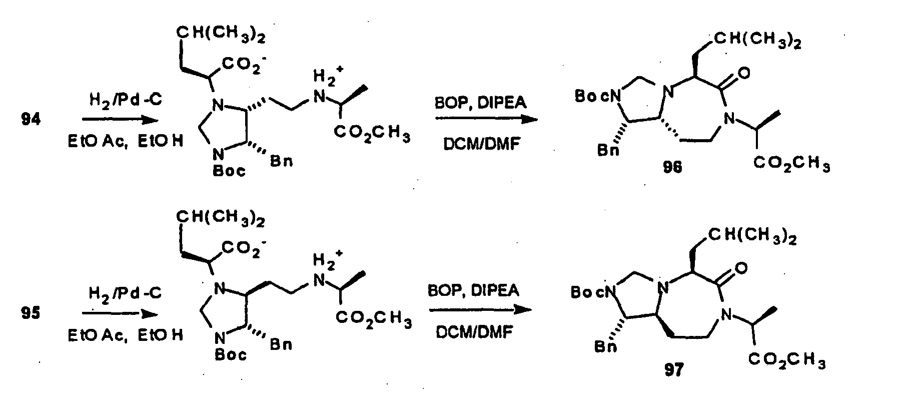

The 4,5-cis amine **94** (42 mg, 0.07 mmol) was dissolved in ethyl acetate:ethanol 10:3 (13 mL) and 35 mg of 10% palladium on activated carbon was added and the mixture hydrogenated at 32 psi H₂ for 3 h to deprotect the benzyl ester to the amino acid (MH⁺ = 506 Da).

The solution was filtered and the solvent removed *in vacuo,* then the residue was dissolved in DMF (2 mL) and diluted with CH₂Cl₂ (15 mL) and DIEA (50 mg, ~0.4 mmol) and BOP reagent (50 mg, 0.11 mmol) were added to the stirred solution at room temperature. The cyclisation was complete within a few minutes; the CH₂Cl₂ was then removed *in vacuo* and the residue diluted with ethyl acetate and washed in turn with 10% aq.Na₂CO₃/brine, water (x2), brine and then dried (MgSO₄) and the solvent removed *in vacuo* to leave a clear oil which was purified by flash chromatography eluting with 20% EtOAc in light petroleum for a yield of 25 mg (70%) of **96.** TLC 1:1 EtOAc:light pet ~0.45 The NMR spectra in CD₃CN at room temperature were significantly broadened indicating a degree of conformational interconversion slow on the NMR timescale. ¹H **NMR** (300 MHz, CD₃CN): 7.32-7.15, 5H, m, Ar; 4.88, 1H, q, J=7.1 Hz, Ala□; 4.20, 1H, bd, J=4.8 Hz, NCH₂N(a); 4.13, 1H, m, Phe; 4.09, 1H, bd, J=5.0 Hz, NCH₂N(b); 3.72, 1 H, m, Leu, 3.65, 3H, s, OCH₃; 3.52, 1H, bdd, J=10.6, 15.2 Hz, bridge CH₂CH₂N(a); 3.30-3.21, 2H, m's, CH₂CH₂N(b) and PheC'H; 2.94, 1H, bm, Phe(a); 2.76, 1H, bm. Phe□(b); 2.25 water peak; 1.9-1.4, 5H, e, Leu and bridge CH₂CH₂N; 1.29, 3H, d, J=7.1 Hz, Ala; 3.25, 9H, vbr, Boc; 0.92, 6H, d, J=6.2 Hz, Leu. **¹³C NMR** (75 MHz, CD₃CN): 173.5 (the amide and ester peaks appear to be co-incident); 154.9 (carbamate, br); 140.7; 130.7 (br); 129.6; 127.3; 80.54; 66.47; 63.83 (br); 62.36; 60.4 (very br); 56.29; 52.97; 44.77; (36.96, 36.40) very br, just resolved; 33.3 (very br); 28.78 (Boc, br); 26.86; 23.90 (br); 22.63; 15.47. Mass spectrum (ISMS) m/z 250.2 (M+H⁺), calculated for C₂₈H₃₇N₃O₆: 511 fragments (OR 60): 441, (-tBu); 397, (-Boc).

The synthesis of **97** was as for **96** but using the trans amine **95.** TLC 1:1 EtOAc:light pet. Rf=0.53. The NMR spectra in CD₃CN were were well resolved and rotamers were present in the ratio of 11:9; signals attributable to the same atom in the different rotamers are placed in parentheses where possible. **¹H NMR** (300 MHz, CD₃CN, ref 1.94 ppm): 7.34-7.16, 5H, m; 4.69, 1H, m; 4.13, 1H, d, J=4.4 Hz; 3.92, 1 H, m; (3.83, d, J=4.4; 3.79, d, J=4.4 Hz), 1 H; 3.76-3.60, 2H, m's; (3.61, s; 3.81, s), 3H, OCH₃; 3.26, 1H, m; 3.15, 1H, m; 2.99, 1H, m; 2.77, 1 H, m; 1.85-1.49, 3H, m's; (1.44, s; 1.41, s), 9H, Boc; 1.30, 3H, d, J=7.2 Hz, Ala, 1.36-1.24, 2H, m; 0.98-0.91, 6H, m. **¹³C NMR** (75 MHz, CD₃CN, ref 118.69 ppm): 174.4; 173.3; 154.6; (140.54, 140.49); 130.7; 130.6; 129.8; 127.6; (80.65, 80.54), Boc tertiary; (66.12, 65.48, 65.21, 64.90) 2xCH; 60.67, CH₂; (56.82, 56.74), CH; (56.41, 56.24), CH; 52.87, CH₃; (46.19, 46.12), CH₂; (40.72, 39.84), CH₂; 39.16, CH₂; 30.44, CH₂; (29.03, 28.93) Boc; (25.64, 25.58), CH; 24.19, CH₃; 22.43, CH₃; 15.76, CH₃. Mass Spectrum (ISMS) m/z 488 (MH⁺), calculated for C₂₈H₃₇N₃O₆: 487.

### Example (G). Acid catalysed isomerisation of aldehydes 93

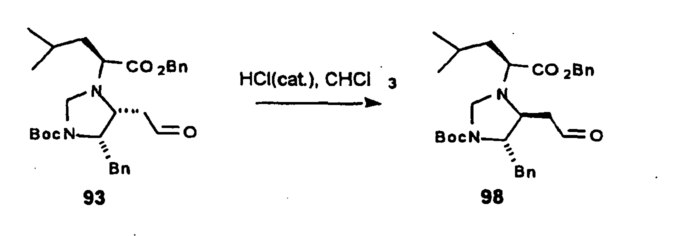

The trans (4(S)) aldehyde was obtained by the acid catalysed isomerisation of the cis diastereomer **93** in chloroform solution with catalytic HCl present. Significant decomposition to multiple unidentified by-products (most having high Rf) also occurs under the isomerisation conditions. The product was purified by flash chromatography eluting with 15% ethyl acetate in petroleum ether for a yield of about 35% **98** from crude **93. ¹H NMR** (300 MHz, CD₃CN, ref. 1.94 ppm): 9.41, t, J=1.8 Hz; 7.45-7.10, 10H, m; 5.12, 2H, m, OCH₂Ph; 4.46, 1 H, br; 4.01, 1 H, bd; 3.82, 1H, m; 3.62-3.46, 2H, m; 2.95, 1H, bdd, J=13.0, 4.4 Hz; 2.81, 1H, dd, J=13.2, 8.0 Hz; 2.37, 2H, m (ABq of dd, J_{AB}=31, J_{ddA}=4.6, 1.8 Hz; J_{ddB}=7.2, 2.1 Hz), aldehyde; 1.75-1.25, 12H, e (1.4, bs, Boc); 0.9, 6H, **bm. ¹³C NMR** (75 MHz, CDCl₃): 202.9; 174.5; 154.4; 139.7; 137.5; 131.0; 129.9; 129.8; 129.7; 127.7; 80.81; 67.61; 64.03 (br); 63.18 60.49; 59.9 (br); 47.0 (br); 45.95; 39.88; 28.96 (Boc); 26.12; 23.25; 22.97.

### Example (H). Synthesis of a turn mimetic II(i)

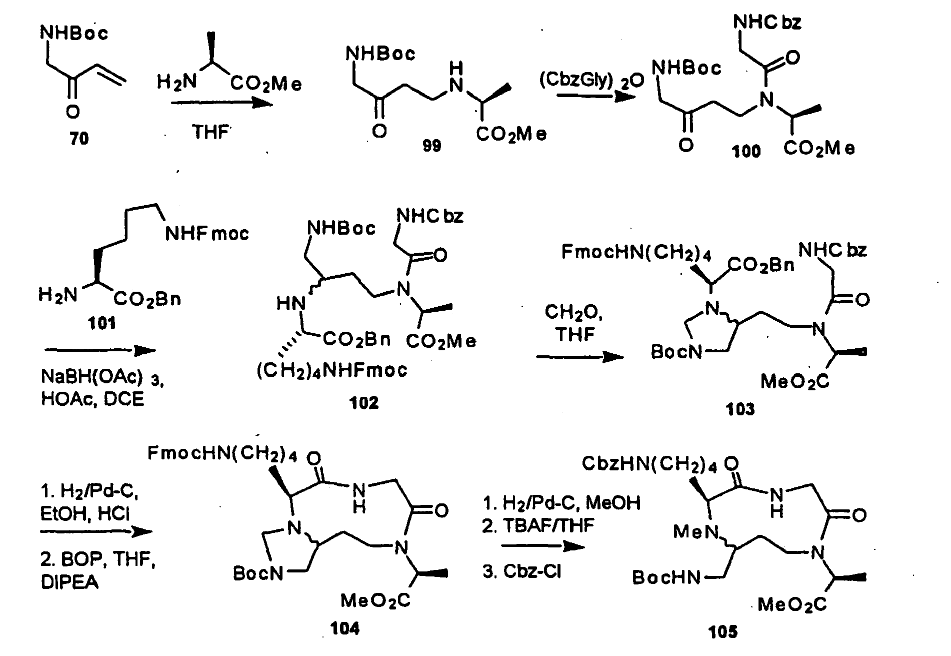

Compound **70** was prepared as described above, and reacted with alanine methyl ester to form **99** using the same method previously described for the synthesis of **71.** The crude amino ketone **99** (1.22g) was reacted with Cbz-glycine symmetric anhydride (synthesised from 1.95g CbzGlyOH and 9.3mls 0.5M dicyclohexylcarbodiimide in dichloromethane) and 0.6g DIEA in dichloromethane. The reaction was stirred at room temperature for 10 hours then diluted with ether (any DCU precipitate was filtered off) and the ether solution was washed with 1 M HCl, aqueous sodium bicarbonate then brine and then dried over magnesium sulfate (removed by filtration) and the volatiles removed under reduced pressure to leave the crude product as an oil which was purified by flash chromatography eluting with 2:1 ethyl acetate:light petroleum ether, yield of **100** was 1.8g (90%). Reductive amination of **100** with **101** derived from the deprotection of BocLys(Fmoc)OBn (TFA. CH₂Cl₂) is carried out by the previously described method for the formation of **73** (71% yield after flash chromatography eluting with 2:1 to 3:1 ethyl acetate:light petroleum). The product amine **102** was dissolved in ethyl acetate and formalin added to the stirred solution resulting in the formation of imidazolidine **103.** The ethyl acetate solution was washed with aqueous sodium bicarbonate, water (twice), brine and then dried over magnesium sulfate (removed by filtration) and the volatiles removed under reduced pressure to leave the crude product as an oil which was purified by flash chromatography eluting with 3:2 ethyl acetate:light petroleum ether (yield >75%). The protected pre-cyclisation compound **103** (400 mgs) was dissolved in 0.1M ethanolic HCl (20 mls) and hydrogenated with 250mgs of 10% Pd-C. The hydrogenation was complete after 7 hours (about 40 psi H₂, room temperature). The solution was filtered through a celite pad to remove the catalyst and 50 mls of DMF added. Volatiles (ethanol) were removed under reduced pressure then a solution of BOP reagent (300 mgs) and DIEA (300 mgs) in 150 mls of DMF was added and the mixture stirred at room temperature for 15 minutes. Most of the DMF was removed under reduced pressure and the residue dissolved in ethyl acetate and washed with 1 M HCl, aqueous sodium bicarbonate, water (twice), brine and then dried over magnesium sulfate (removed by filtration) and the volatiles removed under reduced pressure to leave about 300 mgs of crude product **104.** The crude product was dissolved in 30 mls methanolic HCl (0.1 M) and hydrogenated (200mgs Pd-C, 40psi H₂) for 24 hours reducing the imidazolidine to an N-methyl group. The catalyst was filtered off (celite) and the solvent removed under reduced pressure, the residue was then treated with tetrabutylammonium fluoride in THF to remove the FMOC group. The free amine was then reprotected by addition of benzyl chloroformate (65 mgs) and DIEA (100 mgs). After stirring for 1 hour ethyl acetate was added and the organic layer was washed with 1 M HCl, water, then brine, dried over magnesium sulfate (removed by filtration) and the volatiles removed under reduced pressure to leave an oil which was purified by flash chromatography eluting with 3-5% ethanol in chloroform for a yield of about 40% of **105** based on **103.**

### APPENDIX

### Previous reports of the turn mimetic system I(i)

A theoretical study of the suitability of various heterocyclic systems as turn mimetics has been published (Alkorta *et al.,* 1996). The study included the 1,3,5-substituted-1,4-diaza-2-oxocycloheptane system (the basis of the turn mimetics described herein). No synthesis was described or referenced in the paper for this mimetic system, in contrast to other known mimetic systems where the synthesis was referenced.

Although a search of the Chemical Abstracts registry file on the substructure of the turn system gave only the above modelling study, we are aware of a reported synthesis of the turn mimetic system by a different synthetic approach. The alternative approach was described in a poster presented at the 23rd European Peptide Symposium (1994), and repeated at the end of a review published in the Bulletin of the Chemical Society of Belgium (Guilbourdenche *et a*/., 1994) and again the following year (Ma *et al.,* 1995). Our research and other literature results do not support this alternative method, the reports are in error and do not represent a reduction to practice. We have repeated the cyclisation reaction described by Ma *et al.,* 1995 and confirmed by NMR analysis and chemical transformation that the actual product is a structural isomer, not the turn mimetic claimed. The synthesis and analyses and other material in support of the assertion that the method of Ma et al. does not represent a reduction to practice are presented below.

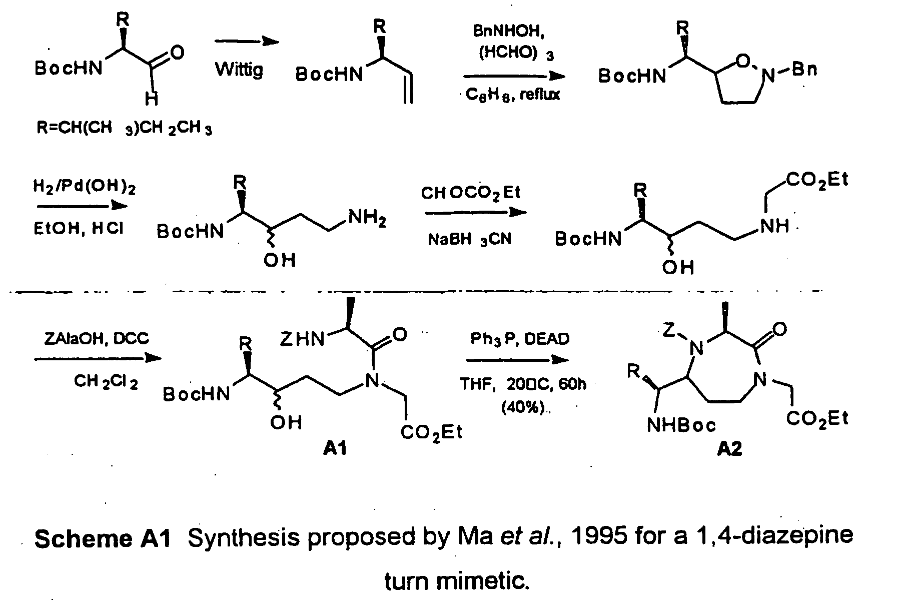

The key step in the proposed synthesis of Ma *et al.,* 1995 is the cyclisation of **A1** to the protected target **A2** using the Mitsunobu reagents. We repeated the synthesis of the cyclisation precursor by our own methods as described below.

The alcohol **A1** was more conveniently prepared by the conjugate addition method described earlier than as illustrated in Scheme A1 (4 steps vs. 6 steps). The procedure used is summarised in Scheme A2.

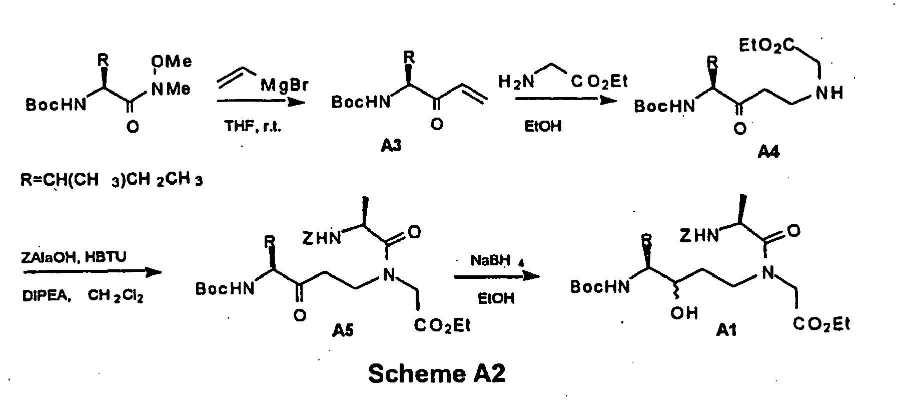

Thus the Weinreb amide of Boc isoleucine was reacted with vinyl Grignard in THF to give the α, β unsaturated ketone **A3** by the following procedure: Boc-isoleucine-N-methoxy-N-methylamide (2.25 g, 8.2 mmol) was dissolved in anhydrous THF (20 mL) and cooled to 0°C under nitrogen. To the stirred solution was added vinyl magnesium bromide in THF (20 mL of a ~1M solution) over 5 min. The reaction was very slow at 0°C (negligible progress over 1 h), but much faster at room temperature (~70% products after 20 min). After stirring at room temperature for 90 min the reaction was poured into crushed ice/1M HCl and extracted with ether. The organic layer was washed with 0.5M HCl, water, aq.NaHCO₃ then brine and then dried over MgSO₄. The crude product was formed in good yield and purity and was used directly for the next reaction. TLC 25%EA/light pet. Rf=0.64. **¹H NMR** (300 MHz, CDCl3): 6.50, 1H, dd, J = 10, 17 Hz; 6.37, 1H dd, J = 1, 17 Hz; 5.85, 1 H, d, J = 10 Hz; 5.23, 1 H, bd, J = 7 Hz; 4.58, 1 H, dd, J = 4, 8 Hz; 1.88, 1H, m; 1.45, 9H, s; 1.32, 1 H, m; 1.10, 1H, m; 0.98, 3H, d, J = 7 Hz; 0.90, 3H, d, J = 7 Hz. **¹³C NMR** (75 MHz, CDCl₃): 199.0; 155.7; 134.0; 129.6; 79.60; 61.71; 37.50; 28.28 (Boc); 24.09; 16.04; 11.61.

Reaction of **A3** with glycine ethyl ester in ethanol to give **A4** by the following procedure: Glycine ethyl ester hydrochloride (1.0 g, 7.1 mmol) was reacted with **A3** (1.1 g, ~4.7 mmol) and DIEA (450 mg, 3.5 mmol) in ethanol (20 mL) at room temperature overnight. The reaction was diluted with ether (100 mL) and extracted in turn with aq. NaHCO₃ and water (x3). Petroleum ether was added (100 mL) and the solution extracted with 0.5M HCl:MeOH 4:1 (x3) (discard the organic layer). The acid washings were immediately neutralised with solid NaHCO₃ and then extracted with ethyl acetate and the ethyl acetate layer washed with water then brine and then dried over MgSO₄. Evaporation of the solvent *in vacuo* left 800 mg (~50%) of crude product of sufficient purity for use in the next reaction. TLC EtOAc Rf=0.52 **¹³C NMR** (75 MHz, CDCl₃): 209.0; 171.7; 155.8; 79.57; 63.95; 60.76; 50.67; 43.69; 40.82; 36.74; 28.19 (Boc); 24.05; 16.01; 14.08; 11.51. Mass Spectrum (ISMS) m/z 345 (MH⁺), calculated for C₁₇H₃₂N₂O₅: **344.**

The amino ketone **A4** (690 mg, 2 mmol) was then coupled with Z-alanine to give **A5** using standard solution phase coupling procedure with HBTU reagent and DIEA in CH₂Cl₂/THF. The crude product was purified by flash chromatography eluting with 30% EtOAc in light petroleum for a yield of 94% (1.03 g). TLC EtOAc:light pet. 1:2 Rf=0.25. **¹H NMR** (300 MHz, CDCl₃): 7.34, 5H, m; 5.68, 1H, bm; 5.18-5.02, 3H, m's; 4.72, 0.5H, m; 4.48-4.07, 5H, m's; 3.88-3.54, 2.5H, m's; 2.75-2.05, 2H, m's; 1.89, 1 H bs; 1.44, 1.43: 9H, 2s, Boc; 1.38, 1.5H, d, J = 6.9 Hz (alaH□, one rotamer); 1.34-1.28, 5.5H, m's; 1.07, 1H, m; 1.00-0.82, 6H, m's. **¹³C NMR** (75 MHz, CDCl₃), signals due to the equivalent carbon in different rotamers are grouped in parentheses where possible: (209.0, 207.9); (173.39, 173.25); (169.15, 168.84); 155.75, 155.67, 155.56, 155.33: carbamate signals; 136.20; 128.31; 127.91; 127.80; (79.72, 79.57); 66.60; (64.01, 63.85); (61.61, 61.09); (50.96, 48.65); (46.63, 46.57); (43.75, 43.23); (40.02, 39.07); (36.56, 36.29); 28.14 (Boc); (24.09, 24.03); 18.74; 15.92; 13.85; (11.44, 11.38). Mass Spectrum (ISMS) m/z 550 (MH⁺), calculated for C₂₈H₄₃N₃O₈: 549

The ketone **A5** (430 mg, 0.78 mmol) was dissolved in ethanol (5 mL) and NaBH₄ (15 mg, 0.40 mmol) added to the stirred solution at room temperature, and stirring continued for 1 h. The solvent was removed *in vacuo* and the residue dissolved in ethyl acetate and washed with 1M HCl, water, aq. NaHCO₃, brine and then dried over MgSO₄. The residue after solvent evaporation was purified by flash chromatography eluting with ethyl acetate:light petroleum ~1:1 (some separation of diastereomers occurred) for an approximately quantitative yield of the alcohol **A1.** TLC EtOAc:light pet. 1:1 Rf=0.28. **¹H NMR** (300 MHz, CDCl₃), late eluting fractions, rotamers/diastereomers >2:1: 7.39-7.29, 5H, m; 5.80, 1 H, d, J=9 Hz; 5.15, 1 H, d, J=12 Hz; 5.11-5.49, ~1H, m; 4.96, ~1H, d, J=12 Hz; 4.67-4.42, ~1H, m's; 4.19, ~2H, bq, J=7.2 Hz; 4.03-3.88, ~2H, bm; 3.88-3.40, ~4H, m's; 3.30-3.09, 1H, m; 1.96-1.66, ~2H, m; 1.55, ~1H, m; 1.42, 9H, s, (Boc); 1.331.33, d, J=7 Hz; 1.28, t, J=7.2 Hz; 1.15, d (minor isomer), J=6.8 Hz; 1.37-1.05 ~8H; 1.0-0.82, ~6H, m's. **¹³C NMR** (75 MHz, CDCl₃), major peak only shown unless otherwise indicated: 174.0; 169.0; 156.4; 156.3; 135.9; 128.4; 128.1; (128.0, minor isomer); 127.9; 78.92; 66.96; (66.56, minor isomer); 66.11; 61.26; 59.49; 47.74; 46.10; 45.24; 34.38; 31.31; 28_{:}30 (Boc); 22.29; 18.85; 16.41; 14.00; 11.90. -Mass Spectrum (ISMS) m/z 552 (M+H⁺), calculated for C₂₈H₄₅N₃O₈: 551.

The alcohol **A1** was reacted with the Mitsunobu reagents as described by Ma *et al.*, 1995 (Scheme 4.37) as follows: The alcohol **A1** (150 mg, early eluting fraction) was dissolved in dry THF and triphenylphosphine (71 mg) added. To the stirred solution at room temperature under nitrogen was added DEAD (43 uL), and stirring continued for 24 h. Analysis of the crude reaction revealed the formation of a dehydration product (M+H⁺=534 Da) in moderate yield. Another equivalent of triphenylphosphine/DEAD was added and stirring continued for a further 48 h. The solvent was removed *in vacuo* and the residual oil dissolved in ether/petroleum ether and left to stand to encourage the precipitation of the triphenylphosphine oxide and diethoxycarbonyl hydrazine (white solid, filtered off). The oil remaining after evaporation of the filtrate was purified by flash chromatography eluting with petroleum ether and 10-100% ether in petroleum ether, yield was ~40% (60 mg). TLC ethyl ether Rf=0.61. The NMR spectra were quite complex, as may be expected from the possible mixture of diastereomers/ rotamers. However, it was possible to clearly identify the alanine spin system with H at 4.71 ppm (1H, broad pentuplet, J~8Hz). 1 D decoupling experiments were performed: irradiation at 4.7 ppm-caused the collapse of two signals to singlets, a doublet centred on 1.40 ppm (J=7Hz, alanine H), and a broad doublet (1H, J=8Hz) at 5.62ppm (alanine NH). These assignments were confirmed by irradiation at 1.4 ppm which caused collapse of the multiplet at 4.71 ppm to a doublet with J=8Hz. The presence of the NH proton in the alanine spin system rules out the turn mimetic **A2** proposed by Ma *et al.,* 1995 as a possible structure for the product, and leaves open the possibility of **A6** or **A7** (Scheme A3) which we felt were more probable products, as the true structure. **¹H NMR** (300 MHz, CDCl₃): (selected peaks) 5.62, ~1H, bd, J=8 Hz; 4.71, ~1H, m(q); 1.40, d, J=6.8 Hz. Decoupling experiments: irradiate 1.4 ppm -> 4.71 = doublet, J=8 Hz; irradiate 4.71 ppm -> 1.4 = singlet, 5.62 = singlet. **¹³C NMR** (75 MHz, CDCl₃): the spectra were difficult to analyse due to the presence of rotamers/diastereomers, peak broadening and impurities which co-eluted. There were a couple of notable features: (i) the appearance of a new peak at the relatively unusual shift of 160.7 ppm possibly due to the carbamate derived oxazoline carbon (only one carbamate resonance was observed, 155.5 ppm), and (ii) the downfield shift of the tertiary Boc carbon resonance which was observed at 81.22 ppm, whereas NHBoc tertiary carbon shifts are normally at a shift upfield of 80 ppm (e.g. 78.9 in the alcohol precursor). Mass Spectrum (ISMS) m/z 534 (MH⁺), calculated for C₂₈H₄₃N₃O₇: 533.

To confirm the results of the NMR analysis a further experiment was carried out. The product material was hydrogenated (EtOH, Pd-C) to remove the Z group. If the product has structure **A6** or **A7** then the amine will now be free to form the diketopiperazine **A8,** a facile reaction in such a system, Scheme A3. If any of the target turn mimetic **A2** is present then it will be deprotected to the (very stable) free amine **A9** and be easily detected in the ionspray mass spectrum (ISMS). analysis of the product mixture from the hydrogenation revealed the presence of a mass peak corresponding to the diketopiperazine (MH⁺=354Da), but no trace whatsoever of **A9** (MH⁺=400Da).

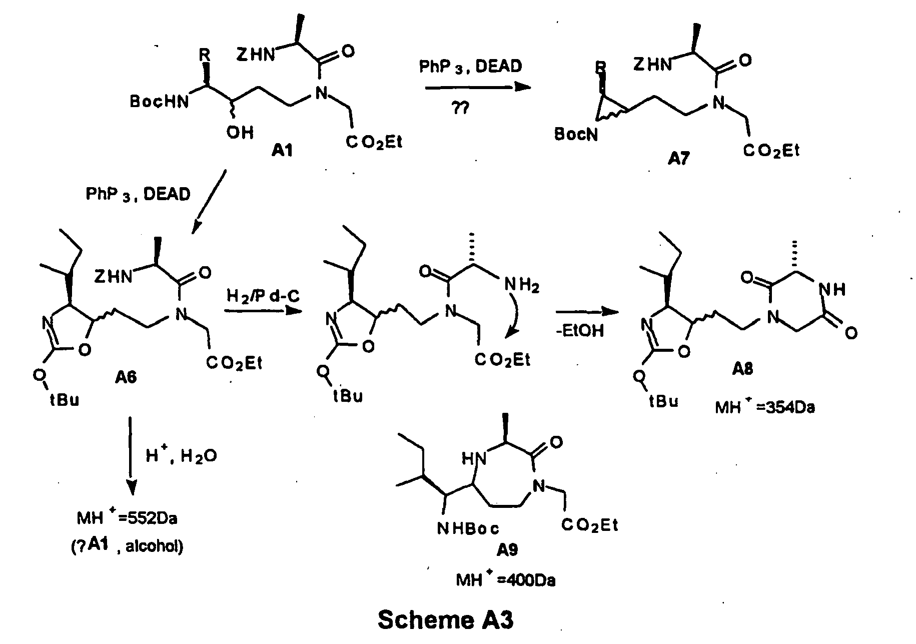

Finally, it was also observed that the cyclisation product (which we propose to be **A6**) was easily hydrolysed by dilute aqueous acid (e.g. room temperature 0.1% aq. TFA, 12 h), back to the alcohol **A1** (or a compound of the same mass). This last observation is more consistent with the product structure being the oxazoline **A6** rather than the aziridine **A7** as the oxazoline is more probably subject to facile hydrolysis by aqueous acid, the facile hydrolysis is entirely inconsistent with the structure **A2** proposed by Ma *et al.*, 1995

In further support of **A6** as the product structure, peptide alcohols similar in structure to **A1** have been reported to form oxazolines, (Galéotti *et al.*, 1992) for example: 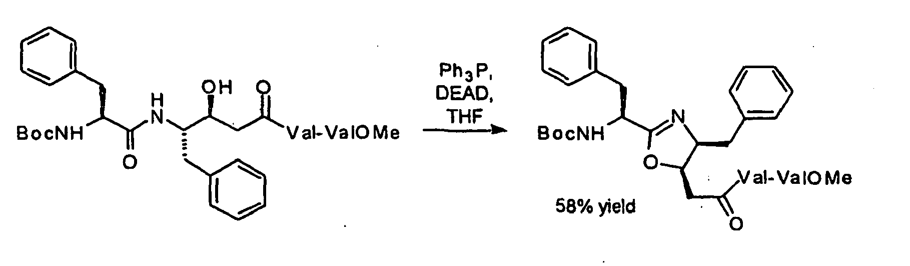 Other evidence against formation of **A2** by the Mitsunobu reaction as proposed by Ma *et al.*, 1995 is presented below.

### (1) Difficulty of forming seven membered rings via the Mitsunobu reaction

### (a) Literature precedent

The literature on-the-formation of-cyclic amines and amides with the Mitsunobu reaction contains numerous examples of the formation of 3-6 membered rings (Carlock and Mack, 1978; Robinson *et al.*, 1983; Pfister, 1984; Kelly *et al.*, 1986; Henry *et a*/*.,* 1989; Bernotas and Cube, 1991), but very few cases of seven membered ring formation. In one paper on the cyclisation of amino alcohols the faliure to form a simple seven membered target is specifically described (Bemotas and Cube, 1991) In the organic reactions entry on the Mitsunobu reaction (Hughes, 1992) three instances of seven membered ring formation with carbon-nitrogen bond formation are described: all three involve a primary alcohol, two occur in polycyclic systems and appear to be special cases, and the third involves alkylation of a hydroxamide - far easier than an amide due to higher NH acidity.

There appears to be no literature precedent for the formation of a seven membered ring to a simple amide or carbamate nitrogen. In addition there is little precedent for secondary amide N-alkylation with hindered secondary alcohols, as is proposed to occur in the formation of **A2**.

### (b) Synthetic studies

Extensive studies on the use of the Mitsunobu reaction for the formation of the target system were carried out in our laboratories prior to becoming aware of the proposed synthesis. In our hands this approach was ineffective. The key reactions are described in Schemes **A4** and **A5**.

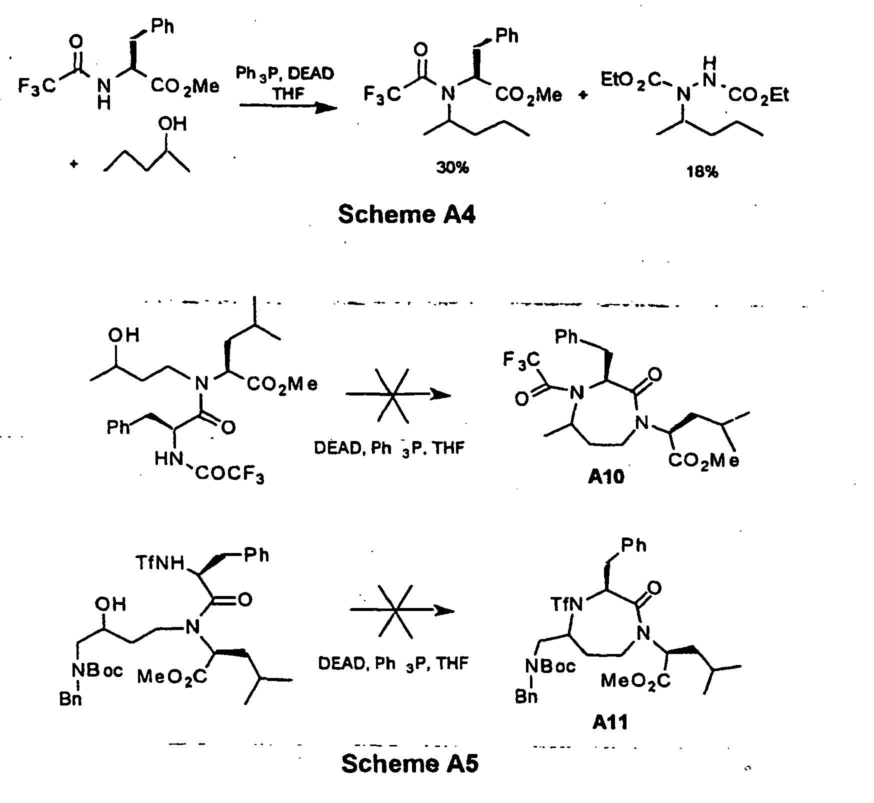

The formation of the alkylation product was somewhat successful in the intermolecular reaction (Scheme A4), but this success was not repeated in cyclic systems (Scheme A5). No significant amount of the target cyclic products **A10** or **A11** was detected.

### (2) Competing reactions -oxazoline and aziridine formation

Cyclisation of β-hydroxy amide derivatives A12 with the aim of forming β-lactams **A13** also results in the formation of the aziridine **A14** and oxazoline **A15** products shown in Scheme A6 (Hughes, 1992). Another example of oxazoline formation was described above (Galéotti *et* a/., 1992).

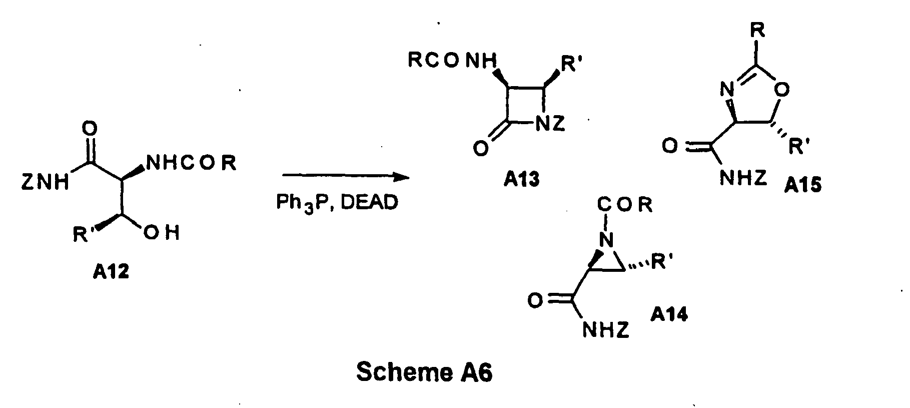

As the Mitsunobu reaction is relatively effective for the formation of small ring sizes, it is quite probable that the formation of aziridines and oxazolines will compete with other possible cyclisations, other factors being equal. Such competition can take place in the proposed synthesis, the products would then be **A6** and/or **A7,** Scheme **A3**. Both the aziridine and oxazoline are isomeric with the target compound **A2,** possibly leading to their confusion with the target, a situation easily resolved by ¹H NMR as we demonstrated above.

In summary, the proposed method is in error because:
● We have repeated the cyclisation and found the product to be a structural isomer of the target, probably the oxazoline **A6.**

This finding is supported by:-
● Literature contrindications (competing cyclisations favoured), lack of precedent (seven membered rings difficult to form by the Mitsunobu reaction).
● Extensive studies in our laboratories which indicate the Mitsunobu approach is generally ineffective for the synthesis of the turn mimetics.

### REFERENCES

Abdel-Magid et al., 1996, Journal of Organic Chemistry 61 3849-3862
Alkorta et al., 1996, J. Mol. Model. 1 16-25.
Arrhenius et al., 1987, The chemical synthesis of structured peptides using covalent hydrogen-bond mimics. In Protein Structure, Folding and Design 2 Ed. D. Oxender. Alan R. Liss, Inc. 453-465.
Ball, J.B. and Alewood, P.F., 1990, Journal of Molecular Recognition 3(2) 55-64.
Ball et al., 1993, Tetrahedron 49(17) 3467-3478.
Basile et al., 1994, Journal of Organic Chemistry 59 7766-7773.
Bernotas, R.C. and Cube, R.V., 1991, Tetrahedron Letters 32(2) 161-164.
Bocoum et al., 1991, Tetrahedron Letters 32 1367-1370.
Bodansky, M. and Bodansky, A., 1984, The Practice of Peptide Synthesis. Berlin-Heidelberg, Springer-Verlag.
Borch et al., 1971, J. Am. Chem. Soc. 93 2897-2904.
Boutin, R.H. and Rapoport, H., 1986, Journal of Organic Chemistry 51 5320-5327.
Brown, H.C., 1975, Organic Syntheses Via Boranes. New York, Wiley.
Brown et al., 1986, Tetrahedron 42 5515.
Brown, H.C. and Bhat, K.S., 1986, J. Am. Chem. Soc. 108 293.
Brown, H.C. and Jadhav, P.K., 1983, J. Am. Chem. Soc. 105 2092-2093.
Brown, H.C. and Jadhav, P.K., 1984, Journal of Organic Chemistry 49 4089.
Brown, H.C. and Krishnamurthy, 1979, Tetrahedron 35 567-607.
Brown et al., 1980, Synthesis : 151.
Brown et a/. ,1990,. J. Am. Chem. Soc. 112 2389.
Callahan et al., 1992, JMedChem 35 3970-3972.
Carlock, J.T.and Mack, M.P., 1978, Tetrahedron Letters 52 5153-5156.
Carpino et al., 1994, Tetrahedron Letters 35 2279-2280.
Carpino et al., 1990, J. Am. Chem. Soc. 112 9651-9652.
Chalmers, D.K. and Marshall, G.R., 1995, J. Am. Chem. Soc. 117(22) 5927-37.
Chen et al., 1992, PNAS 89(Biochemistry) 5872-5876.
Cupps et al., 1985, Journal of Organic Chemistry 50 3972-3979.
Ehrlich et al.,1993. Tetrahedron Letters 34 4781-4784.
Farmer, P.S. and Arièns, E.J., 1982, Topics in Peptide Science 362-365.
Fehrentz, J.-A. and Castro, B.,1983, SYNTHESIS 676-678.
Frigerio, M. and Sangostino, M., 1994, Tetrahedron Letters 35 8019-8022.
Früchtel, J.S. and Jung, G., 1996, Angew. Chem. Int. Ed. Engl. 35 17-42.
Galéotti et al., 1992, Tetrahedron Letters 33(20) 2807-2810.
Gallop et al., 1994, Journal of Medicinal Chemistry 371233-1251.
Gardner et al., 1993, Tetrahedron 49(17) 3433-3448.
Giannis, A. and Kolter, T., 1993, Angew. Chem., Int. Ed. Engl. 32 1244-1267.
Gordon et al., 1994, Journal of Medicinal Chemistry 37 1385-1401.
Greene, T.W. and Wuts, P.G., 1991, Protective Groups. New York, John Wiley & Sons.
Gribble and Nutatits,1985, Org. Prep. Proc. Int. 17 317.
Griffith, W.P. and Ley, S.V., 1990, Aldrichimica Acta 23 13-19.
Guilbourdenche et al., 1994, Bull. Soc. Chim. Belg. 103(1) 1-8.
Henry et al., 1989, Tetrahedron Letters 30(42) 5709-5712.
Hirschmann et al., 1993, J. Am. Chem. Soc. 115 12550-12568.
Hirschmann et al., 1992, J. Am. Chem. Soc. 114 9217-9218.
Hirschmann et al., 1996, Tetrahedron Letters 37 5637-5640.
Hölzemann, G., 1991, Kontakte (Darmstadt) 3-12.
Hölzemann, G.,1991, Kontakte (Darmstadt) 55-63.
Hudlicky, M., 1990, Oxidations in Organic Chemistry. Washington, American Chemical Society.
Huffman et al., 1989, Mimics of Secondary Structural Elements of Peptides and Proteins. Synthetic Ppetides: Approaches to Biological Problems. Alan R. Liss, Inc. 257-266.
Huffman et al., 1988, Reverse turn mimics. Peptides: Chemistry and Biology, Proceedings of the Tenth American Peptide Symposium Ed. G. R. Marshall. Leiden, The Netherlands, ESCOM. 105-108.
Hughes, D.L., 1992, Organic reactions 42 335-656.
Humphries et al., 1994, Exp. Opin. Ther. Patents 4(3) 227-235.
Hutchins, R.O. and Natale, N.R., 1979, Org. Prep. Proc. Int. 11 203-241.
Jurczak, J. and Golebiowski, A., 1989, Chem. Rev. 89 149-164.
Kahn, M., 1993, SYNLETT 821-826.
Kahn, M., 1996, Library of conformationally constrained reverse- turn peptides. 64 pp. PCT Int. Appl., Molecumetics, Ltd., USA.
Kelly et al., 1986, Journal of Organic Chemistry 51 95-97.
Kessler et al., 1995, Letters in Peptide Science 2 155-160.
Knapp et al., 1992, Journal of Organic Chemistry 57 6239-6256.
Koskinen, A.M.P. and Rapoport, H., 1989, Journal of Organic Chemistry 54 1859-1866.
Kramer, G.W. and Brown, H.C., 1974, Journal of Organometallic Chemistry 73 1-15.
Kramer, G.W. and Brown, H.C., 1977, Journal of Organometallic Chemistry 132 9-27.
Krstenansky et al., 1982, Biochem. Biophys. Res. Commun. 109 1368-1374.
Kuntz, I.D.; 1972, J. Am. Chem. Soc. 94 4009-4012.
Lewis et al., 1973, Biochim. Biophys. Acta 303 211-229.
Ma, X. et al., 1995, Protein and Peptide Letters 347-350.
Meier, H. and Zeller, K., 1975, Angew. Chem. Int. Ed. Engl. 14 32-43.
Milner-White, E.J.,1988, Journal of Molecular Biology 204 777-782.
Nahm, S. and Weinreb, S.M., 1981, Tetrahedron Letters 22(39) 3815-3818.
Nakanishi, H. and Kahn, M., 1996, Design of Peptidomimetics. The Practice of Medicinal Chemistry. Academic Press Limited. Ch. 27, 571-590.
Newlander et al., 1993, JMedChem 36 2321-2331.
Olson et al., 1993, Journal of Medicinal Chemistry 36 3039-3049.
Pelter et al., 1988, Borane Reagents. New York, Academic Press.
Pfister; J.R.; 1984, Synthesis 969-970.
Qabar et al., 1996, Letters in Peptide Science 3 25-30.
Racherla et al., 1992, Journal of Organic Chemistry 57 6614-6617.
Ray, R. and Matteson, D.S., 1980, Tetrahedron Letters 21 449-450.
Richardson, J.S., 1981, Adv. Prot. Chem. 34 167-339.
Robinson et al., 1983, Journal of Organic Chemistry 48 5396-5398.
Rose et al., 1985, Advan. Protein Chem. 37 1-109.
Sardina, F.J. and Rapoport, H., 1996, Chem. Rev. 96 1825-1872.
Soderquist, J.A. and Najafi, M.R., 1986, Journal of Organic Chemistry 51 1330.
Szeja, W., 1985, Synthesis 983.
Thompson, L.A. and Ellman, J.A., 1996, Chem. Rev. 96 555-600.
Valle et al., 1989, International Journal of Peptide and Protein Research 33 181-190.
VanRheenen et al., 1976, Tetrahedron Letters 23 1973-1976.
Virgilio, A.A. and Ellman, J.A., 1994, J. Am. Chem. Soc. 116(25) 11580-1.
Virgilio et al., 1996, Tett. Lett. 37 6961-6964.
Wenschuh et al., 1994, Journal of Organic Chemistry 59: 3275-3280.
Wilmont, C.M. and Thornton J.M., 1990, Protein Engineering 3 479-493.
Wolf, J. and Rapoport, H., 1989, Journal of Organic Chemistry 54 3164-3173.
Yamamoto, Y. and Asao, N., 1993, Chem. Rev. 93 2207-2293.

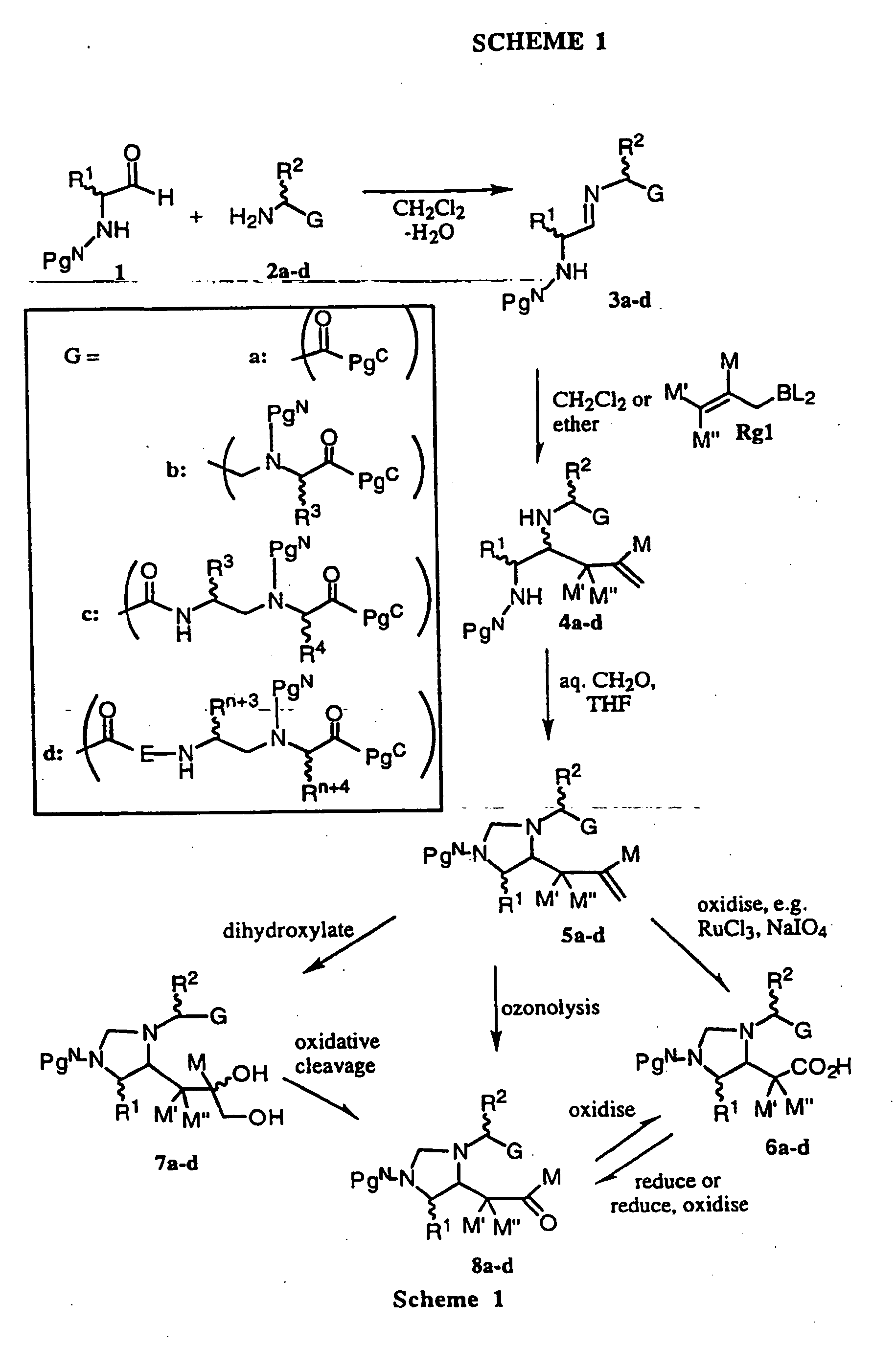

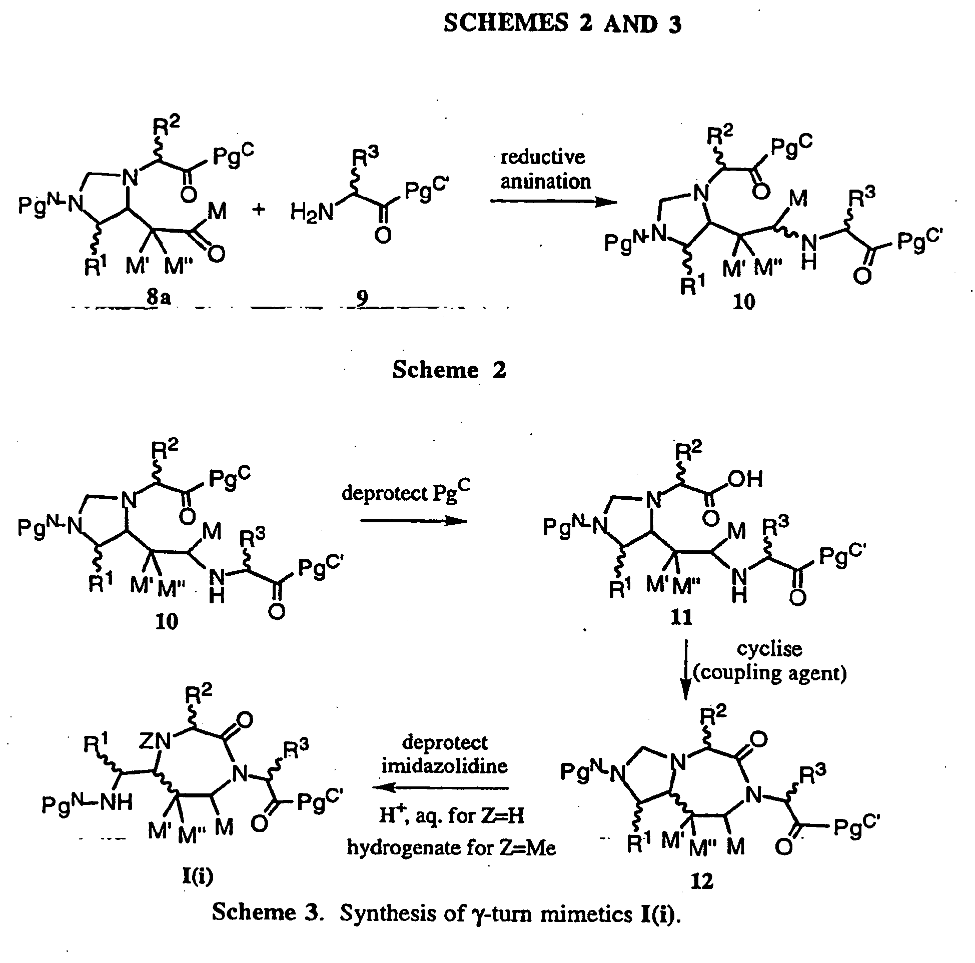

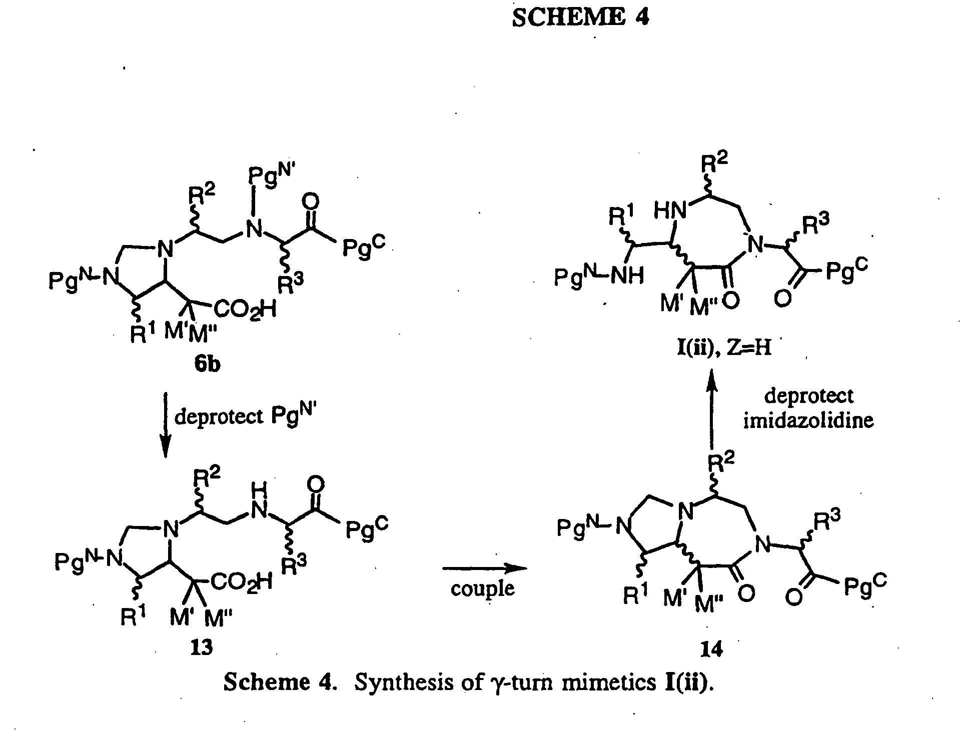

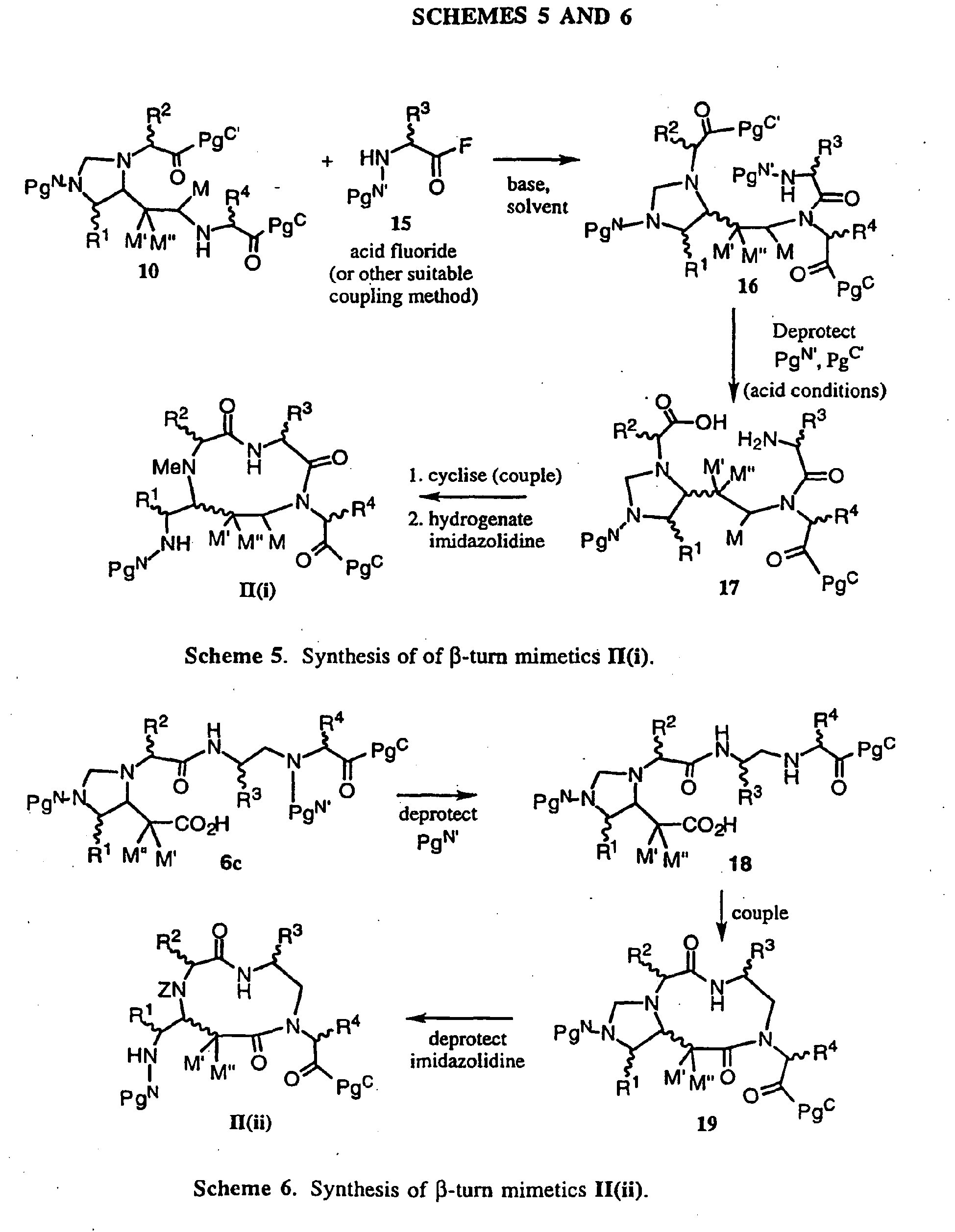

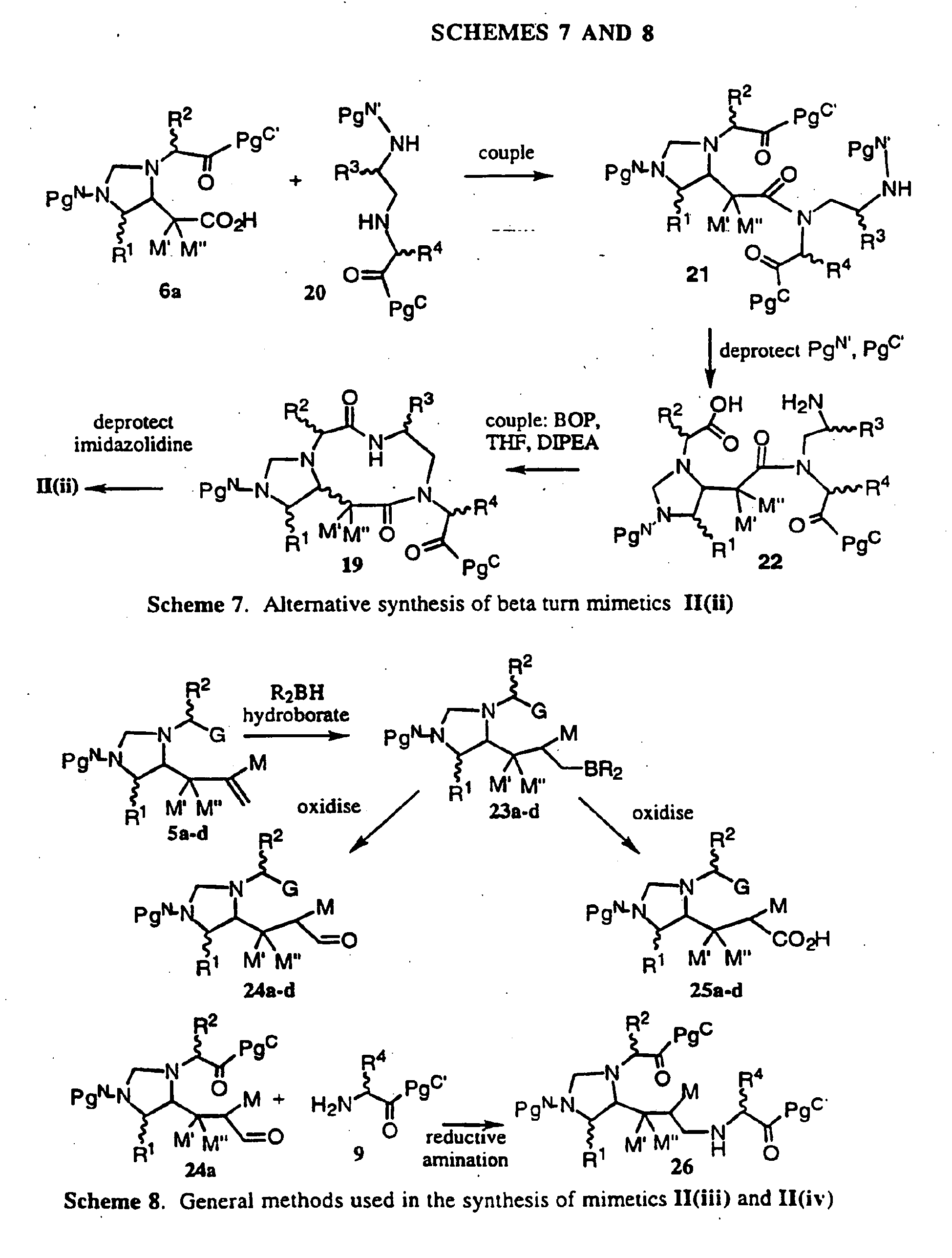

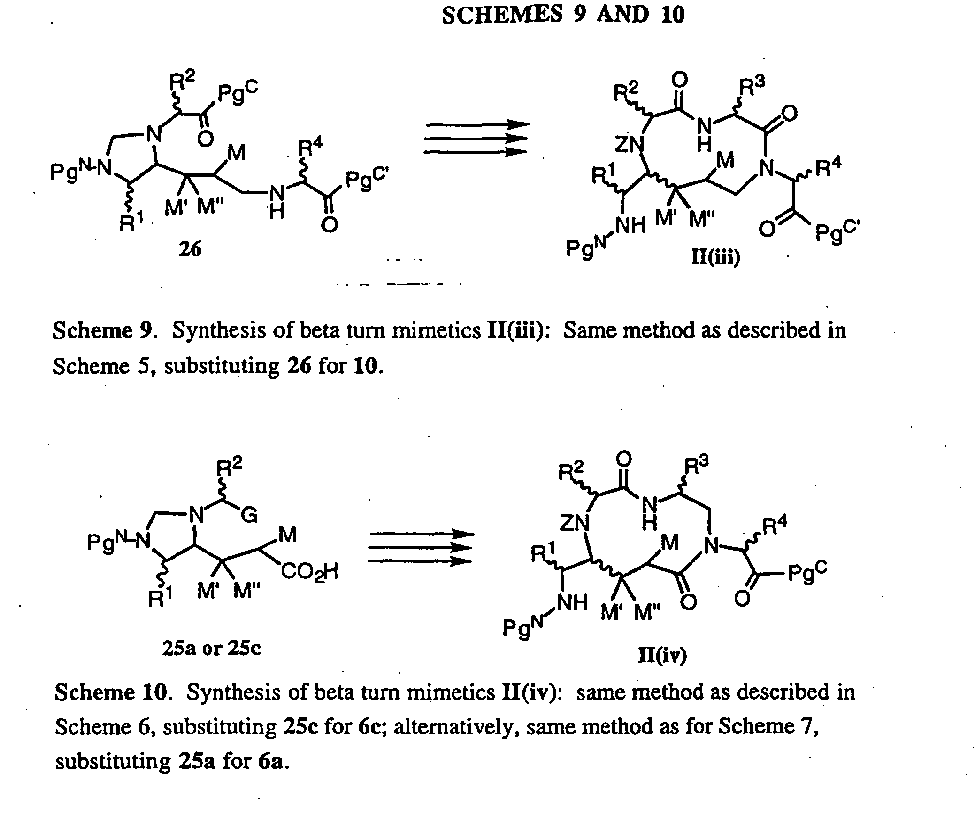

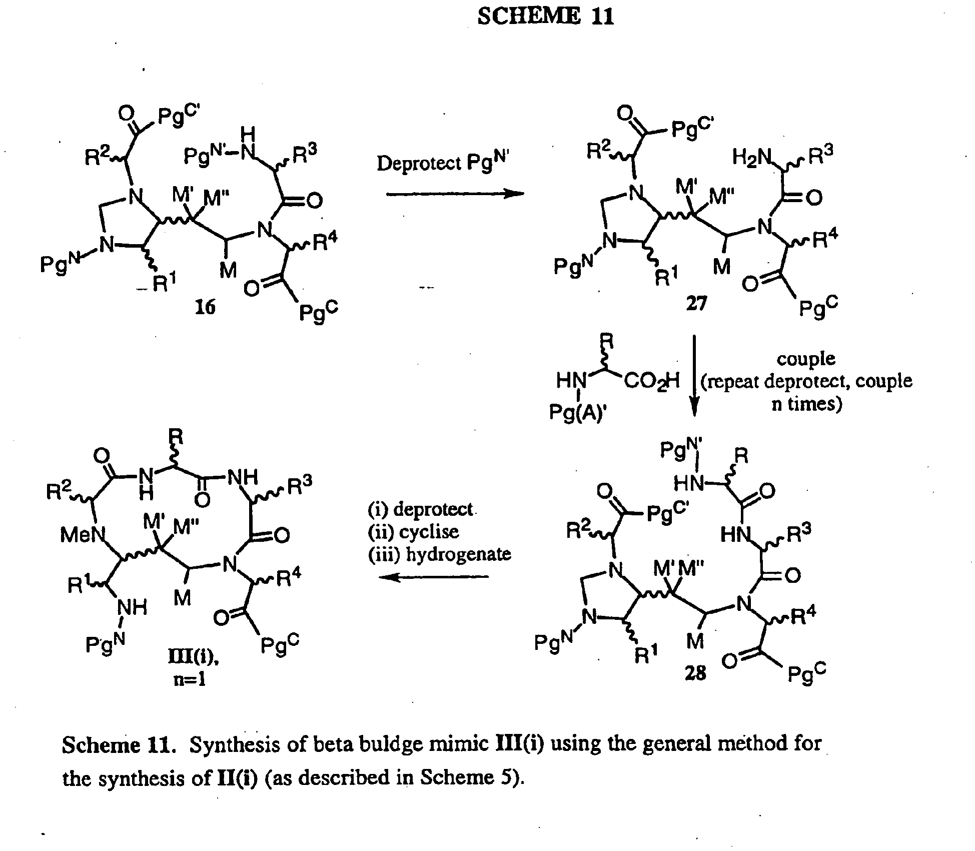

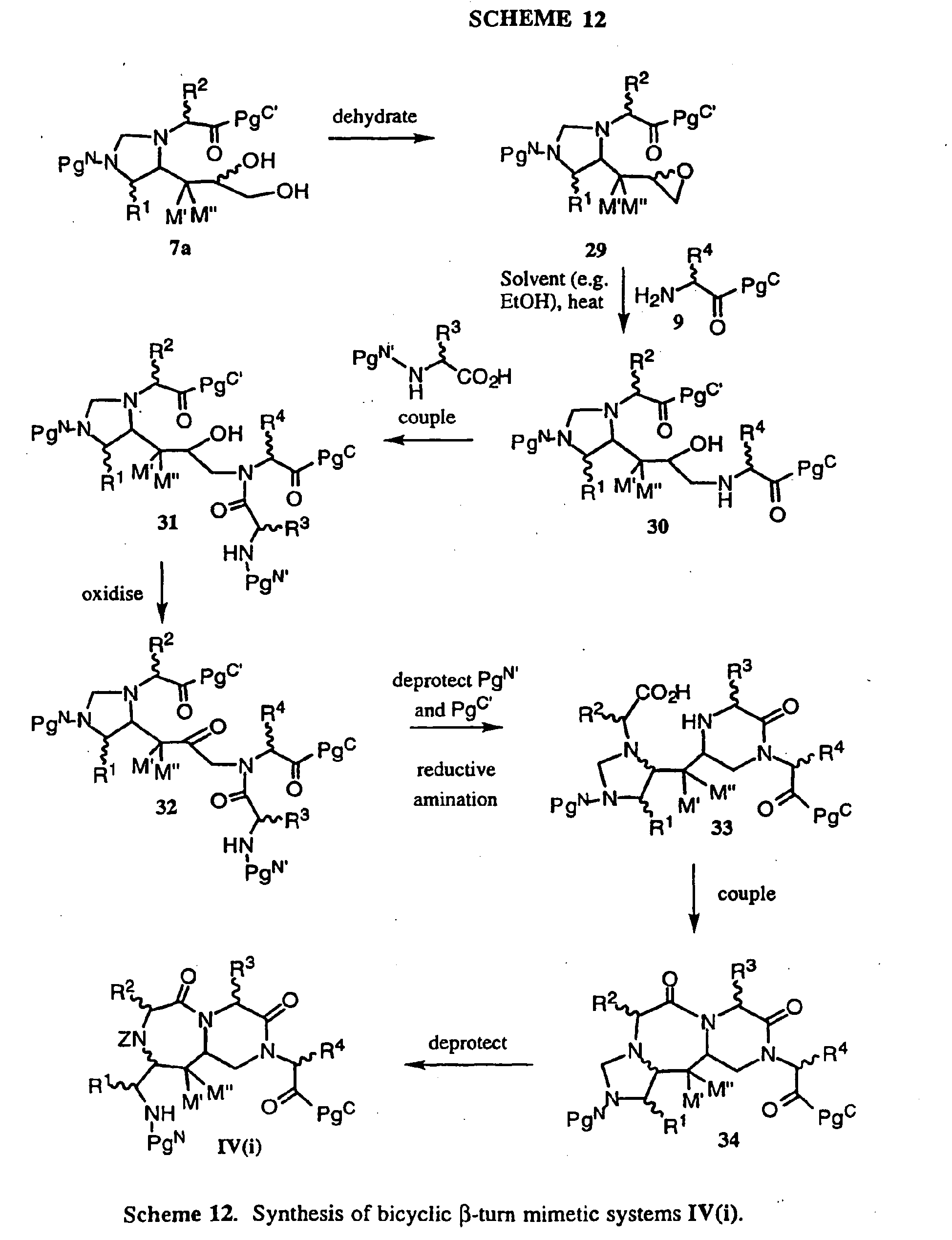

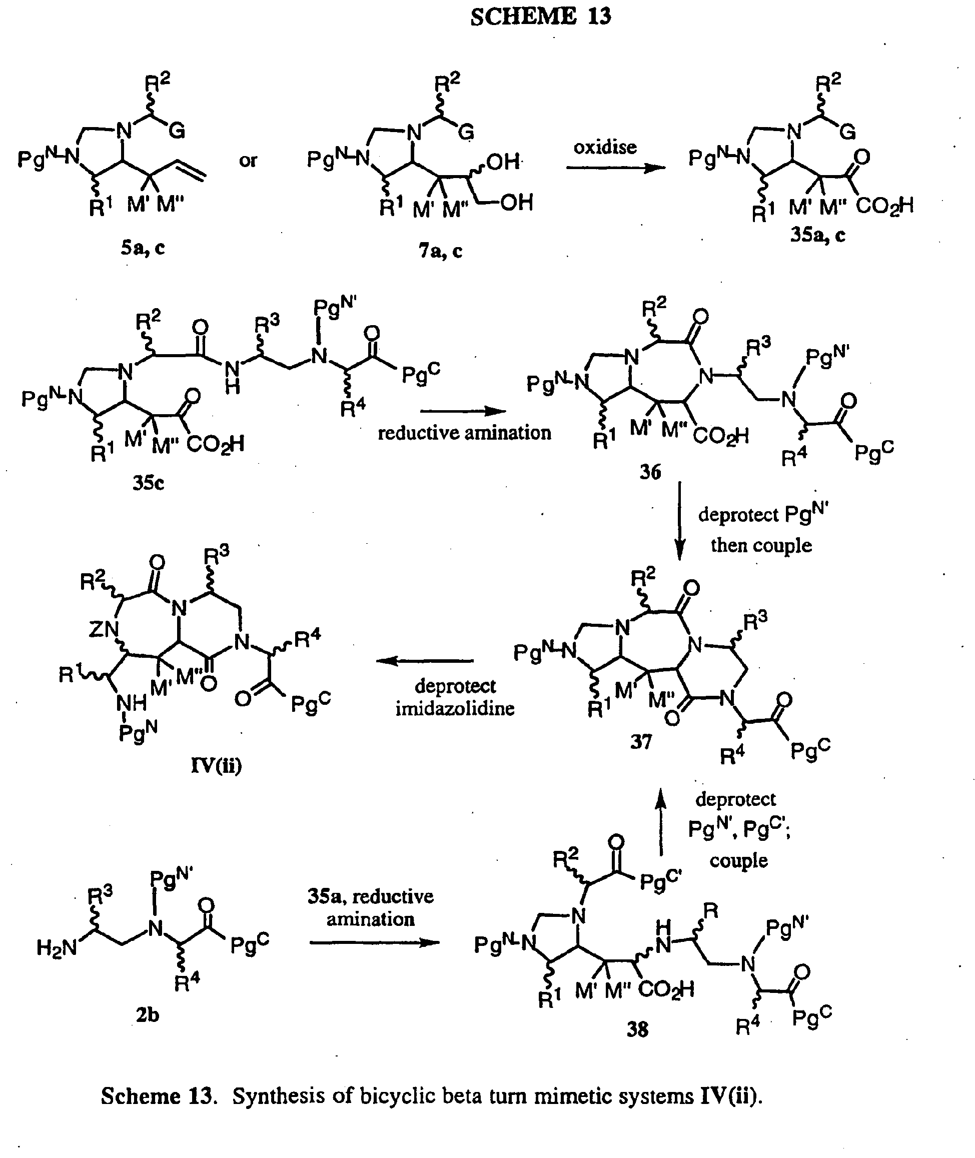

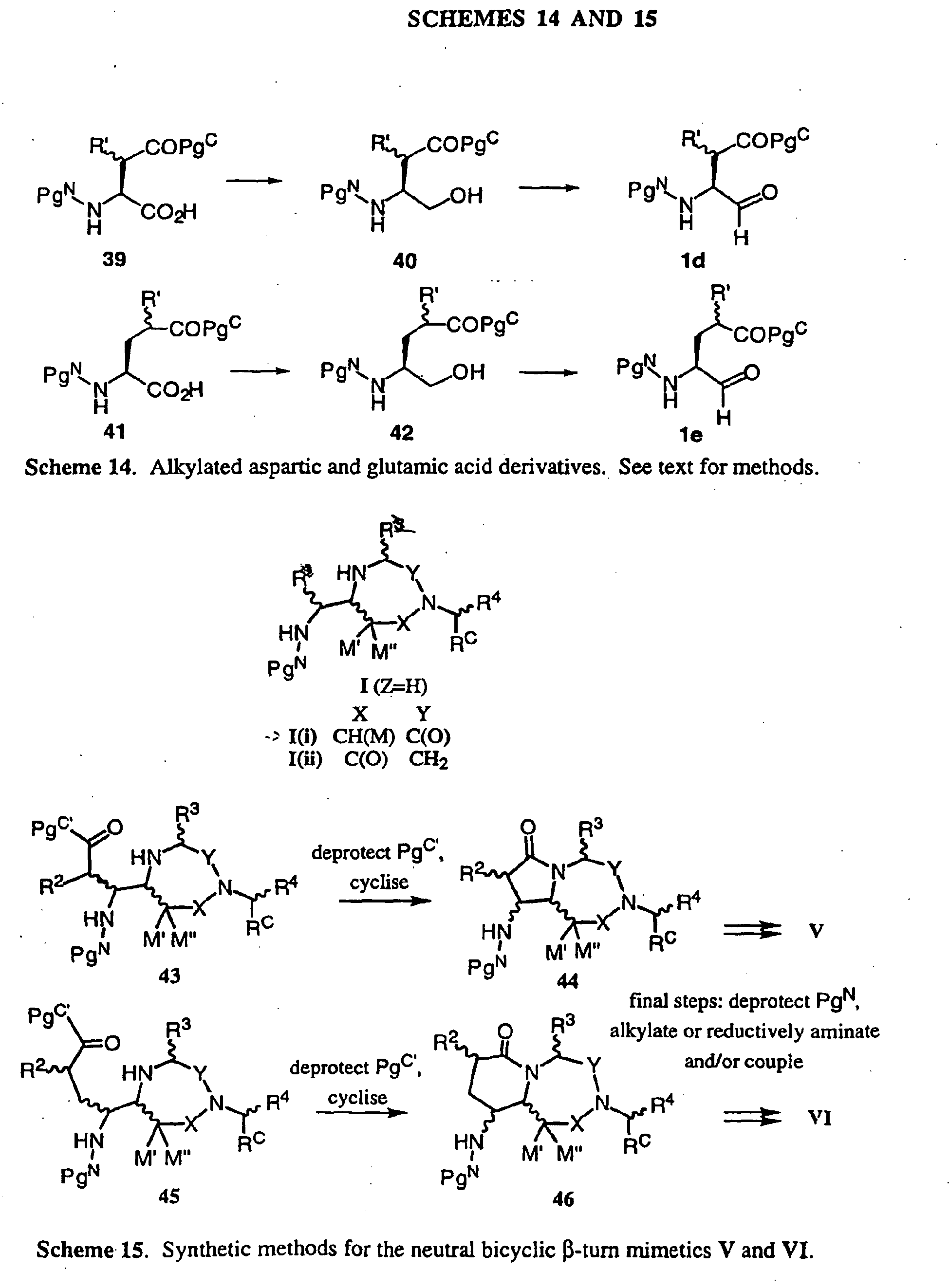

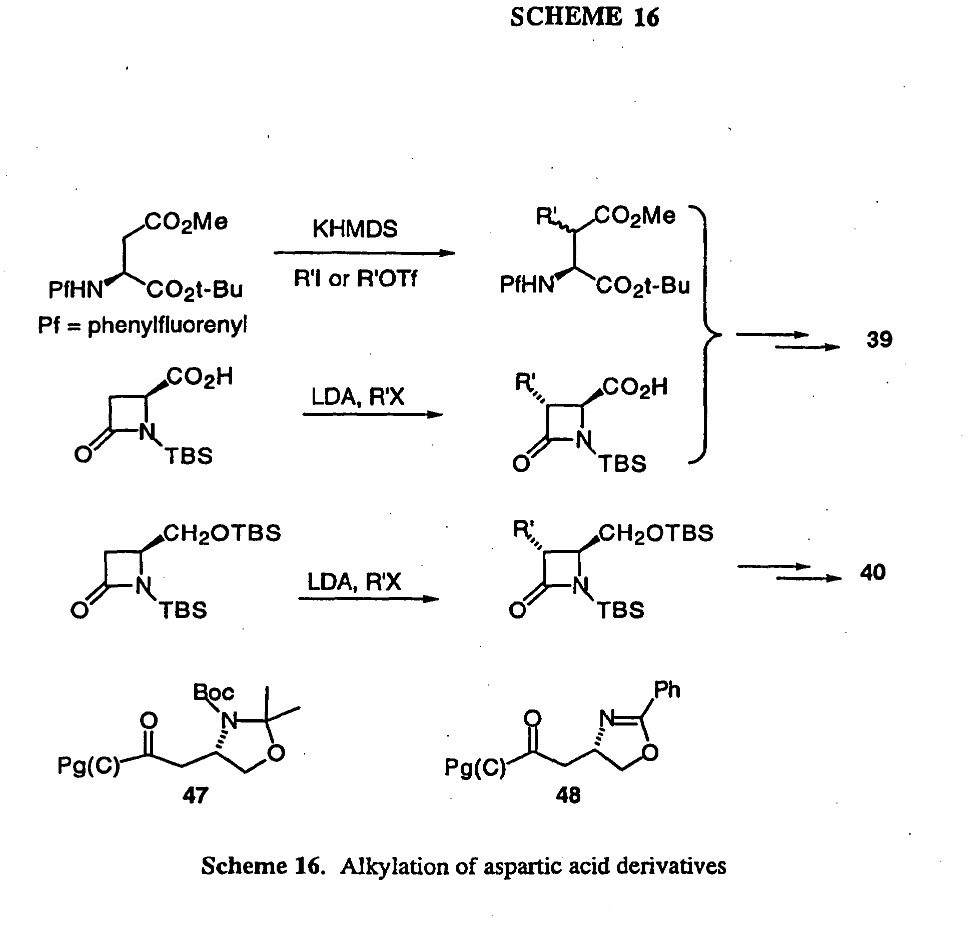

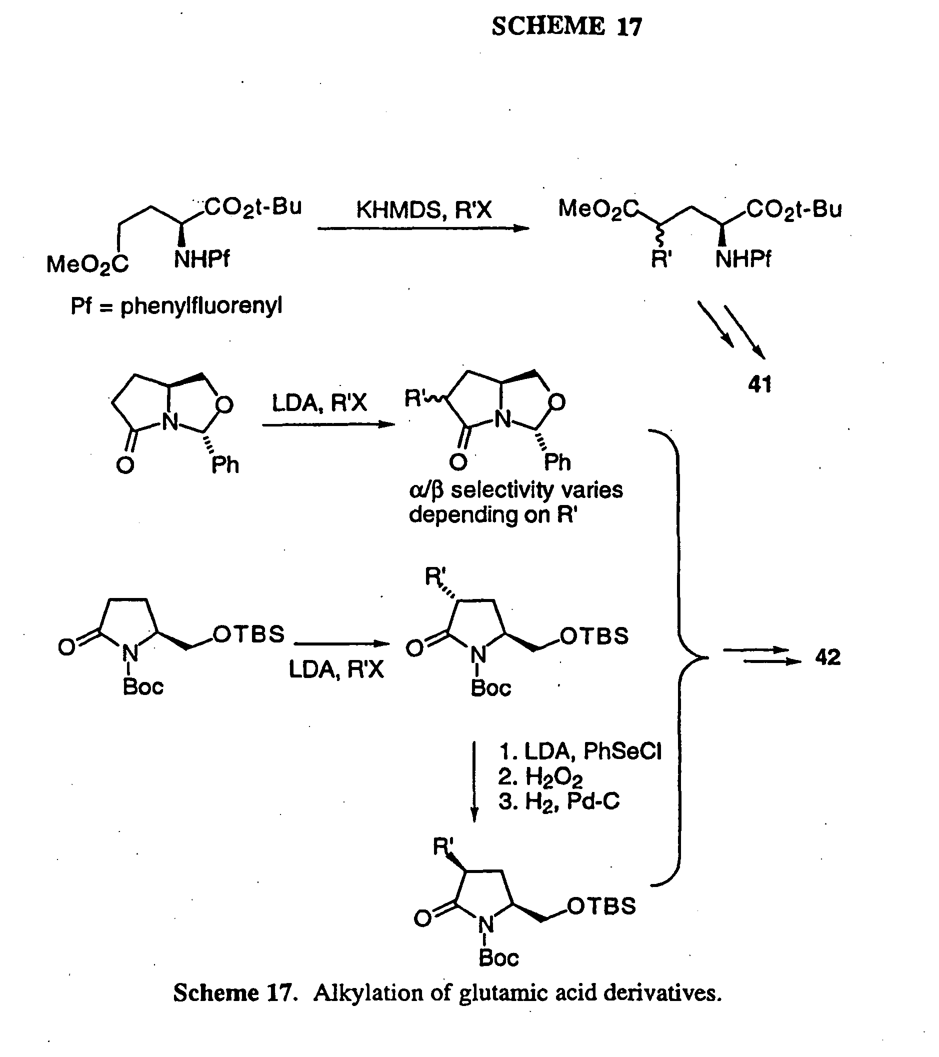

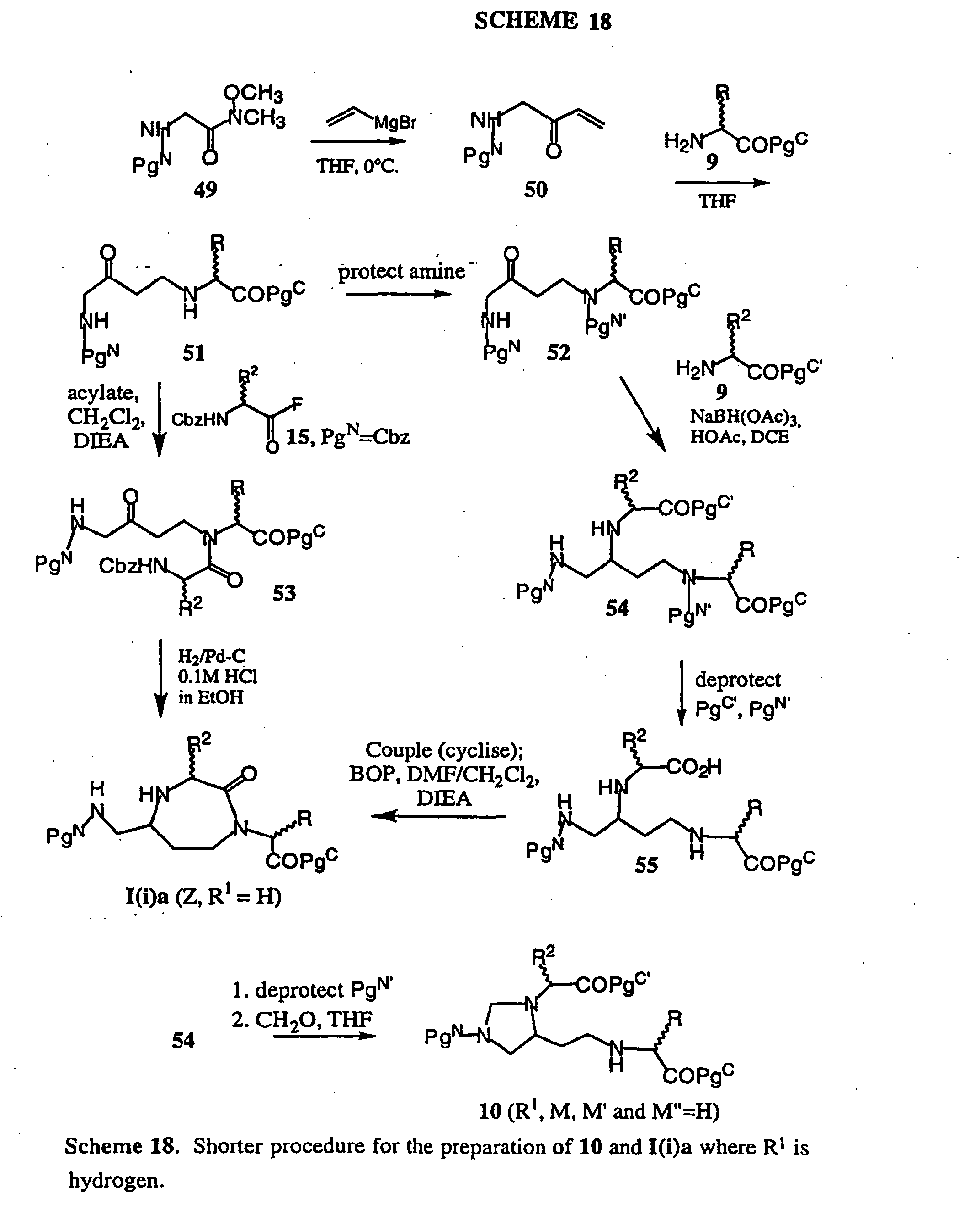

## Claims

1. A general mimetic of the structure: 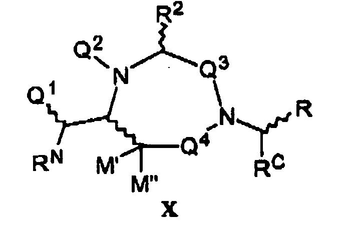 wherein:-
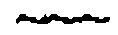 indicates a bond at a chiral centre of the structure which centre may be in the R or S configuration or a mixture thereof;
R and R² is an amino acid side chain group which may be the same or different;
M' and M" may be the same or different and are selected from the group consisting of hydrogen, C₁-C₄ alkyl, chloro and C₁-C₄ alkoxy;
R^{N} is -N(Z')Pg^{N} where Z' is selected from the group consisting of hydrogen, methyl and part of a cyclic amino acid side chain joined to Q¹, and wherein Pg^{N} is selected from the group consisting of a protecting group for an amine, a cleavable linker to a solid support, the solid support, hydrogen, R, C(O)R or part of the remaining N-terminal portion of the mimetic;
R^{C} is selected from the group consisting of a carboxy terminal pari of the mimetic, hydrogen, R, and CH₂R;
Q¹ = R¹ which has the same definition as R and R² above and Q² = Z where Z is selected from the group consisting of hydrogen, methyl, ethyl, formyl and acetyl, -CH₂R. and C(O)R or alternatively Z is part of a cyclic amino acid side chain group joined to R²; or Q¹ and Q² taken together represent a cyclic group;
Q³ is selected from the group consisting of Y, -C(O)NHCH(R)Y-, -C(O)ENHCH(R)Y-, -C(O)N(Q⁵)CH(R)Y- wherein Y is selected from the group consisting of C(O) and CH₂ and Q⁵ is a covalent bond from the Q⁴ group to the nitrogen atom in Q³ to form a bicyclic ring system or alternatively, is selected from the group consisting of hydrogen, C₁-C₄ alkyl, chloro and C₁-C₄ alkoxy and E is (AA)ₙ where n is 1 or 2 and AA is an amino acid residue; and
Q⁴ is selected from the group consisting of CH(M'), C(O), CH(Q⁵)CH₂ and CH(Q⁵)C(O);
with the provisos that when:-
(i) Q⁴ = CH(M'), then Y is C(O);
(ii) Q⁴ = C(O), then Y is CH₂;
(iii) Q⁴ = CH(Q⁵)CH₂, Y is C(O); and
(iv) Q⁴ = CH(Q⁵)C(O), Y is CH₂.
(v) Q³ = -C(O)N(O⁵)CH(R)Y, Q⁵ is a covalent bond from the Q⁴ group to the nitrogen atom in Q³ which is a cyclization forming a bicyclic ring system.

2. A peptide mimetic as claimed in claim 1 wherein when Q¹ and Q² form a cyclic group Q¹Q² which is selected from the group consisting of -CH(R)C(O)-, -CH₂CH(R)C(O)-, -CH₂CH₂CH(R)C(O)-, -CH(R)CH₂-, -CH₂CH(R)CH₂-, -CH₂CH₂CH(R)CH₂-, -CH₂CH(R)-, -CH₂CH₂CH(R)-, -CH(R)CH₂CH₂-, -CH₂CH(R)CH₂CH₂-, -CH(R)CH₂C(O)- and -CH₂CH(R)CH₂C(O)-.

3. A peptide mimetic as claimed in claim 1 wherein n is 1.

4. A peptide mimetic as claimed in claim 1 wherein Q¹ is R, Q² is Z, Q³ is Y.

5. A peptide mimetic as claimed in claim 1 wherein Q¹ is R, Q² is Z, Q³ is C(O)NHCH(R)Y and Q⁵ is M'.

6. A peptide mimetic as claimed in claim 1 wherein Q¹ is R, Q² is Z, Q³ is C(O)NHCH(R)C(O)-NHCH(R)Y and Q⁵ is M'.

7. A peptide mimetic as claimed in claim 1 wherein Q¹ is R, Q² is Z, Q³ is C(O)N(Q⁵)CH(R)Y and Q⁵ is a covalent bond to Q³.

8. A peptide mimetic as claimed in claim 1 wherein Q¹ is CH(R)C(O)Q², Q² is a covalent bond to Q¹, Q³ is Y and Q⁵ is M'.

9. A peptide mimetic as claimed in claim 1 wherein Q¹ is CH₂CH(R)C(O)Q², Q² is a covalent bond to Q¹, Q³ is Y and Q⁵ is M'.

10. A peptide mimetic as claimed in claim 1 wherein R^{C} is C(O)Pg^{C} where Pg^{C} is a protecting group for carboxylic acid.

11. A peptide mimetic as claimed in claim 10 wherein Pg^{C} is selected from the group consisting of alkoxy, benzyloxy, allyloxy, fluorenylmethyloxy amines forming easily removable amides, a cleavable linker to a solid support, the solid support, hydroxy, NHR, R, OR or the remaining C-terminal portion of the mimetic.

12. A peptide mimetic as claimed in claim 11 wherein Pg^{C} is methoxy or ethoxy.

13. A peptide mimetic as claimed in claim 1 wherein Pg^{N} is a protecting group for an amine.

14. A peptide mimetic as claimed in claim 1 wherein Pg^{N} is selected from a group consisting of Boc, Cbz, Alloc and trityl.

15. A peptide mimetic as claimed in claim 1 wherein M' or M" is methoxy.

16. A peptide mimetic as claimed in claim 1 wherein M' or M" is methyl.

17. A peptide mimetic according to claim 1 of the formula I(i)a: 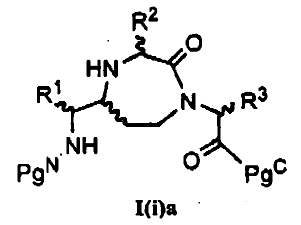 wherein R¹, R² and Pg^{N} are as defined in claim 1, Pg^{C} is a protecting group for carboxylic acid and R³ is an amino acid side chain group.

18. A peptide mimetic according to claim 17 where R¹ and R² ≠ H.

19. A peptide mimetics according claim 1 of the formula I(ii)a: 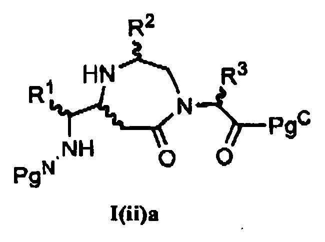 wherein R¹, R² and Pg^{N} are as defined in claim 1, Pg^{C} is a protecting group for carboxylic acid and R³ is an amino acid side chain group.

20. A peptide mimetic according to claim 19 where R¹ and R² ≠ H.

21. A peptide mimetic according claim 1 of the formula II(i)a: 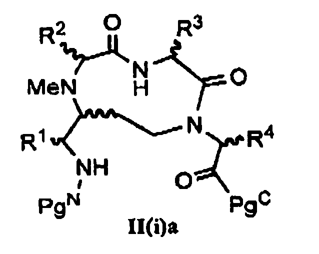 wherein R¹, R² and Pg^{N} are as defined in claim 1, Pg^{C} is a protecting group for carboxylic acid and R³ and R⁴ are independently amino acid side chain groups.

22. A peptide mimetic according to claim 21 where R¹ and R² ≠ H.

23. A peptide mimetic according to claim 1 of the formula II(iii)a: 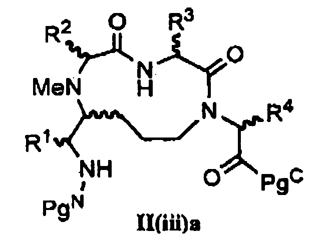 wherein R¹, R² and Pg^{N} are as defined in claim 1, Pg^{C} is a protecting group for carboxylic acid and R³ and R⁴ are independently amino acid side chain groups.

24. A peptide mimetic according to claim 23 where R¹ and R² ≠ H.

25. A peptide mimetic according to claim 1 of the formula III(i)a: 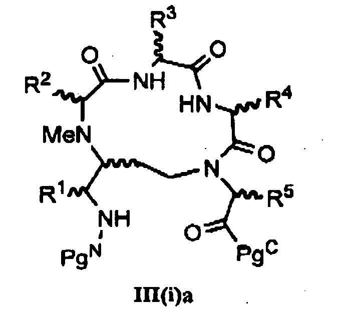 wherein R¹, R² and Pg^{N} are as defined in claim 1, Pg^{C} is a protecting group for carboxylic acid and R³, R⁴ and R⁵ are each independently amino acid side chain groups.

26. A peptide mimetic according to claim 1 of the formula III(ii)a: 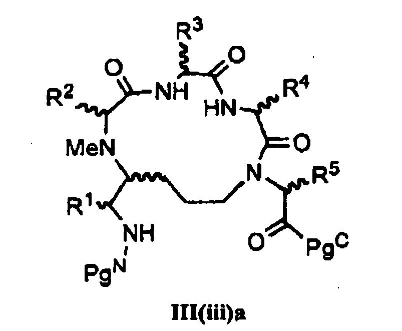 wherein R¹, R² and Pg^{N} are as defined in claim 1, Pg^{c} is a protecting group for carboxylic acid and R³, R⁴ and R⁵ are each independently amino acid side chain groups.

27. A peptide mimetic according to claim 1 of the formula IV(i)a: 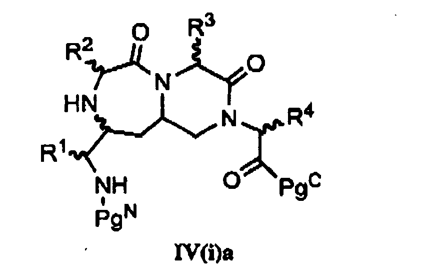 wherein R¹, R² and Pg^{N} are as defined in claim 1, Pg^{C} is a protecting group for carboxylic acid and R³ and R⁴ are each independently amino acid side chain groups.

28. A peptide mimetic according to claim 1 of the formula IV(ii)a: 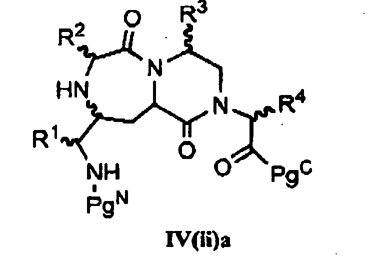 wherein R¹, R² and Pg^{N} are as defined in claim 1, Pg^{c} is a protecting group for carboxylic acid and R³ and R⁴ are each independently amino acid side chain groups.

29. A peptide mimetic according to claim 1 of the formula V(i)a or V(ii)a; 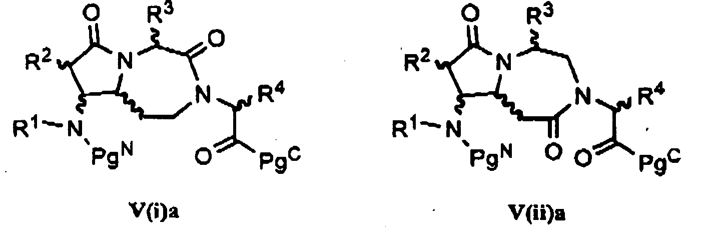 wherein R¹, R², and Pg^{N} are as defined in claim 1, Pg^{c} is a protecting group for carboxylic acid and R³ and R⁴ are each independently amino acid side chain groups.

30. A peptide mimetic according to claim 1 of the formula VI(i)a and VI(ii)a herein: 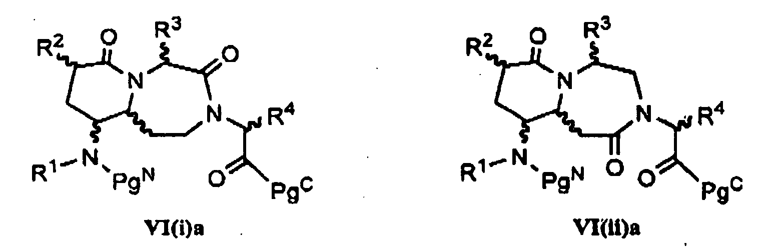 wherein R¹, R², and Pg^{N} are as defined in claim 1, Pg^{c} is a protecting group for carboxylic acid and R³ and R⁴ are each independently amino acid side chain groups.

31. A compound of the formula 4a-d: 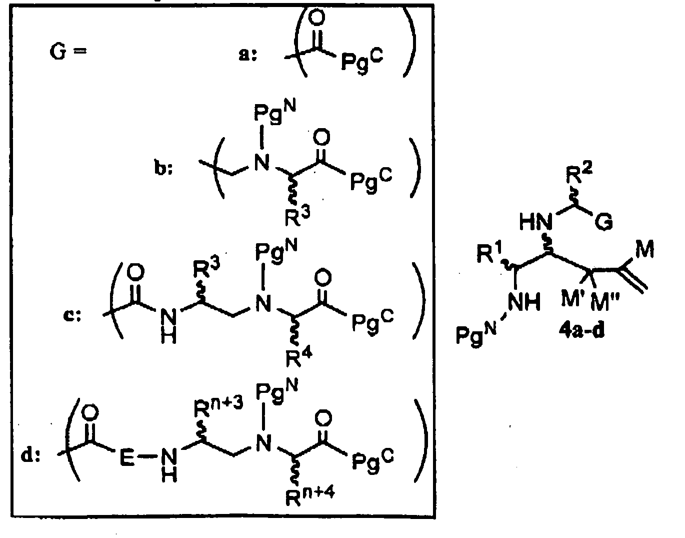 wherein R¹, R², E, M, M', M" and Pg^{N} are as defined in claim 1, Pg^{C} is a protecting group for carboxylic acid and R³, R⁴, Rⁿ⁺³ and Rⁿ⁺⁴ are each independently amino acid side chain groups.

32. A compound of the formula 5a-d: 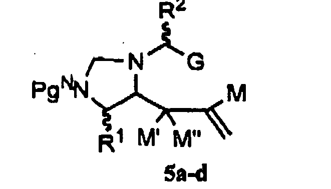 wherein R¹, R², M, M', M" and Pg^{N} are as defined in claim 1, and G is as defined in claim 31.

33. A process for the preparation of compounds 4a-d according to claim 31 comprising the reaction of imines 3a-d herein with an allyl boron reagent to provide compounds 4a-d: 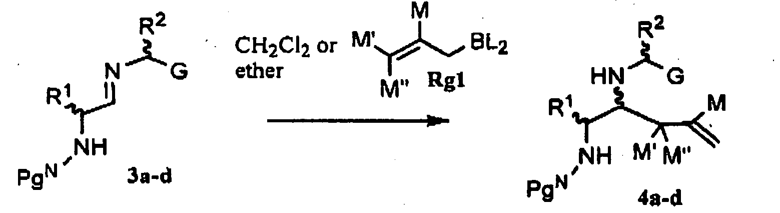 wherein R¹, R², M, M', M" and Pg^{N} are as defined in claim 1 and G is as defined in claim 31.

34. A process as claimed in claim 33 wherein imines 3a-d are prepared by condensation of amino acid aldehydes 1 herein and amines 2a-d: 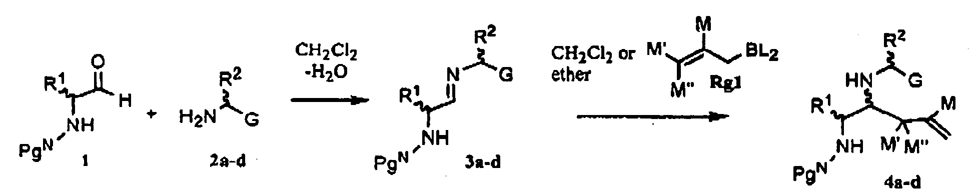 wherein R¹, R², M, M', M" and Pg^{N} are as defined in claim 1 and G is as defined in claim 31.

35. A process as claimed in claim 33 wherein addition of formaldehyde solution to compounds 4a-d provides imidazolidines 5a-d herein. 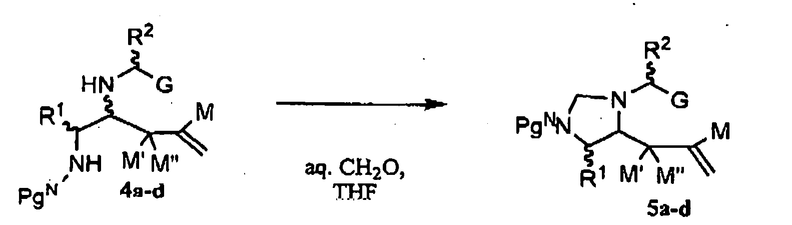 wherein R¹, R², M, M', M" and Pg^{N} are as defined in claim 1 and G is as defined in claim 31.

36. A library of peptide mimetics comprising at least one mimetic from any one of claims 1 to 30.

37. A process of making mimetics (I)(ia) herein wherein initially compounds 49 herein are converted to compounds 50 herein which thereafter after reaction with compounds 9 herein produces compounds 51 herein which are subsequently converted to compounds 52 herein which are then reductively aminated with compounds 9 to provide compounds 54 herein: 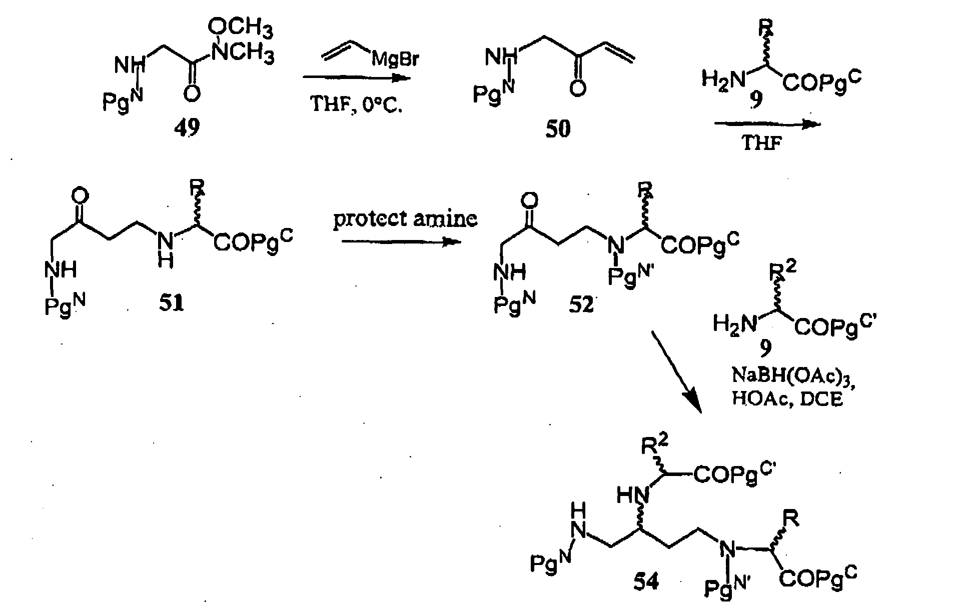 and further wherein compounds 54 are converted to compounds 55 after removal of groups Pg^{N'} and Pg^{C'} which are then converted to mimetics I(i)a where Z and R¹ are H: 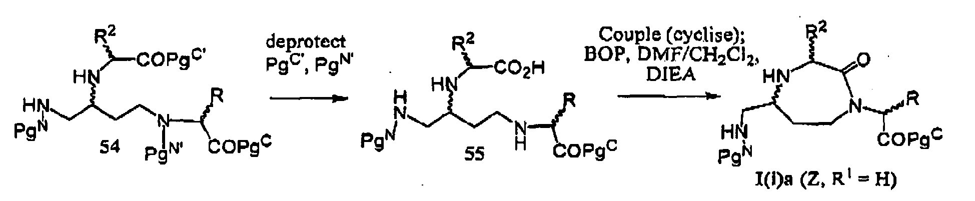 wherein R, R², and Pg^{N} are as defined in claim 1; Pg^{c} and Pg^{C'} are each independently a protecting group for carboxylic acid, a cleavable linker to a solid phase support, a support, OH, -OR, -NHR or the remaining C-terminal portion of the mimetic; and Pg^{N'} is selected from the group consisting of a protecting group for an amine, a cleavable linker to a solid support, the solid support, hydrogen, R, C(O)R or part of the remaining N-terminal portion of the mimetic.

38. A process for making mimetics I(i)a herein stereospecifically wherein compounds 49 herein are reacted with vinyl magnesium bromide to compounds 50 herein which are then reacted with compounds 9 herein to form compounds 51 herein which are then reacted with compounds 15 herein to form compounds 53 herein which are then converted to mimetics I(i)a by deprotection of PgN' followed by reductive amination: 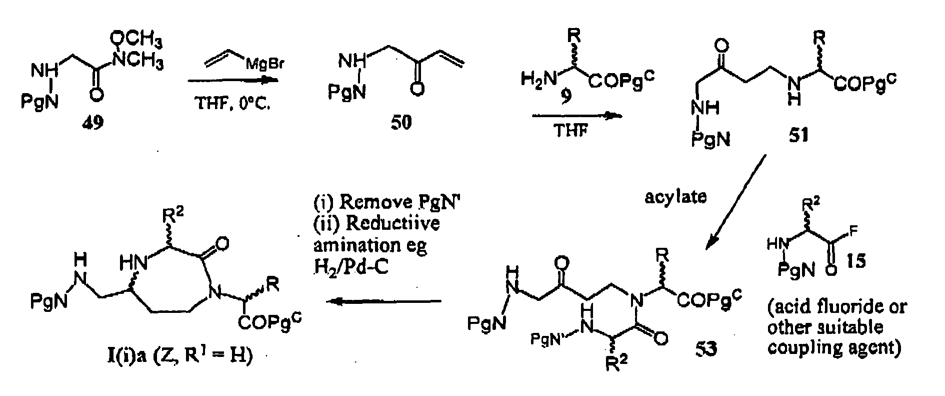 wherein R, R², and Pg^{N} are as defined in claim 1; Pg^{C} and Pg^{C'} are each independently a protecting group for carboxylic acid, a cleavable linker to a solid phase support, a support, OH, -OR, -NHR or the remaining C-terminal portion of the mimetic; and Pg^{N'} is selected from the group consisting of a protecting group for an amine, a cleavable linker to a solid support, the solid support, hydrogen, R, C(O)R or part of the remaining N-terminal portion of the mimetic.

## Patentansprüche

1. Allgemeines Mimetikum der Struktur: 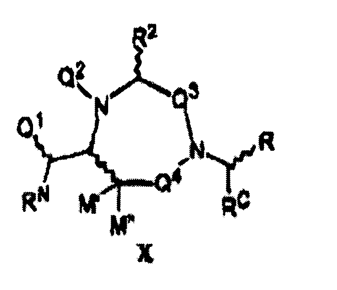 wobei:
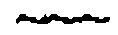 eine Bindung an einem Chiralitätszentrum der Struktur anzeigt, wobei das Zentrum in der R- oder S-Konfiguration oder einer Mischung davon sein kann;
R und R² eine Aminosäureseitenkettengruppe ist, die dieselbe oder unterschiedlich sein kann;
M' und M" dieselben oder unterschiedlich sein können und ausgewählt sind aus der Gruppe bestehend aus Wasserstoff, C₁-C₄-Alkyl, Chloro und C₁-C₄-Alkoxy;
R^{N} -N(Z')Pg^{N} ist, wobei Z' ausgewählt ist aus der Gruppe bestehend aus Wasserstoff, Methyl und einem Teil einer zyklischen Aminosäureseitenkette, die mit Q¹ verbunden ist, und wobei Pg^{N} ausgewählt ist aus der Gruppe bestehend aus einer Schutzgruppe für ein Amin, einem spaltbaren Linker zu einem festen Träger, dem festen Träger, Wasserstoff, R, C(O)R oder einem Teil des verbleibenden N-terminalen Abschnitts des Mimetikums;
R^{C} ausgewählt ist aus der Gruppe bestehend aus einem Carboxy-terminalen Teil des Mimetikums, Wasserstoff, R und CH₂R;
Q¹ = R¹, mit derselben Definition wie oben R und R², und Q² = Z, wobei Z ausgewählt ist aus der Gruppe bestehend aus Wasserstoff, Methyl, Ethyl, Formyl und Acetyl, -CH₂R und C(O)R, oder Z alternativ Teil einer zyklischen Aminosäureseitenkettengruppe ist, die mit R² verbunden ist; oder Q¹ und Q² gemeinsam eine zyklische Gruppe darstellen; Q³ ausgewählt ist aus der Gruppe bestehend aus Y, -C(O)NHCH(R)Y-, - C(O)ENHCH(R)Y-, -C(O)N(Q⁵)CH(R)Y-, wobei Y ausgewählt ist aus der Gruppe bestehend aus C(O) und CH₂ und Q⁵ eine kovalente Bindung von der Q⁴-Gruppe zu dem Stickstoffatom in Q³ ist, um ein bizyklisches Ringsystem zu bilden, oder alternativ ausgewählt ist aus der Gruppe bestehend aus Wasserstoff, C₁-C₄-Alkyl, Chloro und C₁-C₄-Alkoxy, und E (AA)ₙ ist, wobei n 1 oder 2 ist und AA ein Aminosäurerest ist; und
Q⁴ ausgewählt ist aus der Gruppe bestehend aus CH(M'), C(O), CH(Q⁵)CH₂ und CH(Q⁵)C(O);
unter der Voraussetzung, dass, wenn:
(i) Q⁴ = CH(M'), Y C(O) ist;
(ii) Q⁴ = C(O), Y CH₂ ist;
(iii) Q⁴ = CH(Q⁵)CH₂, Y C(O) ist; und
(IV) Q⁴ = CH(Q⁵)C(O), Y CH₂ ist,
(v) Q³ = -C(O)N(Q⁵)CH(R)Y, Q⁵ eine kovalente Bindung von der Q⁴-Gruppe zu dem Stickstoffatom in Q³ ist, welches eine Zyklisierung darstellt, die ein bizyklisches Ringsystem bildet.

2. Peptidmimetikum nach Anspruch 1, wobei, wenn Q¹ und Q² eine zyklische Gruppe Q¹Q² bilden, die ausgewählt ist aus der Gruppe bestehend aus -CH(R)C(O)-, -CH₂CH(R)C(O)-, -CH₂CH₂CH(R)C(O)-, -CH(R)CH₂-, -CH₂CH(R)CH₂-, -CH₂CH₂CH(R)CH₂-, -CH₂CH(R)-, -CH₂CH₂CH(R)-, -CH(R)CH₂CH₂-, -CH₂CH(R)CH₂CH₂-, -CH(R)CH₂C(O)- und -CH₂CH(R)CH₂C(O)-.

3. Peptidmimetikum nach Anspruch 1, wobei n 1 ist.

4. Peptidmimetikum nach Anspruch 1, wobei Q¹ R ist, Q² Z ist, Q³ Y ist.

5. Peptidmimetikum nach Anspruch 1, wobei Q¹ R ist, Q² Z ist, Q³ C(O)NHCH(R)Y ist und Q⁵ M' ist.

6. Peptidmimetikum nach Anspruch 1, wobei Q¹ R ist, Q² Z ist, Q³ C(O)NHCH(R)C(O)-NHCH(R)Y ist und Q⁵ M' ist.

7. Peptidmimetikum nach Anspruch 1, wobei Q¹ R ist, Q² Z ist, Q³ C(O)N(Q⁵)CH(R)Y ist und Q⁵ eine kovalente Bindung an Q³ darstellt.

8. Peptidmimetikum nach Anspruch 1, wobei Q¹ CH(R)C(O) Q² ist, Q² eine kovalente Bindung an Q¹ ist, Q³ Y ist und Q⁵ M' ist.

9. Peptidmimetikum nach Anspruch 1, wobei Q¹ CH₂CH(R)C(O)Q² ist, Q² eine kovalente Bindung an Q¹ ist, Q³ Y ist und Q⁵ M' ist.

10. Peptidmimetikum nach Anspruch 1, wobei R^{C} C(O)Pg^{C} ist, wobei Pg^{C} eine Schutzgruppe für Carbonsäure ist.

11. Peptidmimetikum nach Anspruch 10, wobei Pg^{C} ausgewählt ist aus der Gruppe bestehend aus Alkoxy, Benzyloxy, Allyloxy, Fluorenylmethyloxyaminen, die leicht entfernbare Amide bilden, einem spaltbaren Linker zu einem festen Träger, dem festen Träger, Hydroxy, NHR, R, OR oder dem verbleibenden C-terminalen Abschnitt des Mimetikums.

12. Peptidmimetikum nach Anspruch 11, wobei Pg^{C} Methoxy oder Ethoxy ist.

13. Peptidmimetikum nach Anspruch 1, wobei Pg^{N} eine Schutzgruppe für ein Amin ist.

14. Peptidmimetikum nach Anspruch 1, wobei Pg^{N} ausgewählt ist aus einer Gruppe bestehend aus Boc, Cbz, Alloc und Trityl.

15. Peptidmimetikum nach Anspruch 1, wobei M' oder M" Methoxy ist.

16. Peptidmimetikum nach Anspruch 1, wobei M' oder M" Methyl ist.

17. Peptidmimetikum nach Anspruch 1 der Formel 1(1)a: 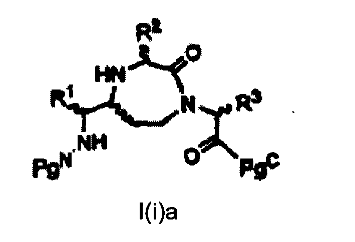 wobei R¹, R² und Pg^{N} wie in Anspruch 1 definiert sind, Pg^{C} eine Schutzgruppe für Carbonsäure ist und R³ eine Aminosäureseitenkettengruppe ist.

18. Peptidmimetikum nach Anspruch 17, wobei R¹ und R² ≠ H sind.

19. Peptidmimetikum nach Anspruch 1 der Formel I(ii)a: 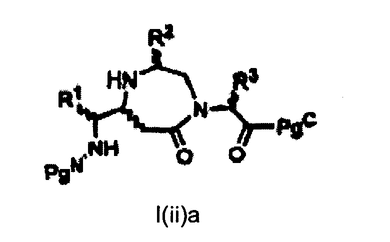 wobei R¹, R² und Pg^{N} wie in Anspruch 1 definiert sind, Pg^{C} eine Schutzgruppe für Carbonsäure ist und R³ eine Aminosäureseitenkettengruppe ist.

20. Peptidmimetikum nach Anspruch 19, wobei R¹ und R ² ≠ H sind.

21. Peptidmimetikum nach Anspruch 1 der Formel II(i)a: 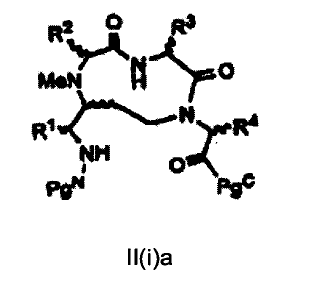 wobei R¹, R² und Pg^{N} wie in Anspruch 1 definiert sind, Pg^{C} eine Schutzgruppe für Carbonsäure ist und R³ und R⁴ unabhängig Aminosäureseitenkettengruppen sind.

22. Peptidmimetikum nach Anspruch 21, wobei R¹ und R² ≠ H sind.

23. Peptidmimetikum nach Anspruch 1 der Formel II(iii)a: 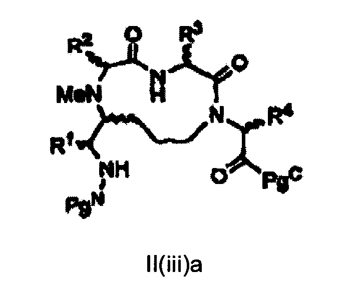 wobei R¹, R² und Pg^{N} wie in Anspruch 1 definiert sind, Pg^{C} eine Schutzgruppe für Carbonsäure ist und R³ und R⁴ unabhängig Aminosäureseitenkettengruppen sind.

24. Peptidmimetikum nach Anspruch 23, wobei R¹ und R² ≠ H sind.

25. Peptidmimetikum nach Anspruch 1 der Formel III(i)a: 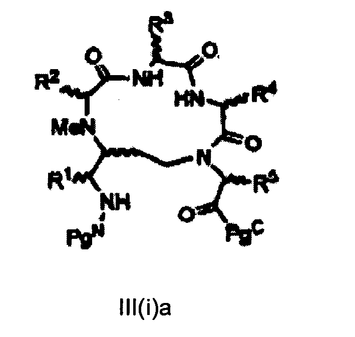 wobei R¹, R² und Pg^{N} wie in Anspruch 1 definiert sind, Pg^{C} eine Schutzgruppe für Carbonsäure ist und R³, R⁴ und R⁵ jeweils unabhängig Aminosäureseitenkettengruppen sind.

26. Peptidmimetikum nach Anspruch 1 der Formel III(ii)a: 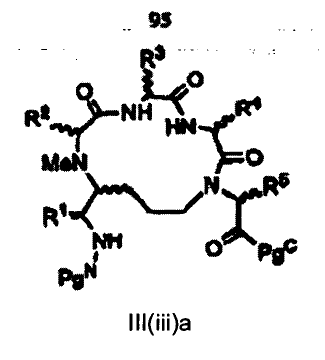 wobei R¹, R² und Pg^{N} wie in Anspruch 1 definiert sind, Pg^{C} eine Schutzgruppe für Carbonsäure ist und R³, R⁴ und R⁵ jeweils unabhängig Aminosäureseitenkettengruppen sind.

27. Peptidmimetikum nach Anspruch 1 der Formel IV(i)a: 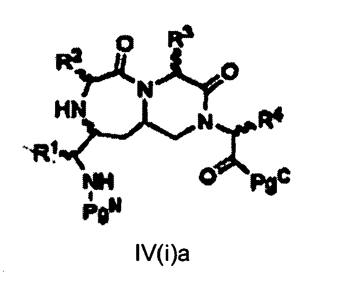 wobei R¹, R² und Pg^{N} wie in Anspruch 1 definiert sind, Pg^{C} eine Schutzgruppe für Carbonsäure ist und R³ und R⁴ jeweils unabhängig Aminosäureseitenkettengruppen sind.

28. Peptidmimetikum nach Anspruch 1 der Formel IV(ii)a: 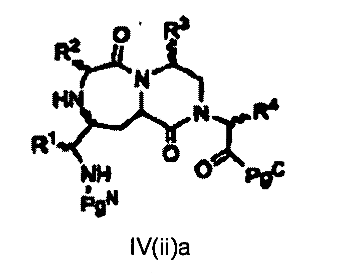 wobei R¹, R² und Pg^{N} wie in Anspruch 1 definiert sind, Pg^{C} eine Schutzgruppe für Carbonsäure ist und R³ und R⁴ jeweils unabhängig Aminosäureseitenkettengruppen sind.

29. Peptidmimetikum nach Anspruch 1 der Formel V(i)a oder V(ii)a: 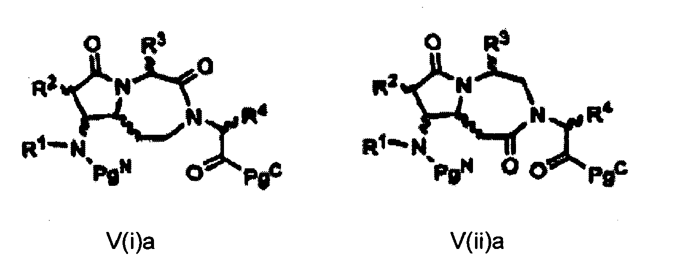 wobei R¹, R² und Pg^{N} wie in Anspruch 1 definiert sind, Pg^{C} eine Schutzgruppe für Carbonsäure ist und R³ und R⁴ jeweils unabhängig Aminosäureseitenkettengruppen sind.

30. Peptidmimetikum nach Anspruch 1 der Formel VI(i)a und VI(ii)a: 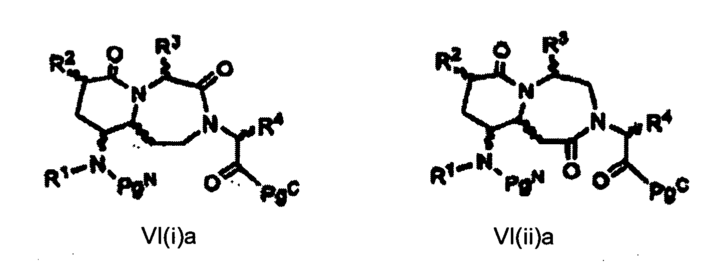 wobei R¹, R² und Pg^{N} wie in Anspruch 1 definiert sind, Pg^{C} eine Schutzgruppe für Carbonsäure ist und R³ und R⁴ jeweils unabhängig Aminosäureseitenkettengruppen sind.

31. Verbindung der Formel 4a-d: 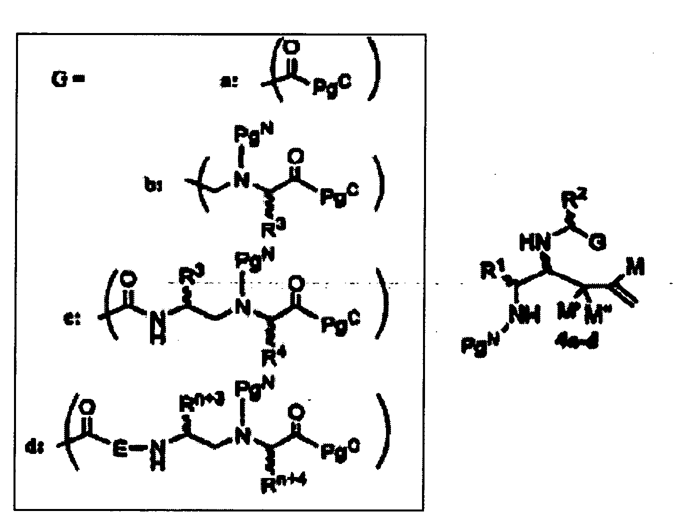 wobei R¹, R², E, M, M', M" und Pg^{N} wie in Anspruch 1 definiert sind, Pg^{C} eine Schutzgruppe für Carbonsäure ist und R³, R⁴, Rⁿ⁺³ und Rⁿ⁺⁴ jeweils unabhängig Aminosäureseitenkettengruppen sind.

32. Verbindung der Formel 5a-d: 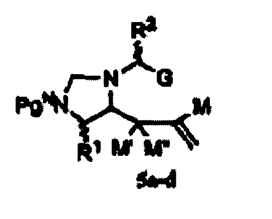 wobei R¹, R², M, M', M" und Pg^{N} wie in Anspruch 1 definiert sind und G wie in Anspruch 31 definiert ist.

33. Verfahren zur Herstellung von Verbindungen 4a bis d nach Anspruch 31, umfassend die Reaktion von Iminen 3a bis d mit einem Allylborreagenz, um die Verbindungen 4a bis d bereitzustellen: 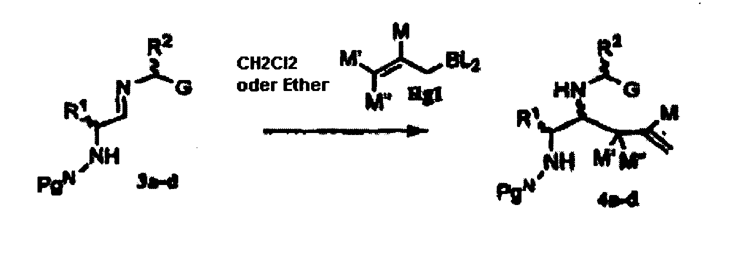 wobei R¹, R², M, M', M" und Pg^{N} wie in Anspruch 1 definiert sind und G wie in Anspruch 31 definiert ist.

34. Verfahren nach Anspruch 33, wobei die Imine 3a bis d durch Kondensation von Aminosäurealdehyden 1 und Aminen 2a bis d hergestellt werden: 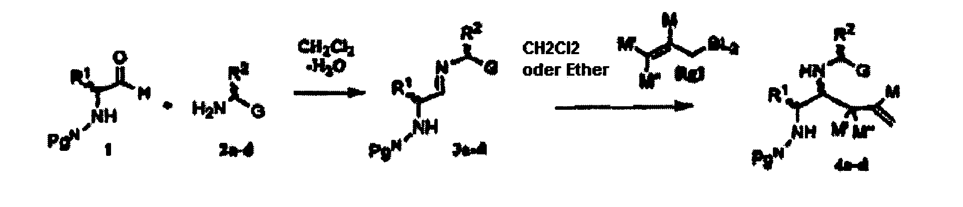 wobei R¹, R², M, M', M" und Pg^{N} wie in Anspruch 1 definiert sind und G wie in Anspruch 31 definiert ist.

35. Verfahren nach Anspruch 33, wobei die Zugabe einer Formaldehydlösung zu den Verbindungen 4a bis d Imidazolidine 5a bis d ergibt. 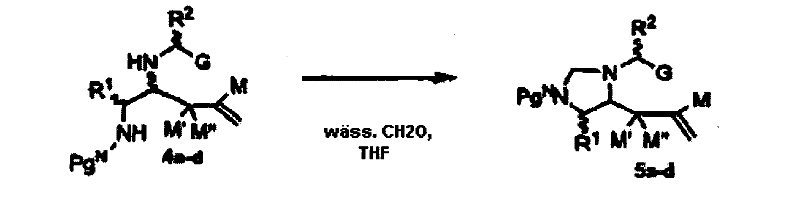 wobei R¹, R², M, M', M" und Pg^{N} wie in Anspruch 1 definiert sind und G wie in Anspruch 31 definiert ist.

36. Bibliothek von Peptidmimetika, umfassend mindestens ein Mimetikum nach einem der Ansprüche 1 bis 30.

37. Verfahren zur Herstellung von Mimetika (I)(ia), wobei Ausgangsverbindungen 49 zu Verbindungen 50 umgesetzt werden, die danach, nach einer Reaktion mit Verbindungen 9, Verbindungen 51 erzeugen, die anschließend zu Verbindungen 52 umgesetzt werden, die dann mit Verbindungen 9 reduktiv aminiert werden, um Verbindungen 54 bereitzustellen: 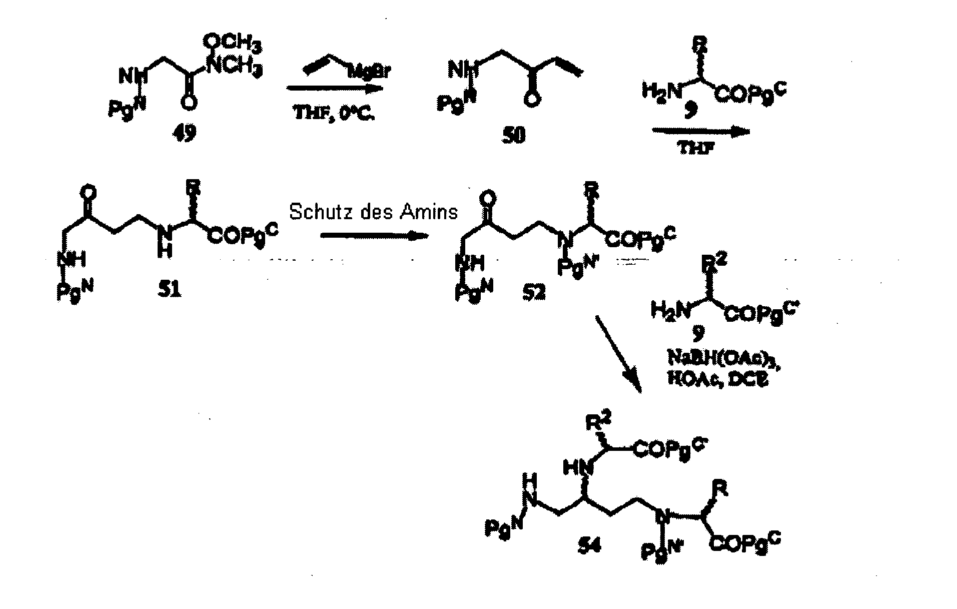 und wobei ferner Verbindungen 54 nach Entfernung von Gruppen Pg^{N'} und Pg^{C'} zu Verbindungen 55 umgesetzt werden, die dann zu Mimetika I(i)a umgesetzt werden, wobei Z und R¹ H sind: 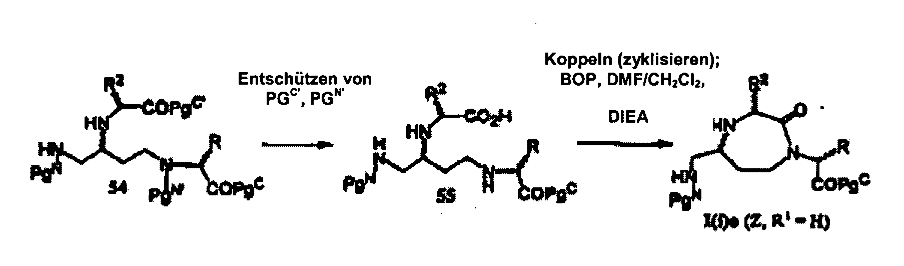 wobei R, R² und Pg^{N} wie in Anspruch 1 definiert sind; Pg^{C} und Pg^{C'} jeweils unabhängig eine Schutzgruppe für Carbonsäure, ein spaltbarer Linker zu einem Festphasenträger, ein Träger, OH, -OR, -NHR oder der verbleibende C-terminale Abschnitt des Mimetikums sind; und Pg^{N'} ausgewählt ist aus der Gruppe bestehend aus einer Schutzgruppe für ein Amin, einem spaltbaren Linker zu einem festen Träger, dem festen Träger, Wasserstoff, R, C(O)R oder einem Teil des verbleibenden N-terminalen Abschnitts des Mimetikums.

38. Verfahren zur stereospezifischen Herstellung von Mimetika I(i)a, wobei Verbindungen 49 mit Vinylmagnesiumbromid zur Reaktion gebracht werden, um Verbindungen 50 zu erhalten, die dann mit Verbindungen 9 zur Reaktion gebracht werden, um Verbindungen 51 zu bilden, die dann mit Verbindungen 15 zur Reaktion gebracht werden, um Verbindungen 53 zu bilden, die dann durch Entschützen von PgN', gefolgt von einer reduktiven Aminierung zu Mimetika I(i)a umgesetzt werden: 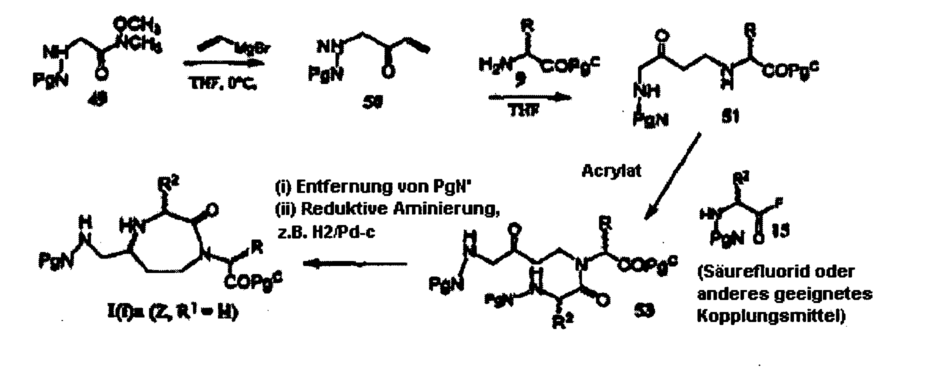 wobei R, R² und Pg^{N} wie in Anspruch 1 definiert sind; Pg^{C} und Pg^{C'} jeweils unabhängig eine Schutzgruppe für Carbonsäure, ein spaltbarer Linker zu einem Festphasenträger, ein Träger, OH, -OR, -NHR oder der verbleibende C-terminale Abschnitt des Mimetikums sind, und Pg^{N'} ausgewählt ist aus der Gruppe bestehend aus einer Schutzgruppe für ein Amin, einem spaltbaren Linker zu einem festen Träger, dem festen Träger, Wasserstoff, R, C(O)R oder einem Teil des verbleibenden N-terminalen Abschnitts des Mimetikums.

## Revendications

1. Mimétique général de structure : 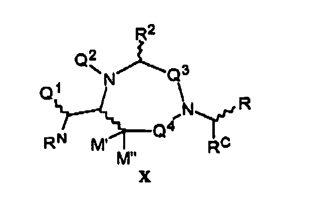 dans laquelle :
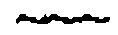 indique une liaison au niveau d'un centre chiral de la structure, lequel centre peut être de configuration R ou S ou un mélange de celles-ci ; R et R² sont un groupe de chaînes latérales d'acide aminé pouvant être identique ou différent ;
M' et M" peuvent être identiques ou différents et sont choisis dans le groupe consistant en hydrogène, un groupe alkyle en C₁-C₄, alcoxy en C1-C4 substitué par un atome de chlore ;
R^{N} est -N(Z')Pg^{N} où Z' est choisi dans le groupe consistant en hydrogène, méthyle et une partie d'une chaîne latérale d'acide aminé cyclique liée à Q¹, et dans lequel Pg^{N} est choisi dans le groupe consistant en un groupe protecteur pour une amine, un lieur clivable à un support solide, le support solide, l'hydrogène, R, C(O)R ou une partie de la partie N-terminale restante du mimétique ;
R^{C} est choisi dans le groupe consistant en une partie carboxyle terminale du mimétique, l'hydrogène, R et CH₂R ;
Q¹ = R¹ qui a la même définition que R et R² ci-dessus et Q² = Z où Z est choisi dans le groupe consistant en hydrogène, méthyle, éthyle, formyle et acétyle, -CH₂R, et
C(O)R ou bien en variante Z est une partie d'un groupe de chaînes latérales d'acide aminé lié à R² ; ou Q¹ et Q² pris conjointement représentent un groupe cyclique ;
Q³ est choisi dans le groupe consistant en Y, C(O)NHCH(R)Y-, -C(O)ENHCH(R)Y-,
-C(O)N(Q⁵)CH(R)Y- dans lequel Y est choisi dans le groupe consistant en C(O) et CH₂ et Q⁵ est une liaison covalente entre le groupe Q⁴ et l'atome d'azote dans Q³ afin de former un système d'anneau bicyclique ou, en variante, est choisi dans le groupe consistant en hydrogène, un groupement alkyle en C₁-C₄, alcoxy en C1-C4 substitué par un atome de chlore et E est (AA)ₙ où n vaut 1 ou 2 et AA est un résidu d'acide aminé ; et
Q⁴ est choisi dans le groupe consistant en CH(M'), C(O), CH(Q⁵) CH₂ et CH(Q⁵)C(O) ;
sous réserve que, lorsque :
(i) Q⁴ = CH(M'), alors Y est C(O) ;
(ii) Q⁴ = C(O), alors Y est CH₂ ;
(iii) Q⁴ = CH(Q⁵)CH₂, Y est C(O) ; et
(iv) Q⁴ = CH(Q⁵)C(O), Y est CH₂ ;
(v) Q³ = -C(O)N(Q⁵)CH(R)Y, Q⁵ est une liaison covalente entre le groupe Q⁴ et l'atome d'azote dans Q³ qui est une cyclisation formant un système d'anneau bicyclique.

2. Mimétique peptidique selon la revendication 1, dans lequel, lorsque Q¹ et Q² forment un groupe cyclique Q¹Q² qui est choisi dans le groupe consistant en -CH(R)C(O)-, -CH₂CH(R)C(O)-, -CH₂CH₂CH(R)C(O)-, -CH(R)CH₂-, - CH₂CH(R)CH₂-, -CH₂CH₂CH(R)CH₂-, -CH₂CH(R)-, -CH₂CH₂CH(R)-, - CH(R)CH₂CH₂-, -CH₂CH(R)CH₂CH₂-, -CH(R)CH₂C(O)- et -CH₂CH(R)CH₂C(O)-.

3. Mimétique peptidique selon la revendication 1 dans lequel n vaut 1.

4. Mimétique peptidique selon la revendication 1 dans lequel Q¹ est R, Q² est Z, Q³ est Y.

5. Mimétique peptidique selon la revendication 1 dans lequel Q¹ est R, Q² est Z, Q³ est C(O)NHCH(R)Y et Q⁵ est M'.

6. Mimétique peptidique selon la revendication 1 dans lequel Q¹ est R, Q² est Z, Q³ est C(O)NHCH(R)C(O)-NHCH(R)Y et Q⁵ est M'.

7. Mimétique peptidique selon la revendication 1 dans lequel Q¹ est R, Q² est Z, Q³ est C(O)N(Q⁵)CH(R)Y et Q⁵ est une liaison covalente à Q³.

8. Mimétique peptidique selon la revendication 1 dans lequel Q¹ est CH(R)C(O)Q², Q² est une liaison covalente à Q¹, Q³ est Y et Q⁵ est M'.

9. Mimétique peptidique selon la revendication 1 dans lequel Q¹ est CH₂CH(R)C(O)Q², Q² est une liaison covalente à Q¹, Q³ est Y et Q⁵ est M'.

10. Mimétique peptidique selon la revendication 1 dans lequel R^{C} est C(O)Pg^{C} où Pg^{C} est un groupe protecteur pour l'acide carboxylique.

11. Mimétique peptidique selon la revendication 10 dans lequel Pg^{C} est choisi dans le groupe consistant en amines alcoxyliques, benzyloxyliques, allyloxyliques, fluorénylméthyloxyliques formant des amides facilement éliminables, un lieur clivable à un support solide, le support solide, un radical hydroxy, NHR, R, OR ou la partie C-terminale restante du mimétique.

12. Mimétique peptidique selon la revendication 11 dans lequel Pg^{C} est le radical méthoxy ou éthoxy.

13. Mimétique peptidique selon la revendication 1 dans lequel Pg^{N} est un groupe protecteur pour une amine.

14. Mimétique peptidique selon la revendication 1 dans lequel Pg^{N} est choisi dans un groupe consistant en Boc, Cbz, Alloc et trityle.

15. Mimétique peptidique selon la revendication 1 dans lequel M' ou M" est le radical méthoxy.

16. Mimétique peptidique selon la revendication 1 dans lequel M' ou M" est le méthyle.

17. Mimétique peptidique selon la revendication 1 de formule I(i)a : 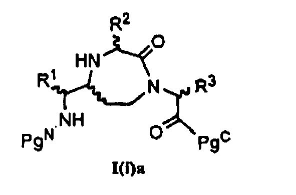 dans laquelle R¹, R² et Pg^{N} sont tels que définis dans la revendication 1, Pg^{C} est un groupe protecteur pour l'acide carboxylique et R³ est un groupe de chaînes latérales d'acide aminé.

18. Mimétique peptidique selon la revendication 17 où R¹ et R2 ≠ H.

19. Mimétique peptidique selon la revendication 1 de formule I(ii)a : 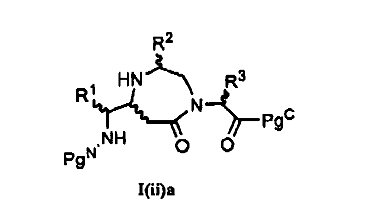 dans laquelle R¹, R² et Pg^{N} sont tels que définis dans la revendication 1, Pg^{C} est un groupe protecteur pour l'acide carboxylique et R³ est un groupe de chaînes latérales d'acide aminé.

20. Mimétique peptidique selon la revendication 19 où R¹ et R² ≠ H.

21. Mimétique peptidique selon la revendication 1 de formule II(i)a : 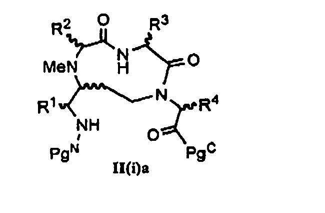 dans laquelle R¹, R² et Pg^{N} sont tels que définis dans la revendication 1, Pg^{C} est un groupe protecteur pour l'acide carboxylique et R³ et R⁴ sont indépendamment des groupes de chaînes latérales d'acide aminé.

22. Mimétique peptidique selon la revendication 21 où R¹ et R² ≠ H.

23. Mimétique peptidique selon la revendication 1 de formule II(iii)a : 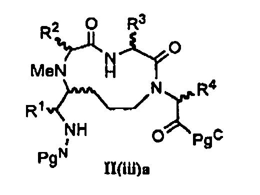 dans laquelle R¹, R² et Pg^{N} sont tels que définis dans la revendication 1, Pg^{C} est un groupe protecteur pour l'acide carboxylique et R³ et R⁴ sont indépendamment des groupes de chaînes latérales d'acide aminé.

24. Mimétique peptidique selon la revendication 23 où R¹ et R² ≠ H.

25. Mimétique peptidique selon la revendication 1 de formule III(i)a : 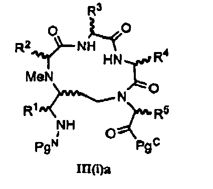 dans laquelle R¹, R² et Pg^{N} sont tels que définis dans la revendication 1, Pg^{C} est un groupe protecteur pour l'acide carboxylique et R³, R⁴ et R⁵ sont chacun indépendamment des groupes de chaînes latérales d'acide aminé.

26. Mimétique peptidique selon la revendication 1 de formule III(ii)a : 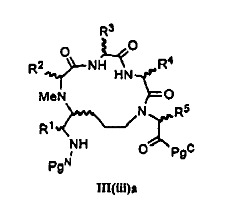 dans laquelle R¹, R² et Pg^{N} sont tels que définis dans la revendication 1, Pg^{C} est un groupe protecteur pour l'acide carboxylique et R³, R⁴ et R⁵ sont chacun indépendamment des groupes de chaînes latérales d'acide aminé.

27. Mimétique peptidique selon la revendication 1 de formule IV(i)a : 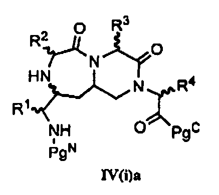 dans laquelle R¹, R² et Pg^{N} sont tels que définis dans la revendication 1, Pg^{C} est un groupe protecteur pour l'acide carboxylique et R³ et R⁴ sont chacun indépendamment des groupes de chaînes latérales d'acide aminé.

28. Mimétique peptidique selon la revendication 1 de formule IV(ii)a : 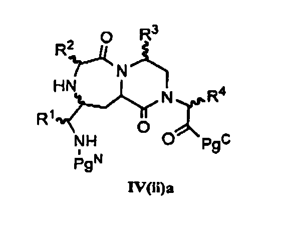 dans laquelle R¹, R² et Pg^{N} sont tels que définis dans la revendication 1, Pg^{C} est un groupe protecteur pour l'acide carboxylique et R³ et R⁴ sont chacun indépendamment des groupes de chaînes latérales d'acide aminé.

29. Mimétique peptidique selon la revendication 1 de formule V(i)a ou V(ii)a : 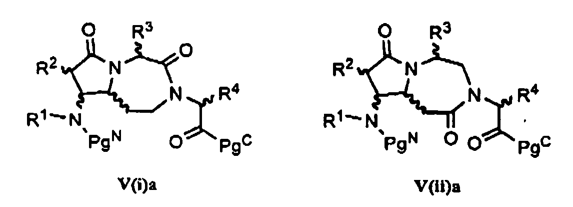 dans laquelle R¹, R² et Pg^{N} sont tels que définis dans la revendication 1, Pg^{C} est un groupe protecteur pour l'acide carboxylique et R³ et R⁴ sont chacun indépendamment des groupes de chaînes latérales d'acide aminé.

30. Mimétique peptidique selon la revendication 1 de formule VI(i)a et VI(ii)a aux présentes : 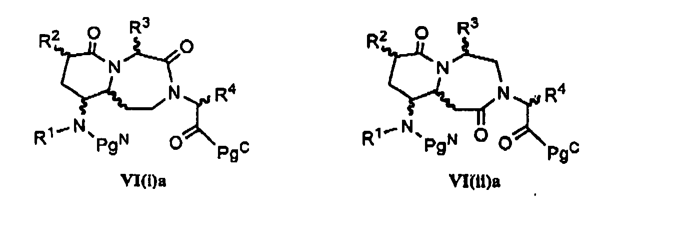 dans laquelle R¹, R² et Pg^{N} sont tels que définis dans la revendication 1, Pg^{C} est un groupe protecteur pour l'acide carboxylique et R³ et R⁴ sont chacun indépendamment des groupes de chaînes latérales d'acide aminé.

31. Composé de formule 4a-d : 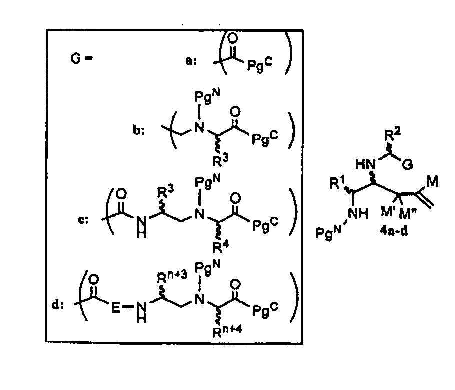 dans laquelle R¹, R², E, M, M', M" et Pg^{N} sont tels que définis dans la revendication 1, Pg^{C} est un groupe protecteur pour l'acide carboxylique et R³, R⁴, Rⁿ⁺³ et Rⁿ⁺⁴ sont chacun indépendamment des groupes de chaînes latérales d'acide aminé.

32. Composé de formule 5a-d : 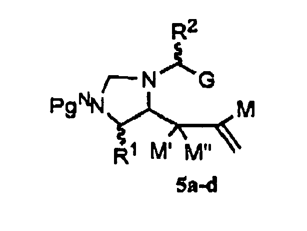 dans laquelle R¹, R², M, M', M" et Pg^{N} sont tels que définis dans la revendication 1, et G est tel que défini dans la revendication 31.

33. Procédé pour la préparation de composés 4a-d selon la revendication 31 comprenant la réaction d'imines 3a-d ci-dessous avec un réactif à base de bore allylique pour donner les composés 4a-d : 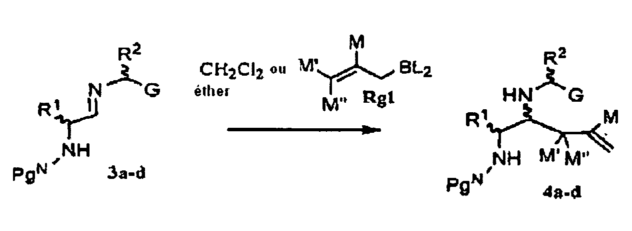 dans lesquels R¹, R², M, M', M" et Pg^{N} sont tels que définis dans la revendication 1 et G est tel que défini dans la revendication 31.

34. Procédé selon la revendication 33 dans lequel des imines 3a-d sont préparées par condensation d'aldéhydes d'acides aminés 1 ci-dessous et d'amines 2a-d : 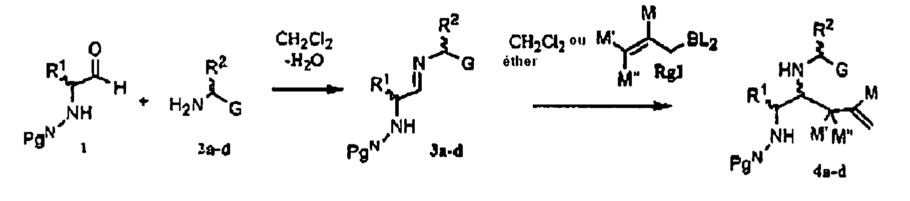 dans lesquelles R¹, R², M, M', M" et Pg^{N} sont tels que définis dans la revendication 1 et G est tel que défini dans la revendication 31.

35. Procédé selon la revendication 33 dans lequel l'addition d'une solution de formaldéhyde aux composés 4a-d donne les imidazolidines 5a-d ci-dessous. 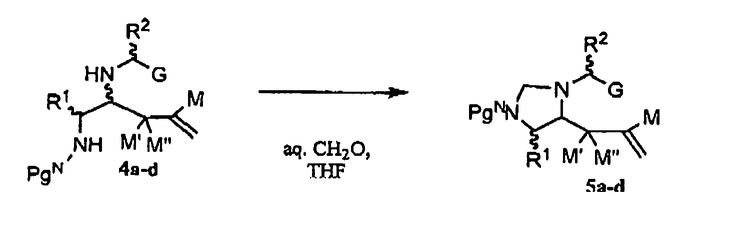 dans lesquelles R¹, R², M, M', M" et Pg^{N} sont tels que définis dans la revendication 1 et G est tel que défini dans la revendication 31.

36. Bibliothèque de mimétiques peptidiques comprenant au moins un mimétique selon l'une quelconque des revendications 1 à 30.

37. Procédé de fabrication de mimétiques (I)(ia) aux présentes dans lequel, dans un premier temps, les composés 49 ci-dessous sont convertis en composés 50 ci-dessous, puis, après une réaction avec les composés 9 ci-dessous, conduisent aux composés 51 ci-dessous qui sont par la suite convertis en composés 52 ci-dessous qui sont ensuite aminés par réduction avec les composés 9 pour donner les composés 54 ci-dessous : 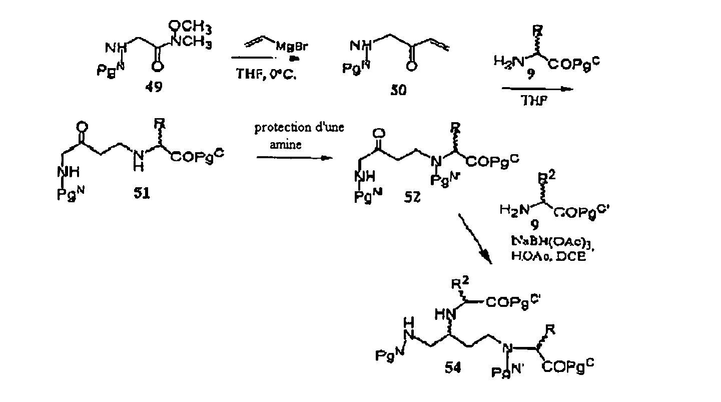 et, dans un second temps, les composés 54 sont convertis en composés 55 après l'élimination des groupes Pg^{N'} et Pg^{C'} qui sont par la suite convertis en mimétiques I(i)a où Z et R¹ sont H : 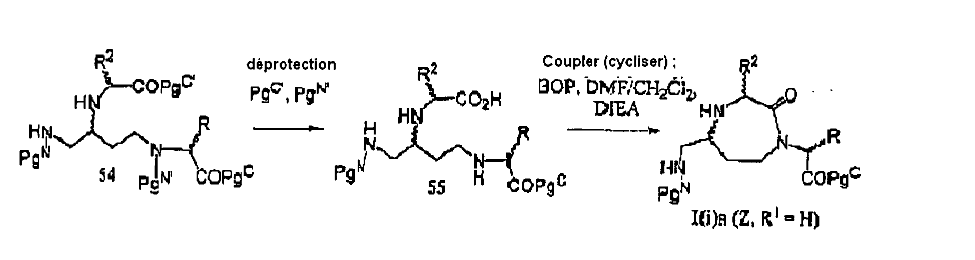 dans lesquels R, R² et Pg^{N} sont tels que définis dans la revendication 1 ; Pg^{C} et Pg^{C'} sont chacun indépendamment un groupe protecteur pour l'acide carboxylique, un lieur clivable à un support en phase solide, un support, OH, -OR, -NHR ou la partie C-terminale restante du mimétique ; et Pg^{N'} est choisi dans le groupe consistant en un groupe protecteur pour une amine, un lieur clivable à un support solide, le support solide, l'hydrogène, R, C(O)R ou une partie de la partie N-terminale restante du mimétique.

38. Procédé de fabrication de mimétiques I(i)a ci-dessous de façon stéréospécifique
dans lequel les composés 49 ci-dessous sont mis à réagir avec du bromure de magnésium vinylique pour donner des composés 50 ci-dessous qui, après réaction avec les composés 9 ci-dessous, conduisent les composés 51 ci-dessous qui sont par la suite mis à réagir avec les composés 15 ci-dessous afin de former les composés 53 ci-dessous qui sont ensuite convertis en mimétiques I(i)a par la déprotection de Pg^{N'} suivie par une amination réductrice : 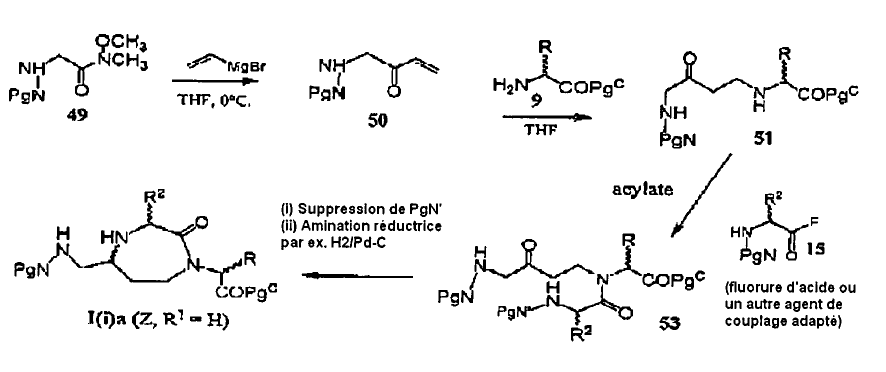 dans laquelle R, R² et Pg^{N} sont tels que définis dans la revendication 1 ; Pg^{C} et Pg^{C'} sont chacun indépendamment un groupe protecteur pour l'acide carboxylique, un lieur clivable à un support en phase solide, un support, OH, -OR, -NHR ou la partie C-terminale restante du mimétique ; et Pg^{N'} est choisi dans le groupe consistant en un groupe protecteur pour une amine, un lieur clivable à un support solide, le support solide, l'hydrogène, R, C(O)R ou une partie de la partie N-terminale restante du mimétique.
